(19) Europäisches Patentamt
European Patent Office
Office européen des brevets

(11) Veröffentlichungsnummer : **0 283 428 B1**

# (12) EUROPÄISCHE PATENTSCHRIFT

(45) Veröffentlichungstag der Patentschrift :
**30.10.91 Patentblatt 91/44**

(21) Anmeldenummer : **88730061.4**

(22) Anmeldetag : **11.03.88**

(51) Int. Cl.$^5$ : **C07J 53/00,** C07J 63/00, C07J 71/00, A61K 31/565, A61K 31/58, // C07J1/00, C07J21/00, C07J41/00, C07J31/00

## (54) 19,11beta-Überbrückte Steroide, deren Herstellung und diese enthaltende pharmazeutische Präparate.

(30) Priorität : **18.03.87 DE 3708942**

(43) Veröffentlichungstag der Anmeldung :
**21.09.88 Patentblatt 88/38**

(45) Bekanntmachung des Hinweises auf die Patenterteilung :
**30.10.91 Patentblatt 91/44**

(84) Benannte Vertragsstaaten :
**AT BE CH DE ES FR GB GR IT LI LU NL SE**

(56) Entgegenhaltungen :
**EP-A- 0 188 396**
**WO-A-83/03099**

(73) Patentinhaber : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **Ottow, Eckhard, Dr.**
**Sonnenallee 124**
**W-1000 Berlin 44 (DE)**
Erfinder : **Wiechert, Rudolf, Prof. Dr.**
**Petzower Strasse 8 a**
**W-1000 Berlin 39 (DE)**
Erfinder : **Neef, Günter, Dr.**
**Darmstädter Strasse 9**
**W-1000 Berlin 15 (DE)**
Erfinder : **Beier, Sybille, Dr.**
**Uhlandstrasse 121**
**W-1000 Berlin 31 (DE)**
Erfinder : **Elger, Walter, Dr.**
**Schorlemer Allee 12 B**
**W-1000 Berlin 33 (DE)**
Erfinder : **Henderson, David Andrew, Dr.**
**Jahnstrasse 17**
**W-1000 Berlin 28 (DE)**

**EP 0 283 428 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 19,11β-überbrückte Steroide, Verfahren zu ihrer Herstellung, diese Verbindungen enthaltende pharmazeutische Präparate, ihre Verwendung zur Herstellung von Arzneimitteln sowie die dazu benötigten neuen Zwischenprodukte.

Die erfindungsgemäßen 19,11β-überbrückten Steroide werden durch die allgemeine Formel I beschrieben

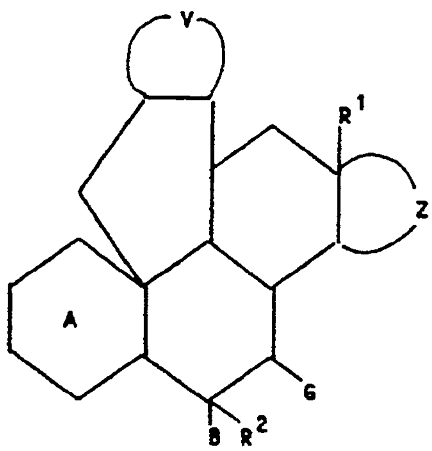

(I),

worin

| | |
|---|---|
| $R^1$ | für einen Methyl- oder Ethylrest, |
| $R^2$ | für ein Wasserstoff-, Chloratom, einen $C_1$-$C_4$-Alkylrest, |
| B und G, | die gleich oder verschieden sind, jeweils für ein Wasserstoffatom, einen $C_1$-$C_4$-Alkylrest oder gemeinsam für eine zweite Bindung zwischen den Kohlenstoffatomen 6 und 7, |
| B und $R^2$ | gemeinsam für eine Methylen- oder Ethylengruppe, |
| Z | für den Rest eines Ringes der Formel |

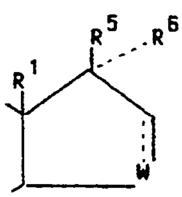

steht, worin

| | |
|---|---|
| $R^1$ | die in Formel I genannte Bedeutung hat, die von W ausgehende gestrichelte Linie die etwaige Anwesenheit einer Doppelbindung bedeutet, |

W einen $CH_2$-, CH-, $CH_2CH_2$- oder $CHCH_2$-Rest,

$R^5/R^6$

$-OR^7/-C{\equiv}C-U$

$-OR^7/-\underset{\underset{O}{\|}}{C}-CH_2-R^8$

$-\underset{\underset{O}{\|}}{C}-CH_2-R^8/-OR^7$

$-\underset{\underset{O}{\|}}{C}-CH_2-R^8/-CH_3$

$-\underset{\underset{O}{\|}}{C}-CH_2-R^8/-H$

$-OR^7/-(CH_2)_m-CH_2-R^9$

$-OR^7/-CH{=}CH(CH_2)_k-CH_2-R^9$

$-OR^{10}/-H$

$-OR^{10}/-(CH_2)_k-C{\equiv}C-U$

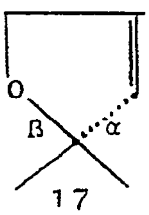

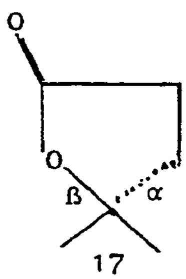

mit

| | |
|---|---|
| $R^7$ | in der Bedeutung eines Wasserstoffatoms oder Acylrestes mit 1 bis 4 Kohlenstoffatomen, |
| U | in der Bedeutung eines Wasserstoffatoms, einer Alkyl-, Hydroxyalkyl-, Alkoxyalkyl-, Acyloxyalkylgruppe mit jeweils 1 bis 4 Kohlenstoffatomen im Alkyl- bzw. Acylrest oder eines Halogenatoms, |
| $R^8$ | in der Bedeutung eines Wasserstoffatoms, einer Hydroxygruppe, einer Alkyl-, O-Alkyl- oder O-Acylgruppe mit jeweils 1 bis 4 Kohlenstoffatomen, |
| $R^9$ | in der Bedeutung eines Wasserstoffatoms, eines Hydroxy- oder Cyanidrestes, einer O-Alkyl- oder O-Acylgruppe mit jeweils 1 bis 4 Kohlenstoffatomen, |
| $R^{10}$ | in der Bedeutung eines Wasserstoffatoms, einer Alkyl- oder Acylgruppe mit jeweils 1 bis 10 Kohlenstoffatomen, |
| m | in der Bedeutung 0, 1, 2 oder 3 |
| k | in der Bedeutung 0, 1 oder 2, bedeuten, |
| V | für den Rest eines Phenylrings der Formel |

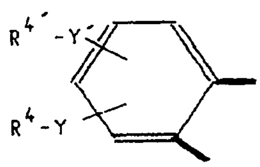

oder für den Rest eines fünf- oder sechsgliedrigen heteroaromatischen Rings mit 1 bis 2 N—, O— oder S-Atomen der Formel

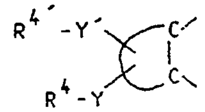

worin

| | |
|---|---|
| $R^4$ und $R^{4'}$, | die gleich oder verschieden sind, jeweils ein Wasserstoffatom einen Cyanidrest, eine $—OR^{11}$, $—S(O)_kR^{11}—$, $—N(O)_nR^{11}R^{12}—$, $—O\text{-}SO_2R^{13}—$, $—P(O)(OR^{14})_2$, $—SiR^{14}_3$ oder $—SnR^{14}_3$-Gruppe mit k in der Bedeutung der Ziffern 0, 1 oder 2, n in der Bedeutung der Ziffern 0 oder 1, |
| $R^{11}$ | in der Bedeutung eines Wasserstoffatoms oder eines $C_1$-$C_8$-Alkylrestes, |
| $R^{12}$ | in der Bedeutung von $R^{11}$, eines Cyanid- oder eines $C_1$-$C_{10}$-Acylrestes, |
| $R^{13}$ | in der Bedeutung eines perfluorierten $C_1$-$C_4$-Alkylrestes, |
| $R^{14}$ | in der Bedeutung eines $C_1$-$C_4$-Alkylrestes oder |
| $R^{11}$ und $R^{12}$ | innerhalb einer $—N(O)_nR^{11}R^{12}$-Gruppe gemeinsam unter Einschluß von N einen 5- oder 6gliedrigen heterocyclischen Ring, wobei im Ring noch ein weiteres Heteroatom N, O oder S enthalten sein kann, |
| Y und Y', | die gleich oder verschieden sind, jeweils für eine direkte Bindung, eine geradkettige oder verzweigte, gegebenenfalls Doppel- oder Dreifachbindung(en) aufweisende Alkylengruppe mit bis zu 20 Kohlenstoffatomen, die gegebenenfalls substituiert ist durch eine oder mehrere Oxo-, $C_1$-$C_{10}$-Akyloxy-, $—OR^{11}—$, $—S(O)_kR^{11}—$ und/oder $—N(O)_nR^{11}R^{12}$-Gruppe(n), ein gegebenenfalls substituierter Arylenrest oder |
| $R^4$-Y und $R^{4'}$-Y' | gemeinsam den Rest eines, gegebenenfalls substituierten gesättigten, ungesättigten oder aromatischen S-oder 6-gliedrigen Ringes mit 0 bis 2 Sauerstoff-, Schwefelatomen und/oder $NR^{11}$-Gruppen bedeuten, mit der Maßgabe, daß nur dann k und n größer als 0 sind, wenn $R^{11}$ für einen $C_1$-$C_8$-Alkylrest steht und der Ring A für |

a)

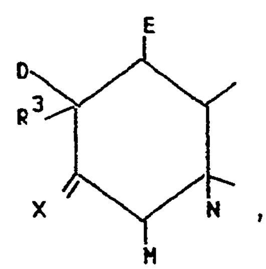

wobei

| | |
|---|---|
| M und N | gemeinsam eine zweite Bindung oder M ein Wasserstoffatom und N eine Hydroxygruppe, wobei dann B, $R^2$, G, $R^3$ D und E Wasserstoffatome sind und |
| X | ein Sauerstoffatom, zwei Wasserstoffatome oder eine Hydroxyiminogruppierung N ~OH, |
| $R^3$ und D, | die gleich oder verschieden sind, jeweils ein Wasserstoffatom, einen Nitrilrest oder einen $C_1$-$C_4$-Alkylrest oder gemeinsam eine Methylen- oder Ethylengruppe, |
| E | ein Wasserstoffatom, einen $C_1$-$C_4$-Alkylrest, |
| D und E | gemeinsam eine zweite Bindung zwischen den Kohlenstoffatomen 1 und 2 oder gemeinsam eine Methylengruppe bedeuten |
| | oder |

b)

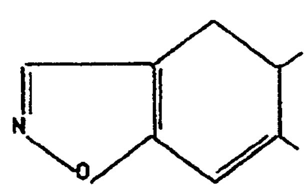

oder

c)

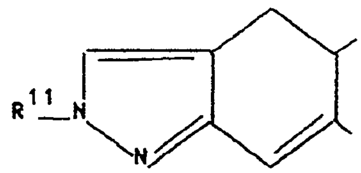

mit $R^{11}$ in der Bedeutung eines Wasserstoffatoms oder eines $C_1$-$C_8$-Alkylrestes stehen, sowie gegebenenfalls deren pharmazeutisch verträgliche Additionssalze mit Säuren.

Als Arylrest V kommen substituierte und unsubstituierte Phenyl- oder heterocyclische Reste, wie zum Beispiel Furyl, Thienyl, Pyridil, Pyrazolyl, Pyrrolyl, Pyrimidinyl, Oxazolyl, Pyridazinyl, Pyrazinyl infrage.

Wenn Y-$R^4$ = H und Y' die in 1-Position mit einer Oxogruppe substituierte Ethylengruppe und $R^{4'}$ = H ist, liegt als Y'-$R^{4'}$ die Acetyl-Gruppe vor, der im Rahmen der Erfindung eine bevorzugte Rolle zukommt.

Bei der Substitution des Phenylringes ist die Honosubstitution in 3-, 4- oder 5-Position sowie die Disubstitution in 4- und 5- oder 3- und 4-Position unter Bildung eines ankondensierten zweiten Ringes, zum Beispiel eines Cyclohexen-, Pyrrol-, Furyl-, Pyrrolin-, 1,3-Dioxacyclopenten-, Pyrazolin-, Didehydromorpholin-, Didehydropiperidin-, Didehydropiperazin-, Dihydropyran-, Pyrimidin-, Pyridin-, Pyrazin-, 1,4-Dioxacyclohexen-Ringes, bevorzugt.

Die für $R^1$ und $R^{11}$ bzw. $R^2$, $R^3$, B, G und D stehenden Alkylreste sollen im Fall von $R^1$ 1 oder 2, im Fall von $R^{11}$ 1 bis 8 und ansonsten 1 bis 4 Kohlenstoffatome tragen, wobei die Hethyl-, Ethyl-, Propyl-, Isopropyl-, Butyl- bzw. Hethyl-, Ethyl-, Propylgruppen bevorzugt sind.

Steht $R^{12}$ für einen Acylrest, so ist die Formyl-, Acetyl-, Propionyl-, Butyryl und Benzoyl-gruppe bevorzugt.

$R^{11}$ und $R^{12}$ stehen auch gemeinsam unter Einschluß des Stickstoffatoms für einen heterocyclischen Fünf- oder Sechsring, der außer N- und C-Atome auch noch zusätzlich ein O- oder S-Atom enthalten kann ; beispielsweise genannt seien der Pyrrol-, Pyrrolidin-, Piperidin-, Piperazin-, Horpholin-, Oxa- und Thiazolidinsowie Thiadiazolidin-Ring.

Die in $R^5$ und $R^6$ bzw. $R^7$, $R^8$, $R^9$, $R^{10}$ und U der allgemeinen Formel I enthaltenen Alkyl-, Alkoxy- sowie Acyloxygruppen sollen jeweils 1 bis 4 Kohlenstoffatome enthalten, wobei die Methyl-, Ethyl-, Propyl-, Isopropyl-, Methoxy-, EthoxyPropoxy-, Isopropoxy-, Formyl-, Acetyl-, Propionyl- und Isopropionylgruppe bevorzugt sind.

Von den Alkenylresten in $R^6$ sind die Propenyl- und Butenylgruppe, die in der E-oder Z-Konfiguration vorliegen können, bevorzugt, d.h. wenn $R^6$ für —CH=CH-$(CH_2)_k CH_2$-$R^9$ steht, dann soll k bevorzugt 0 oder 1 bedeuten.

Die neuen Verbindungen der allgemeinen Formel I werden erfindungsgemäß nach dem Verfahren gemäß Anspruch 14 hergestellt.

Zur Herstellung der Zwischenprodukte der allgemeinen Formel II

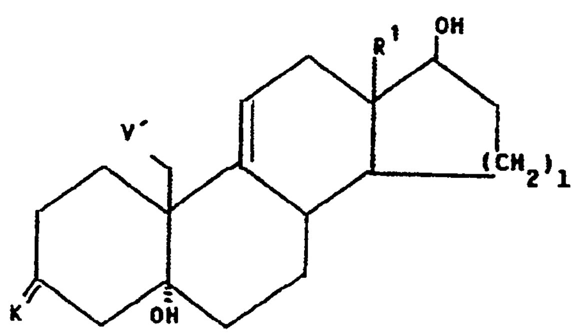

(II),

worin

| | |
|---|---|
| $R^1$ | einen Methyl- oder Ethylrest |
| l | die Ziffern 1 oder 2, |
| K | eine in Form des Ketals oder Thioketals blockierte Ketogruppe bedeuten und |

V' für einen Phenylring der Formel

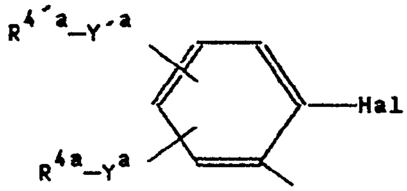

oder für einen 5- oder 6gliedrigen heteroaromatischen Ring mit 1-2 N—, O— oder S-Atomen der Formel

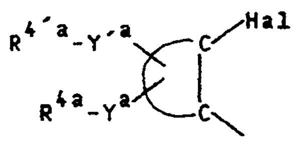

steht,
worin

Hal ein Fluor-, Chlor-, Brom- oder Jodatom bedeutet,

$R^{4a}$, $R^{4'a}$, $Y^a$ und $Y'^a$ unter Ausschluß des Cyanidrestes die gleiche Bedeutung wie $R^4$, $R^{4'}$, Y und Y' haben, wobei gegebenenfalls vorhandene Hydroxy-, Mercapto-, Amino-, Oxo- und/oder terminale Acetylengruppen geschützt sind, die ebenfalls Gegenstand der Erfindung sind, geht man aus von den nach den Vorschriften in zum Beispiel gemäß EP-A-0110434 ; DE-A-3438484 oder EP-A-0127864 erhaltenen Epoxiden der allgemeinen Formel III

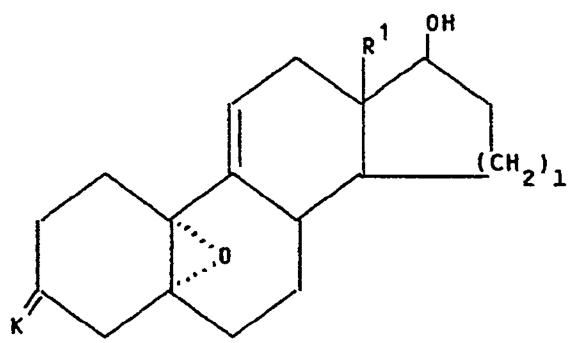

(III),

worin

$R^1$, l und K die oben genannte Bedeutung haben.

Durch Grignard-Addition (Tetrahedron Letters 1979, 2051) von Arylmethylhalogeniden der allgemeinen Formel V

$$V'CH_2Hal \quad (V),$$

worin Hal für ein Chlor-, Brom- oder Jodatom steht, die in 2-Position zur Verknüpfungsstelle des Aromaten (carbocyclisch oder heterocyclisch) an die Hethylgruppe ein Fluor-, Chlor-, Brom- oder Jodatom tragen, an die oben genannten Epoxide, werden die erfindungsgemäßen Zwischenprodukte der allgemeinen Formel II erhalten.

Nach Schutz der gegebenenfalls in V' vorhandenen funktionellen Gruppen werden die neuen Verbindungen der allgemeinen Formel II einer Cyclisierung unterworfen.

Die von V' und K umfaßten Hydroxy-, Mercapto- und Keto-Schutzgruppen sind im sauren Milieu leicht abspaltbare Gruppen, wie zum Beispiel die Hethoxymethyl-, Ethoxymethyl-,Tetrahydropyranyl-, Ethylendioxyketal-, Ethylendithioketal- oder 2,2-Dimethyltrimethylendioxyketalgruppe.

Schutzgruppen für Amino- und terminale Acetylengruppen (zum Beispiel die Trimethylsilyl- und tert.-Butyldimethylsilylgruppe) sind dem Fachmann ebenfalls bekannt und werden nach der gewünschten Reaktionsfolge auch nach literaturbekannten Verfahren gespalten [Synthesis 1980, 627, J. Org. Chem. 46 (1986) 2280]

Die Umwandlung von II zu den neuen 19,11β-überbrückten Steroiden der allgemeinen Formel IVb

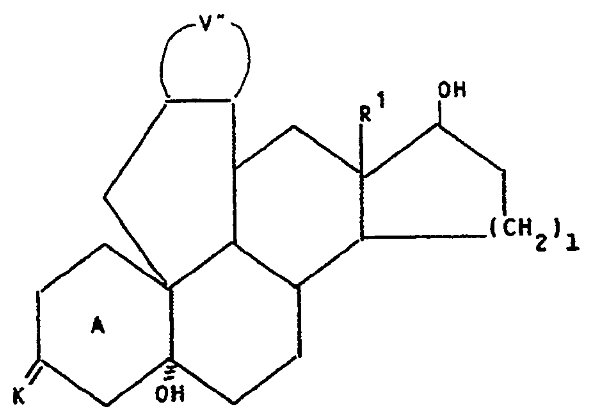

(IVb),

worin $R^1$, K und l die oben genannte Bedeutung haben und V″ die gleiche Bedeutung wie V hat, wobei jedoch in V gegebenenfalls vorhandene Hydroxy-, MercaptoAmino-, Oxo- und/oder terminale Acetylen-Gruppen geschützt sind, die ebenfalls Gegenstand der Erfindung sind, erfolgt im Falle, daß der α-Hologensubstituent in V′ ein Brom- oder Jodatom ist, nach an sich bekannten Methoden (Tetrahedron Letters 1982, 2575 ; 1985, 6001 ; 1986, 2833 ; J. Am. Chem. Soc. 1982, 104, 2321 ; Radicals in Organic Synthesis : Formation of Carbon-Carbon Bonds, Pergamon Press 1986) durch reduktive radikalische Cyclisierung.

Eine entsprechende Methode für fluor- und chlorsubstituierte Aromaten war bisher nicht bekannt. Es wurde nun gefunden, daß diese Cyclisierung in guter Ausbeute überraschenderweise durch Behandlung des Edukts mit einem elektropositiven Metall, wie zum Beispiel Natrium, Kalium, Lithium oder Calcium, in flüssigem Ammoniak, gemischt mit einem oder mehreren geeigneten organischen Lösungsmittel(n) wie zum Beispiel Diethylether, Dimethoxyethan (DME) Dioxan oder Tetrahydrofuran bei Temperaturen zwischen −100 und −30°C, vorzugsweise −78 bis −60°C, gelingt. Daß diese Cyclisierung auch mit dem Fluorid-Ion als Abgangsgruppe durchführbar ist, ist als besonders überraschend anzusehen.

Diese neue Methode ist auch bei den brom- und jodsubstituierten Aromaten anzuwenden.

Die Umwandlung der so erhaltenen Cyclisierungsprodukte in die letzlich gewünschten Endprodukte der allgemeinen Formel I erfolgt analog literaturbekannten Verfahren (zum Beispiel J. Fried, J.A. Edwards, "Organic Reactions in Steroid Chemistry", Van Nostrand Reinhold Company 1972, Vol. 1 und 2 ; "Terpenoids and Steroids", Specialist Periodical Report, The Chemical Society, London, Vol. 1-12) indem man entweder zuerst

a) die C-17-Hydroxygruppe oxidiert und anschließend
b) gegebenenfalls eine eine Schutzgruppe aufweisende Hydroxygruppe in V′ von dieser Schutzgruppe befreit, gewünschtenfalls aus der Hydroxyverbindung ein entsprechendes Perfluoralkylsulfonat ($C_1$-$C_4$) herstellt, gegebenenfalls das Perfluoralkylsulfonat entweder direkt oder durch Austausch der Perfluoralkylsulfonatabgangsgruppe gegen eine Zinntrialkylgruppe über die entsprechende Zinntrialkylverbindung in eine Verbindung überführt, die in V″, gegebenenfalls nach weiteren Reaktionen, das gewünschte Substitutionsmuster aufweist,
oder zuerst b) und dann a) ausführt und danach
c) den Ring D in gewünschter Weise nach an sich bekannten Methoden funktionalisiert, das so erhaltene Produkt der Einwirkung eines Dehydratationsmittels, das auch zur Freisetzung der 3-Oxogruppe befähigt ist, zur Wasserabspaltung unter gleichzeitiger Ausbildung der 4(5)-Doppelbindung unterwirft und anschließend, gegebenenfalls nach erneutem Schutz von intermediär freigesetzten, in V und/oder Z enthaltenen funktionellen Gruppen, die gewünschten Funktionen der Ringe A und B des Steroidgerüstes einführt oder
d) das so erhaltene Produkt der Einwirkung eines Dehydratationsmittels, das auch zur Freisetzung der 3-Oxogruppe befähigt ist zur Wasserabspaltung unter gleichzeitiger Ausbildung der 4(5)-Doppelbindung unterwirft, die gewünschten Funktionen der Ringe A und B des Steroidgerüstes einführt und anschließend, nach Schutz der 3-Oxogruppe, den Ring D in gewünschter Weise funktionalisiert,
oder die Schritte a) und b) nach dem Schritt c) oder d) ausführt,

das so erhaltene Produkt gegebenenfalls von Schutzgruppen befreit, gewünschtenfalls die in V gegebenenfalls enthaltene(n) Hydroxy-, Mercapto- und/oder Aminogruppe(n) alkyliert bzw. acyliert, gewünschtenfalls einen Cyanidrest in den/die Arylsubstituenten einführt, gewünschtenfalls die in dem(n) Arylsubstituenten gegebenenfalls enthaltene(n) Amino- und/oder Sulfidgruppe(n) oxidiert, gewünschtenfalls mit Hydroxylamin-hydrochlorid zum Produkt der allgemeinen Formel I mit X in der Bedeutung der Hydroxyiminogruppierung N OH umsetzt

sowie gegebenenfalls ein pharmazeutisch verträgliches Säureadditionssalz herstellt.

Im Verlaufe dieser Reaktionswege kann es notwendig werden, intermediär erneut Schutzgruppen in Zwischenprodukte, zum Beispiel für in Z enthaltene funktionelle Gruppen bei anschließender Funktionalisierung der Ringe A und B oder für die 3-Ketogruppe bei anschließendem Aufbau des Ringes D, einzuführen.

Die für die Herstellung fast aller Endprodukte nötige Oxidation der 17β-Hydroxygruppe wird in an sich bekannter Weise, zum Beispiel durch Oppenauer-Oxidation oder mit Chromsäurereagenzien (Jones'-Reagenz oder Chromsäure-Pyridin) durchgeführt.

Die Freisetzung der 3-Ketofunktion unter gleichzeitiger Wasserabspaltung und Ausbildung der 4(5)-Doppelbindung erfolgt durch Behandlung mit Säure oder einem sauren Ionenaustauscher. Die saure Behandlung erfolgt in an sich bekannter Weise, indem man das entsprechende 5α-Hydroxy-3-ketal in einem mit Wasser mischbaren Lösungsmittel, wie wäßrigem Methanol, Ethanol oder Aceton, löst und auf die Lösung katalytische Mengen Mineral- oder Sulfonsäure, wie Salzsäure, Schwefelsäure, Phosphorsäure, Perchlorsäure oder p-Toluolsulfonsäure, oder eine organische Säure, wie Essigsäure, so lange einwirken läßt, bis vorhandene Schutzgruppen entfernt sind und gegebenenfalls Wasser abgespalten ist. Die Umsetzung, die bei Temperaturen von 0 bis 100°C abläuft, kann auch mit einem sauren Ionenaustauscher vorgenommen werden. Der Verlauf der Umsetzung kann mit analytischen Methoden, beispielsweise durch Dünnschichtchromatographie entnommener Proben, verfolgt werden.

Im allgemeinen wird die Schutzgruppenentfernung und Wasserabspaltung in einem Reaktionsschritt bewerkstelligt, indem man das entsprechende 5α-Hydroxy-3-ketal bzw. 5-en-3-Ketal in stark saurem Medium eine gewisse Zeit lang reagieren läßt, wie dies in Beispiel 1 c) beschrieben ist. Genausogut ist es aber erfindungsgemäß möglich, die Schutzgruppenentfernung und Wasserabspaltung in zwei voneinander getrennten Reaktionsschritten durchzuführen, indem zuerst durch eine kürzere Behandlung des entsprechenden 5α-Hydroxy-3-ketals in mäßig saurem Medium zunächst die entsprechende 5α-Hydroxy-3-keto-Verbindung gewonnen und gegebenenfalls isoliert wird, wie die exemplarisch in Beispiel 9 gezeigt ist. Die 5αHydroxy-3-keto-Verbindung wird dann durch weiteres Einwirkenlassen von Säure unter Wasserabspaltung in die 3-Keto-4-en-Verbindung überführt.

Ein ganz besonderer Vorteil der vorliegenden Erfindung liegt in der großen Bandbreite der am carbocyclischen oder heterocyclischer Arylrest V einführbaren Substituenten (M. Pereyre, J.-P. Quintard, A. Rahm, Tin in Organic Synthesis ; Butterworths, 1987). Zum einen können die im späteren Endprodukt vorhandenen Substituenten $R^4$-Y- bzw. $R^{4'}$-Y'- direkt eingeführt werden, indem ein am Arylrest entsprechend substituiertes Arylmethylhalogenid der allgemeinen Formel V, V'$CH_2$Hal, nach Grignard-Reaktion, mit einem geeigneten 5α,10αEpoxid der allgemeinen Formel III gekoppelt und das entstandene Zwischenprodukt der allgemeinen Formel II in der bereits beschriebenen Weise weiterverarbeitet wird.

Die Anzahl der auf diese Weise herstellbaren an V substituierten Verbindungen ist relativ beschränkt, da nicht alle im Endprodukt wünschenswerten Substituenten die Bedingungen der Grignard-Reaktion, die an V'$CH_2$Hal vor der Kopplung mit dem jeweiligen 5α,10α--Epoxid-III durchgeführt werden muß, und insbesondere die reduktiven Bedingungen während der Cyclisierung des Zwischenprodukts II zu einem 19,11β-überbrückten Steroid der allgemeinen Formel IV unbeschadet überstehen.

Mit einer weiteren Ausführungsform des erfindungsgemäßen Verfahrens gelingt es aber, den (die) Substituenten im Arylrest V über einen weiten Bereich zu variieren, indem der (die) Substituent(en) erst nach der Cyclisierung, und zwar vor, gleichzeitig mit oder erst nach Fertigstellung der Struktur der Ringe A, B und D eingeführt wird (werden). Dazu wird mindestens eine der im Rest V" bzw. V vorhandenen und geschützten Hydroxygruppe von ihrer Schutzgruppe befreit und aus der freien OH-Verbindung durch Umsetzung mit Perfluoralkylsulfonsäureanhydrid (Alkyl = $C_1$-$C_4$) nach an sich bekannten Methoden [P.J. Stang, M. Hanack and L.R. Subramanian, Synthesis 85 (1982)] die entsprechende Perfluoralkylsulfonatverbindung hergestellt.

Dabei wird entweder so vorgegangen, daß in einer übergangsmetallkatalysierten Reaktion (vorzugsweise Pd°) die Perfluorübergangsgruppe unter im wesentlichen fast gleichzeitiger Substitution durch den gewünschten Substituenten oder dessen Vorstufe verdrängt wird (J.E. McMurry and S. Mohanraj, Tetrahedron Letters, 24, No. 27, S. 2723-2726, 1983 ; X. Lu und J. Zhu, Communications, S. 726-727, 1987 ; Q. -Y. Chen und Z.-Y. Yang, Tetrahedron Letters 27, No. 10, S. 1171-1174, 1986 ; S. Cacchi, P.G. Ciattini, E. Morera und G. Ortar, Tetrahedron Letters, 27, No. 33, S. 3931-3934, 1966 ; A.M. Echavarren und J.K. Stille, J. Am. Chem. Soc. 1987, 109, S. 5478-5486) oder es wird aus der Perfluoralkylsulfonat-Verbindung intermediär und übergangsmetallkatalysiert eine entsprechende Tri-organylstannyl-, vorzugsweise Tri-n-alkylstannyl-Verbindung hergestellt [J.K. Stille, Angew, Chem. 98 (1986), S. 504-519]. Diese wird anschließend in einer Eintopf-Reaktion mit einem halogen-, vorzugsweise brom- oder jodsubstituierten carbocyclischen oder heterocyclischen Aromaten [Y. Yamamoto, Y. Azuma, H. Mitoh, Communications, S. 564-565, 1986 ; T. J. Bailey, Tetrahedron Letters, 27, No. 37, S. 4407-4410, 1986], der gegebenenfalls noch weitere Substituenten tragen kann, zu einem 19,11β-überbrückten Steroid umgesetzt ; der Arylrest V bzw. V" weist darin dann die gewünschte bzw. einen Vorläufer

der gewünschten Substitution auf.

Die intermediär auftretenden Tri-n-alkylstannyl-Verbindungen können auch in Substanz isoliert werden, wie dies im Beispiel 39 a) α) für den Fall des 11β,19(4-Tri-n-butylstannyl-O-phenylen)-3,3-(2,2-dimethyltrimethylendioxy)-androstan-5α,17β-diols stellvertretend belegt ist.

Die Einführung von 1,2 und/oder 6,7-Doppelbindungen neben der 3,4-Doppelbindung gelingt nach bekannten Methoden, zum Beispiel mit Dehydrierungsmitteln wie Selendioxid, Chloranil, Thalliumtriacetat oder Dichlordicyanobenzochinon (DDQ) bzw. durch Allyl- oder Dienoletherbromierung und anschließende Bromwasserstoffabspaltung. [J. Fried, J.A. Edwards, Organic Reactions in Steroid Chemistry, Van Nostrand Reinhold Company 1972, S. 265-374, 1 ; Tetrahedron 42, (1986) 2971]

Die Allylbromierung wird zum Beispiel mit N-Bromsuccinimid, N-Bromacetamid, 1,3-Dibrom-5,5-dimethylhydantoin oder Dibromtetrachlorethan in Gegenwärt eines Radikalbildners wie Dibenzoylperoxid in einem Lösungsmittel vorgenommen. Als Lösungsmittel kommen aprotische Lösungsmittel wie Dioxan und chlorierte Kohlenwasserstoffe, wie zum Beispiel Tetrachlorkohlenstoff, Chloroform oder Tetrachlorethylen infrage. Die Umsetzung erfolgt zwischen 0°C und der Siedetemperatur der Lösung.

Die Dienoletherbromierung wird zum Beispiel analog der Vorschrift in Steroids I, 233 durchgeführt.

Die Bromwasserstoffabspaltung unter Ausbildung der $\Delta^6$-Doppelbindung erfolgt durch Erhitzen der 6-Bromverbindung mit basischen Mitteln, vorzugsweise mit Lithiumbromid und Lithiumcarbonat oder mit Lithiumbromid und Calciumcarbonat in einem aprotischen Lösungsmittel wie Dimethylformamid bei Temperaturen von 50 bis 120°C. Eine weitere Möglichkeit der HBr-Abspaltung besteht darin, daß man die 6-Bromverbindung in Collidin oder Lutidin erhitzt.

Ausgehend von einem gesättigten Ring A können Doppelbindungen in 1,2- und 4,5-Stellung gleichzeitig eingeführt werden, zum Beispiel durch Bromierung zum 2,4-Dibrom-3-keton und Dehydrobromierung des Dibromids mit zum Beispiel Lithium- oder Calciumcarbonat und Lithiumbromid in Dimethylformnamid.

Die Einführung einer 6-Methylengruppe kann zum Beispiel ausgehend von einem 3-Amino-3(4),5(6)-dien-Derivat durch Umsetzung mit Formalin in alkoholischer Lösung (Helv. Chim. Acta. 56 (1973) 2396) zur 6α-Hydroxymethylgruppe und anschließender saurer Wasserabspaltung zum Beispiel mit Salzsäure in Dioxan/Wasser oder ausgehend von einem 3-Alkoxy-3(4),5(6)-dien-Derivat, analog der im US-Patent 4,544,555 beschriebenen Methode oder direkt, ausgehend von einem 3-Oxo-4(5)-en-Derivat analog der Vorschrift in Synthesis (1982) 34 erfolgen.

Die Methylenierung der 6-Methylen- zur 6,6-Ethylenverbindung erfolgt mit Dimethylsulfoxoniummethylid. Hierzu wird das 6-Methylen-Steroid zu einer Suspension von Trimethylsulfoxoniumjodid mit Natriumhydrid in Mineralöl und Dimethylsulfoxid oder zu einer Lösung von Trimethylsulfoxoniumjodid und Natriumhydroxid in Dimethylsulfoxid gegeben. Die Reaktion ist nach 15 bis 60 Minuten bei 20 bis 40°C beendet (J. Am. Chem. Soc. 84 (1962) 866 ; Europäische Patentanmeldung 0150157).

Die Einführung einer 2 Methylengruppe erfolgt analog der Methode von A. J. Manson und D. Wood [J. Org. Chem. 32 (1967) 3434] oder den dort zitierten Methoden.

Die Methylenierung der 2-Methylen- zur 2,2-Ethylenverbindung erfolgt analog der Methylenierung der 6-Methylenverbindung [siehe auch Chem. Ber. 98 (1965) 1470].

Mono- bzw. dialkylierte Verbindungen in 2-Position können zum Beispiel analog der Methode von L. Nedelec, Tetrahedron 30 (1974) 3263 erhalten werden.

Alkylierte Verbindungen in Position 1 bzw. 7 werden durch 1,4- bzw. 1,6-Addition an die entsprechenden Enone nach bekannten Methoden (J. Fried, J.A. Edwards : Organic Reactions in Steroid Chemistry, Van Nostrand Reinhold Company 1972, Seite 75 bis 82, 2 ; und J. Am. Chem. Soc. 99 (1977) 1673) erhalten.

Alkylierte Verbindungen in Position 6 können zum Beispiel durch Öffnung der entsprechenden 5α,6α-Epoxide und Folgereaktionen erhalten werden (J. Fried, J.A. Edwards : Organic Reactions in Steroid Chemistry, Van Nostrand Reinhold Company 1972, Seite 82-86, 2).

1α,2α-, 6α,7α-, 6β,7β-Methylenverbindungen oder eine Kombination des 1α,2α-Methylenstrukturelements mit den beiden 6,7-Methylen-Strukturelementen lassen sich durch Addition von Diazomethan oder Dimethylsulfoxoniummethylid an die entsprechenden Enone bzw. durch Simmons-Smith-Reaction (J. Fried, J.A. Edwards : Reactions in Steroid Chemistry, Van Nostrand Reinold Company 1972, Seite 100-126 ; Rev. Soc. Quim. Mex. (1969) 171A ; Chem. Ber. 101 (1968) 935 ; Chem. Ber. 99 (1966) 1118 ; Zeitschr. f. Naturf. 19b (1964) 944) der entsprechenden Allylalkohole erhalten.

Die Herstellung des an die Positionen 2 und 3 annelierten Isoxazolringes erfolgt über die Synthese der 2-Hydroxymethylenverbindungen [Steroids 6 (1962) 178 ; J. Amer. Chem. Soc. 83 (1961) 1478] und ihre Umsetzung mit Hydroxylamin [J. Med. Chem. 6 (1963) 1].

[2,3-d]isoxazole sind auch gute Ausgangsmaterialien für die Synthese von 2-Cyano-Steroiden [J. Med. Chem. 6 (1963) 1].

Die Herstellung des an die Positionen 2 und 3 annelierten Pyrazolringes erfolgt durch Umsetzung von 2-Hydroxymethylen-3-oxo-Edukten mit $R^{11}$-substituiertem Hydrazin (US-Patent 3,704,295).

Die Einführung des Chlor- bzw. Methyl-Substituenten in C-6 des Steroidgerüsts gelingt z.B. durch die in der Deutschen Auslegeschrift 1,158,966 bzw. in US-Patent 4,544,555 und US-Patent 4,196,203 angegebenen Methoden über die entsprechenden 6,7-Epoxide bzw. 6-Methylen-Derivate sowie durch Oxidation des 6-Chlor-3,5-dienolethers mit Dichlordicyanobenzochinon (DDQ) unter sauren Bedingungen [Belgisches Patent 621,197 (1962)].

Die Entfernung der 3-Oxogruppe zu einem Endprodukt der allgemeinen Formel I mit X in der Bedeutung von zwei Wasserstoffatomen kann z.B. nach der in DOS 2805490 angegebenen Vorschrift durch Thioketalisierung und anschließende reduktive Spaltung erfolgen.

Edukte mit einem D-Homo-Steroidgerüst sind auch z.B. durch Tiffeneau-Umlagerung analog der in Australian J. Chem. 8 (1955), 519 und in "Organic Reactions in Steroid Chemistry" Vol 2, 388 veröffentlichten Vorschrift zu erhalten. Die notwendigen 17α-Aminomethyl-17β-hydroxyverbindungen sind zum Beispiel über die Öffnung der 17,20-Spiroepoxide mit Ammoniak oder auch durch Lithiumaluminiumreduktion der acetylierten 17β-Hydroxy-17α-cyanoverbindungen zugänglich. Die Spiroepoxide sind durch Umsatz der entsprechenden 17-Ketone mit Dimethylsulfoniummethylid in Dimethylformamid [Journal f. prakt. Chemie 314 (1972), 667-668) zugänglich. Die acetylierten Cyanhydrine sind durch Anlagerung von Cyanwasserstoff an die entsprechenden 17-Ketone und anschließende Acetylierung nach bekannten Vorschriften (z.B. Australian J. Chem. 8 (1955), 519) zugänglich.

Edukte mit einem ungesättigten D-Ring sind zum Beispiel durch modifizierte Saegusa-Oxidation (Tetrahedron 42 (1986) 2971) der entsprechenden Enolverbindungen des 17-Ketons zugänglich. Zum Beispiel ist der Trimethylsilylenolether durch Überführung des 17-Ketons mit Lithiumdiisopropylamid in Tetrahydrofuran in das korrespondierende Enolat und Abfang durch Trimethylchlorsilan darstellbar (Synthesis 1983, 1).

Die Einführung der Substituenten $R^5$ und $R^6$ erfolgt nach den üblichen Verfahren des C-17-Seitenkettenaufbaus durch nucleophile Addition an das — durch z.B. Oppenauer-Oxidation der C-17-Hydroxygruppe erhaltene -17-Keton und Folgereaktionen ("Terpenoids and Steroids", Specialist Periodical Report, The Chemical Society, London, Vol. 1-12)

Die Einführung des Substituenten —CℓC-U als $R^6$, wobei U die oben angegebene Bedeutung hat, erfolgt mit Hilfe einer Verbindung der allgemeinen Formel MCℓC-U', in der U' der mit einer Schutzgruppe, wie zum Beispiel Trimethylsilyl oder tert.-Butyldimethylsilyl, geschützte Rest U oder aber im Fall, wenn U eine Alkylgruppe mit 1-4 C-Atomen ist, U' selbst der Rest U ist.

Die metallorganische Verbindung kann auch in situ gebildet und mit dem 17-Keton zur Reaktion gebracht werden. So kann man zum Beispiel auf das 17-Keton in einem geeigneten Lösungsmittel Acetylen und ein Alkalimetall, insbesondere Kalium, Natrium oder Lithium, in Gegenwart eines Alkohols oder in Gegenwart von Ammoniak einwirken lassen. Das Alkalimetall kann auch in Form von zum Beispiel Methyl- oder Butyllithium zur Einwirkung kommen. Als Lösungsmittel sind insbesondere Dialkylether, Tetrahydrofuran, Dioxan, Benzol und Toluol geeignet.

Die Einführung von 3-Hydroxypropin, -propen bzw. -propan in 17-Stellung erfolgt durch Umsetzung des 17-Ketons mit dem Dianion des Propargylalkohols (3-Hydroxypropin), zum Beispiel dem in situ generierten Dikaliumsalz des Propargylalkohols, zum 17α-(3-Hydroxyprop-1-inyl)-17β-hydroxyderivat oder mit metallierten Derivaten des 3-Hydroxypropins, zum Beispiel mit 1-Lithium-3-(tetrahydropyran-2'-yloxy)-prop-1-in-1-id, zum 17-[3-(Tetrahydropyran-2'-yloxy)-prop-1-inyl]-17β-hydroxyderivat, die anschließend zu den 17-(3-Hydroxypropyl- bzw. Hydroxypropenyl)17β-hydroxy-Verbindungen hydriert werden können. Das gelingt zum Beispiel durch Hydrierung bei Raumtemperatur und Normaldruck in Lösungsmitteln wie Methanol, Ethanol, Propanol, Tetrahydrofuran (THF) oder Essigester unter Zusatz von Edelmetall-Katalysatoren wie Platin oder Palladium.

Die Einführung homologer Hydroxyalkin-, Hydroxyalken- und Hydroxyalkangruppen erfolgt in entsprechender Weise mit Homologen des Propargylalkohols.

Die Verbindung mit der Z-konfigurierten Doppelbindung in der Hydroxypropenylgruppe entsteht durch Hydrieren der acetylenischen Dreifachbindung mit einem desaktivierten Edelmetallkatalysator (J. Fried, J.A. Edwards : Organic Reactions in Steroid Chemistry, Van Nostrand Reinhold Company 1972, Seite 134 ; und H.O. House : Modern Synthetic Reactions 1972, Seite 19). Als desaktivierte Edelmetallkatalysatoren kommen beispielsweise 10% Palladium auf Bariumsulfat in Gegenwart eines Amins oder 5% Palladium auf Calciumcarbonat unter Zusatz von Blei(II)acetat infrage. Die Hydrierung wird nach der Aufnahme von einem Äquivalent Wasserstoff abgebrochen.

Die Verbindung mit der E-konfigurierten Doppelbindung in der Hydroxypropenylgruppe entsteht durch Reduktion der acetylenischen Dreifachbindung in an sich bekannter Weise. In der Literatur sind eine ganze Reihe von Methoden zur Umwandlung von Alkinen in trans-Olefine beschrieben, beispielsweise die Reduktion mit Natrium in flüssigem Ammoniak (J. Am. Chem. Soc. 63 (1941) 216), mit Natriumamid in flüssigem Ammoniak (j. Chem. Soc. 1955, 3558), mit Lithium in niedermolekularen Aminen (J. A. Chem. Soc. 77 (1955) 3378),

mit Boranen (J. Am. Chem. Soc. 93 (1971) 3395 und 94 (1972) 6560), mit Diisobutylaluminiumhydrid und Methyl-Lithium (J. Am. Chem. Soc. 89 (1967) 5085) und insbesondere mit Lithiumaluminiumhydrid/Alkoholat (J. Am. Chem. Soc. 89 (1967) 4245). Eine weitere Möglichkeit ist die Reduktion der Dreifachbindung mit Chrom(II)-sulfat in Gegenwart von Wasser oder Dimethylformamid in schwach saurem Milieu (J. Am. Chem. Soc. 86 (1964) 4358) sowie allgemein die Reduktion durch Einwirkung von Übergangsmetallverbindungen unter Wechsel der Oxydationsstufe.

Die Einführung der Hydroxyalkene kann auch direkt erfolgen durch Addition einer entsprechenden metallierten Hydroxyalkenylverbindung, wie zum Beispiel 1-Lithium-3-(tetrahydropyran-2'-yloxy)-prop-1(E)-en (J. Org. Chem. 40 2265) oder 1-Lithium-3-(tetrahydropyran-2'-yloxy)-prop-1(Z)-en (Synthesis 1981, 999) Homologe können auf diese Art ebenfalls eingeführt werden.

Die Einfuhrung von 3-Hydroxypropan in 17-Stellung kann ebenfalls direkt durch Umsetzung des 17-Ketons mit metallierten Derivaten von 3-Halogen-propanolen-wobei die Hydroxygruppe im Metallierungsschritt als Alkoholat (Tetrahedron Letters 1978, 3013) oder als geschützte Funktion (J. Org. Chem. 37, 1947) vorliegt — zu der 17-(3-Hydroxypropyl)-17β-hydroxy-verbindung bzw. zu der an der terminalen Hydroxygruppe geschützten Verbindung erfolgen. Als Schutzgruppen kommen zum Beispiel die Ethoxyethyl-, Tetrahydropyranyl- und Methoxymethyl-Gruppen in Frage.

Werden Endprodukte der Formel I gewünscht mit $R^5/R^6$ in der Bedeutung von

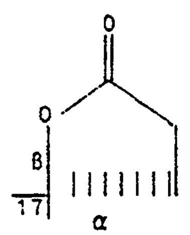

so wird die 17-(3-Hydroxypropyl)-Verbindung in an sich bekannter Weise oxydiert, zum Beispiel mit Jones' Reagenz, Braunstein, Pyridiniumdichromat, Pyridiniumchlorochromat, Chromsäure-Pyridin oder dem Fetizon-Reagenz Silbercarbonat/Celite (Compt. rend. 267 [1968] 900).

Die Darstellung von Endprodukten der Formel I mit $R^5/R^6$ in der Bedeutung von

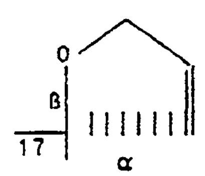

erfolgt durch Ringschlußreaktion des entsprechenden 17-(3-Hydroxyprop-1-(Z)enyl-17-β-hydroxy-Eduktes.

Der Aufbau der 17-Cyanmethylseitenkette erfolgt in an sich bekannter Weise aus dem 17-Keton zum Beispiel über das 17-Spiroepoxid und Spaltung des Spiroepoxids mit HCN gemäß Z. Chem. 18 (1978) 259-260.

Auch die Einführung der 17-Hydroxyacetylseitenkette erfolgt nach an sich bekannten Methoden, beispielsweise nach den in J. Org. Chem. 47 (1982), 2993-2995, Chem. Ber. 113 (1984), 1184 bzw. US-Patent 4.600.538 beschriebenen Methoden.

Zur Einführung der Gruppierungen

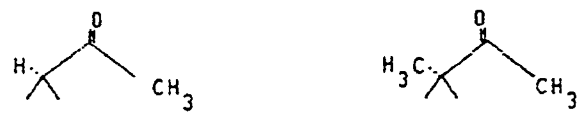

wird das 17-Keton mit Tosylmethylisocyanid (Chem. Ind. 1972 213) in die 17-Nitrilverbindung (Tetrahedron 31 (1975), 2151) überführt, das direkt mit Methyllithium oder Methylmagnesiumbromid in die 17-Acetylverbindung umgewandelt werden kann, welche nach Enolisierung mit K-tert.-Butylat in Tetrahydrofuran und Umsetzung mit Methyljodid die gewünschte 17α-Methyl-17βacylgruppierung liefert. Diese Sequenz Methyladdition an das Nitril und anschließende Alkylierung kann auch in umgekehrter Folge ausgeführt werden.

In Z bzw. V vorhandene freie Hydroxy- bzw. Hydroxy-, Mercapto- und/oder Aminogruppen können in an

sich bekannter Weise alkyliert oder acyliert werden.

In V enthaltene Sulfide und/oder Dialkylamine können durch geeignete Oxidationsmittel (zum Beispiel Wasserstoffperoxid bzw. Persäuren) in die gewünschten Sulfoxide (n = 1), N-oxide (n = 1) [siehe z.B. Kontakte (Darmstadt) 1986, 3, S. 12] bzw. Sulfone (n = 2) überführt werden.

Verbindungen mit einem Dialkylamin Substituenten in V können durch Reaktion mit Bromcyan in aprotischen Lösungsmitteln wie zum Beispiel Dioxan, Benzol oder Toluol bei erhohter Temperatur (Amin-Abbau nach Braun) analog den zum Beispiel in Org. Reactions 7, 198 (1953), K.W. Bentley, Techniques of Organic Chemistry 11, 773 (1963) und Houben Weyl, 5/4, 151 (1960) angegebenen Vorschriften in guter Ausbeute in die entsprechenden (N-Cyan-N-alkylaminoaryl)-derivate überführt werden.

Diese werden je nach der letztlich gewünschten Bedeutung von $R^{12}$ im Endprodukt in an sich bekannter Weise zu den entsprechenden Dialkylamin-Verbindungen (zum Beispiel mit Diisobutylaluminiumhydrid in Toluol zu den N-Formyl-N-alkylaminophenyl-Zwischenprodukten und anschließend mit Lithiumaluminiumhydrid) bzw. N-H-N-alkyl-Verbindungen reduziert (zum Beispiel mit Lithiumaluminiumhydrid oder mit Lithium in flüssigem Ammoniak). Die letzteren werden anschließend gewünschtenfalls in literaturbekannter Weise acyliert und gegebenenfalls anschließend in bekannter Weise mit zum Beispiel Lithiumaluminiumhydrid zum neuen Dialkylaminderivat reduziert (s. DE 3623038).

Die erhaltenen Verbindungen der allgemeinen Formel I mit X in der Bedeutung eines Sauerstoffatoms können gewünschtenfalls durch Umsetzung mit Hydroxylaminhydrochlorid in Gegenwart von tertiären Aminen bei Temperaturen zwischen −20 und +40°C in die Oxime (Formel I mit X in der Bedeutung der Hydroxyiminogruppierung N OH, wobei die Hydroxygruppe syn- oder antiständig sein kann) überführt werden. Geeignete tertiäre Basen sind beispielsweise Trimethylamin, Triäthylamin, Pyridin, N,N-Dimethylaminopyridin, 1,5-Diazabicyclo[4.3.0]nonen5 (DBN) und 1,5-Diazabicyclo[5.4.0]undecen-5 (DBU), wobei Pyridin bevorzugt ist.

Die neuen Verbindungen der allgemeinen Formel I sowie deren Additionssalze mit pharmazeutisch verträglichen Säuren sind wertvolle Pharmaka. So verfügen sie über eine starke Affinität zum Gestagenrezeptor und besitzen einen überraschend großen Bereich an gestagenen, antigestagenen, antiglucocorticoiden, antimineralcorticoiden und antiandrogenen Eigenschaften. Diese wichtigen biologischen Wirksamkeiten können für medizinische Zwecke genutzt werden.

Wirkstoffe dieser Art mit ausgeprägter antigestagener Aktivität sind zur Auslösung von Aborten geeignet, da sie das zur Aufrechterhaltung der Schwangerschaft erforderliche Progesteron vom Rezeptor verdrängen. Sie sind deshalb wertvoll und interessant im Hinblick auf ihre Verwendung zur postcoitalen Fertilitätskontrolle.

Sie können auch gegen hormonelle Unregelmäßigkeiten, zur Menstruationsauslösung und zur Geburtseinleitung eingesetzt werden.

Außerdem können sie für die Behandlung von hormonabhängigen Carcinomen eingesetzt werden.

Die erfindungsgemäßen Verbindungen der allgemeinen Formel I sowie deren Additionssalze mit pharmazeutisch verträglichen Säuren weisen auch eine antiglucocorticoide Aktivität auf und können somit auch als Arzneimittel zur Therapie corticoid-induzierter Störungen (Glaukom) sowie zur Bekämpfung von Nebenwirkungen, die bei langfristiger Behandlung mit Glucocorticoiden auftreten (Cushing-Syndrom) eingesetzt werden. Sie ermöglichen daher auch die auf eine Supersekretion der Glucocorticoide zurückzuführenden Störungen, vor allem die Adipositas, Arteriosklerose, Hypertension, Osteoporose, Diabetes sowie die Insomnie zu bekämpfen.

Die erfindungsgemäßen Verbindungen der allgemeinen Formel I sowie deren Additionssalze mit pharmazeutisch verträglichen Säuren mit gestagener Aktivität können beispielsweise bei der Therapie von Amenorrhoe, Dysmenorrhoe, Hypermenorrhoe und lutealer Insuffizienz, solche mit antimineralcorticoiden Eigenschaften zur Behandlung von Krankheitszuständen, an denen ein Hyperaldosteronismus beteiligt ist, verwendet werden.

Die erfindungsgemäßen Verbindungen der allgemeinen Formel I sowie deren Additionssalze mit pharmazeutisch verträglichen Säuren mit antiandrogener Aktivität können bei der Behandlung der Hypertrophie und des Prostatakarzinoms verwendet werden. Sie ermöglichen weiterhin eine spezifische Therapie von Androgenisierungserscheinungen bei Frauen : die pathologische Behaarung bei Hirsutismus, die androgenetische Alopezie sowie die gesteigerte Talgdrüsenfunktion bei Akne und Seborrhoe sind günstig beeinflußbar.

Die Erfindung betrifft somit auch Arzneimittel auf Basis der pharmazeutisch verträglichen, d.h. in den verwendeten Dosen nicht toxischen Verbindungen der allgemeinen Formel I sowie deren Additionssalze mit pharmazeutisch verträglichen Säuren, gegebenenfalls zusammen mit den üblichen Hilfs- und Trägerstoffen.

Die erfindungsgemäßen Verbindungen und deren Salze können nach an sich bekannten Methoden der Galenik zu pharmazeutischen Präparaten für die enterale, perkutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben, Cremes und Gelen verabreicht werden.

Der oder die Wirkstoffe können dabei mit den in der Galenik üblichen Hilfsstoffen wie z.B. Gummiarabikum,

Talk, Stärke, Mannit, Methylcellulose, Laktose, Tensiden wie Tweens® oder Myrj®, Magnesiumstearat, wäßrigen oder nichtwäßrigen Trägern, Paraffinderivaten, Netz- Dispergier-, Emulgier-, Konservierungsmitteln und Aromastoffen zur Geschmackskorrektur (z.B. ätherischen Ölen) gemischt werden.

Die Erfindung betrifft somit auch pharmazeutische Zusammensetzungen, die als Wirkstoff zumindest eine erfindungsgemäße Verbindung oder eines ihrer Additionssalze mit pharmazeutisch verträglichen Säuren enthalten.

Als Additionssalze der erfindungsgemäßen Produkte mit Säuren sind insbesondere die Hydrochloride und die Methansulfonate zu nennen.

Eine Dosiseinheit enthält etwa 1-100 mg Wirkstoff(e).

Die Dosierung der erfindungsgemäßen Verbindungen liegt beim Menschen bei etwa 1-1000 mg pro Tag.

Zur Kennzeichnung der antigestagenen Wirkung wurde die abortive Wirkung bestimmt.

Die Versuche wurden an weiblichen Ratten im Gewicht von ca. 200 g durchgeführt. Nach erfolgter Anpaarung wurde der Schwangerschaftsbeginn durch Nachweis von Spermien in Vaginalabstrichen gesichert. Der Tag des Spermiennachsweises gilt als Tag 1 der Gravidität (= d1 p.c.).

Die Behandlung der Tiere mit der jeweils zu testenden Substanz bzw. dem Lösungsmittel erfolgt nach der Nidation der Blastocysten von d5 p.c. bis d7 p.c. An d9 p.c. wurden die Tiere getötet und die Uteri auf Implantate und Resorptionsstellen hin untersucht. Von allen Uteri wurden Fotos angefertigt. Das Fehlen von Implantaten, pathologische, hämorrhagische oder sonst abnorme Nidationsstellen, wurde als Abort gewertet.

Die Testsubstanzen wurden in einem Benzylbenzoat-Rizinusöl-Gemisch (Verhältnis 1 + 4) gelöst. Das Vehikelvolumen pro Einzeldosis betrug 0,2 ml. Die Behandlung erfolgte subcutan.

Die Überlegenheit der erfindungsgemäßen Verbindungen soll durch Vergleich der abortiven Wirksamkeit von 17α-(Prop-1-inyl)-17βhydroxy11β,19-(4-dimethylamino-o-phenylen)-4-androsten-3-on (A), 17α-(Prop-1inyl)-17β-hydroxy-11β,19-(4-acetyl-o-phenylen)-4-androsten-3-on (B), 17α-(3-Hydroxyprop-1-(Z)-enyl)-17β-hydroxy-11β,19-(4-dimethylamino-ophenylen)-4-androsten-3-on (C) und 17α-(3-Hydroxyprop-1-(Z)-enyl)17β-hydroxy-11β,19-(4-methylthio-o-phenylen)-4-androsten-3-on mit dem in der Europäischen Patentschrift 0057115 beschriebenen 11β-(4-Dimethylaminophenyl)-17β-hydroxy-17α-(propin-1-yl)-4,9(10)-estradien-3-on (E) und dem aus der Europäischen Patentanmeldung 84730147.0 hervorgehenden 11β-(4-Dimethylaminophenyl)-17β-hydroxy-17α-(3-hydroxyprop-1-(Z)enyl)-4,9(10)-estradien-3-on (F) gezeigt werden.

Aus Tabelle 1 ist zu entnehmen, daß nur die erfindungsgemäßen Verbindungen A-D und die ebenfalls auf die Anmelderin züruckgehende Verbindung F bei einer Dosis von 1.0 mg/d s.c. noch voll abortiv wirksam sind. Die Vergleichssubstanz E zeigt bei dieser Dosis nur noch 50% Wirksamkeit. Die erfindungsgemäße Verbindung B zeigt selbst bei einer Dosis von 0,3 mg/d s.c. noch volle Wirksamkeit, während F bei dieser Dosis keine Wirksamkeit mehr besitzt.

Tabelle 1

Abortivtest bei der graviden Ratte

Behandlung von d5 p.c. bis d7 p.c., Autopsie an d9 p.c.

| Verbindung | Dosis mg/Tier/Tag s.c. | Abortrate n Abort / n Gesamt | % |
|---|---|---|---|
| A | 3.0 | 4/4 | (100) |
| | 1.0 | 4/4 | (100) |
| | 0.3 | 1/4 | ( 25) |
| B | 3.0 | 4/4 | (100) |
| | 1.0 | 4/4 | (100) |
| | 0.3 | 4/4 | (100) |
| C | 3.0 | 4/4 | (100) |
| | 1.0 | 4/4 | (100) |
| | 0.3 | 0/4 | ( 0) |
| D | 3.0 | 4/4 | (100) |
| | 1.0 | 4/4 | (100) |
| | 0.3 | 1/4 | ( 25) |
| E | 3.0 | 4/4 | (100) |
| | 1.0 | 2/4 | ( 50) |
| F | 3.0 | 4/4 | (100) |
| | 1.0 | 4/4 | (100) |
| | 0.3 | 0/4 | ( 0) |
| Lösungsmittel als Kontrolle: | | | |
| 0.2 ml Benzylbenzoat + Rizinusöl (1+4) | - | 0/5 | ( 0) |

n = 4 Ratten

Zur Bewertung der antiglucocorticoiden Aktivität wurde stellvertretend für alle erfindungsgemäßen Verbindungen der allgemeinen Formel I mit 17α-(3-Hydroxy-prop-1-(Z)-enyl)-17β-hydroxy-11β,19-(4-dimethylamino-o-phenylen)-4-androsten-3-on (C) der Anti-Thymolyse-Test bei der Ratte durchgeführt und das Ergebnis wiederum den Vergleichssubstanzen E und F gegenübergestellt.

Unter dem Einfluß von Glucocorticoiden kommt es bei der Ratte zu einer starken Gewichtsabnahme des Thymus (= thymolytischer Effekt). Bei gleichzeitiger Verabreichung von Substanzen mit glucocorticoid-antagonistischen Eigenschaften kann eine Hemmung bzw. Aufhebung der glucocorticoid-induzierten Thymussuppression erwartet werden.

Die Versuche wurden an adrenalektomierten, juvenilen männlichen Ratten im Gewicht von 100-130 g durchgeführt. Haltungsbedingungen : konventionell, Beleuchtungsrhythmus : 10 Stunden Dunkelheit/14 Stunden Helligkeit, Durchnittstemperatur 20 ± 2°C, Ratten-Standarddiät (Pellets), Versorgung mit Leitungswasser und 0,9%iger NaCl-Lösung über separate Trinkflaschen.

Für die subcutane Applikation wurden die Substanzen in einem Gemisch aus Benzylbenzoat + Rizinusöl

(Verhältnis 1 + 4) gelöst und die jeweilige Tagesdosis in einem Vehikelvolumen von 0,2 ml injiziert. Die gewählten Dosierungen gehen aus Tabelle 2 hervor.

Als Glucocorticoid-Standardsubstanz wurde Dexamethason in einer Dosis von 0,01 mg/Tier/Tag s.c. verwendet. Diese Dosierung induziert — bezogen auf die Lösungsmittelkontrolle — eine ca. 75%ige Reduktion des Thymusdrüsengewichts. Lösungsmittel : Benzylbenzoat/Rizinusöl (1 + 4), Vehikelvolumen pro Tagesdosis : 0,2 ml.

Ca. 5 Tage vor Behandlungsbeginn werden die Tiere in Äthernarkose adrenalektomiert. Ihre Zuordnung zu den verschiedenen Versuchsgruppen erfolgt zufällig ; der Stichprobenumfang ergibt sich aus der Tabelle 2.

Behandlungsgruppen : Dexamethason-Kontrolle
Lösungsmittel-Kontrolle
Testsubstanz-Dosis + Dexamethason.

Die Behandlungsdauer betrug 4 Tage (Tag 1-4). Am Tag 5 wurden die Tiere mit $CO_2$ getötet. Das Gewicht des Thymus wurde ermittelt und auf mg/100 g Körpergewicht umgerechnet.

Zur Beurteilung der antiglucocorticoiden Wirkung einer Substanz wird die Differenz zwischen Lösungsmittelkontrolle und Dexamethason (0.01 mg/Tier/Tag s.c.) gleich 100% gesetzt.

Aus den Stichprobenmittelwerten wird dann eine mittlere prozentuale antiglucocorticoide Wirkung (Aufhebung der von Dexamethason induzierten Thymussuppression in %) berechnet durch

$$A = \frac{MW_{s} - MW_{Dexa}}{MW_{Lös.} - MW_{Dexa}} \cdot 100$$

Hierbei ist

$$\left.\begin{array}{l} MW_{s} \\ MW_{Dexa} \\ MW_{Lös.} \end{array}\right\} = \text{Mittelwert für} \left\{\begin{array}{l} \text{Substanz-Dosis + Dexamethason} \\ \text{Dexamethason} \\ \text{Lösungsmittelkontrolle} \end{array}\right.$$

Wie sich aus Tabelle 2 ergibt, bewirkt Verbindung C nur in der geprüften Höchstdosis von 30,0 mg/d s.c. eine geringgradige Aufhebung der Dexamethasoninduzierten Thymussupression. Bei niedrigeren Dosen (3.0; 10,0 mg/d s.c.) konnte kein antiglucocorticoider Effekt festgestellt werden.

Im Vergleich zu den Verbindungen E (Abbildung 2) und F (Abbildung 1) ist also die antiglucocorticoide Aktivität von Verbindung C deutlich reduziert.

Aus der EP-A-0188396 sind zwar strukturähnliche Steroide mit einem substituierten Arylrest in der 10-Stellung und einer 9(11)-Doppelbindung bekannt ; die bekannten Verbindungen weisen aber in der 17α-Position in jedem Fall eine Alkyl-, Alkenyl- oder Alkinylgruppe auf. Diese Verbindungen besitzen jedoch eine beträchtliche antiglucocorticoide Aktivität, während ihre Aktivität gegenüber dem Progesteronrezeptor, und somit ihre antigestagene Wirksamkeit, vernachlässigbar ist.

Mit den erfindungsgemäßen Verbindungen wurden somit Substanzen zur Verfügung gestellt, die ein neues Wirkungsprofil gegenüber dem nächstkommenden Stand der Technik besitzen, nämlich eine wesentlich erhöhte antigestagene Wirksamkeit bei nur mäßiger antiglucocorticoider Aktivität.

Tabelle 2

**THYMOLYSE-TEST AUF ANTIGLUCOCORTICOIDE WIRKUNG**

Aufhebung der Dexamethason-induzierten Thymus-Suppression
(Behandlung adrenalektomierter männlicher Ratten (KG 100 - 130g) über 4 Tage, Autopsie an Tag 5)

| Dexa-methason (mg / d s.c.) | Verbindung C (mg / d s.c.) | n | rel. Thymus-Gew. (mg/100g KG) M ± SD | Aufhebung % | Dexa-methason (mg / d s.c.) | Verbindung F (mg / d s.c.) | n | rel. Thymus-Gew. (mg/100g KG) M ± SD | Aufhebung % | Dexa-methason (mg / d s.c.) | Verbindung E (mg / d s.c.) | n | rel. Thymus-Gew. (mg/100g KG) M ± SD | Aufhebung % |
|---|---|---|---|---|---|---|---|---|---|---|---|---|---|---|
| a) - | - | 7 | 361,0±51,5 | | a) - | - | 12 | 419,8±61,9 | | a) - | - | 24 | 385,4±51,3 | |
| 0,01 | - | 7 | 77,4± 7,8 | | 0,01 | - | 12 | 91,2±16,6 | | 0,01 | - | 18 | 87,2±13,2 | |
| 0,01 | 3,0 | 7 | 72,4±10,9 | - 1,8(-12,1- 7,5) | 0,01 | 3,0 | 6 | 110,6±16,3 | 5,9(- 4,1-15,4) | 0,01 | 3,0 | 6 | 125,6±19,9 | 12,9( 0,5-24,6) |
| 0,01 | 10,0 | 7 | 76,9± 6,6 | - 0,2(-10,4- 9,1) | 0,01 | 10,0 | 6 | 194,4±27,9 | 31,4( 22,1-40,5) | 0,01 | 10,0 | 6 | 178,1±44,8 | 30,5(18,6-42,0) |
| 0,01 | 30,0 | 7 | 125,0±16,4 | 16,8( 7,5-25,5) | 0,01 | 30,0 | 6 | 197,3±49,6 | 32,3( 23,0-41,3) | 0,01 | 30,0 | 6 | 264,7±41,0 | 59,5(48,0-71,0) |

a) Kontrollgruppe: Benzylbenzoat + Rizinusöl (1+4) 0,4 ml/d s.c.

() = 95% Konfidenzintervall für % Aufhebung

n = Stichprobenumfang

In den nachstehenden Beispielen wird die Chromatographie, sofern nicht anders erwähnt, jeweils mit einem Gemisch aus Essigester und Hexan durchgeführt.

**BEISPIEL 1**

17β-Hydroxy-11β, 19-(o-phenylen)-4-androsten-3-on

a) 19-(2-Chlorphenyl)-3, 3-(2, 2-dimethyltrimethylendioxy)-9(11)-androsten-5α, 17β-diol

4,9 g Magnesiumspäne werden bei Raumtemperatur unter Schutzgas in 40 ml abs. Diethylether vorgelegt und zunächst mit 0,5 ml 2-Chlorbenzylchlorid und dann vorsichtig mit 0,4 ml 1,2-Dibromethan versetzt. Nach erfolgtem Start der Reaktion wird anschließend die restliche Menge (18,4 ml) des 2-Chlorbenzylchlorids gelöst in 135 ml abs. Diethylether über 40 Minuten hinweg zugetropft, ohne daß die Innentemperatur im Reaktionsgefäß über 30°C steigt. Nach erfolgter Bildung des Grignard-Reagenzes wird das Reaktionsgemisch auf 0°C abgekühlt und 5α,10α-Epoxy-3,3-(2,2-dimethyltrimethylendioxy)-9(11)-estren-17β-ol (13,6 g) gelöst in 75 ml abs. Tetrahydrofuran langsam zugetropft. Nach einstündigem Nachrühren bei Eisbadtemperatur wird über Nacht das Reaktionsgemisch langsam auf Raumtemperatur erwärmt und anschließend auf verdünnte Ammoniumchloridlösung gegossen. Die wäßrige Phase wird mehrmals mit Essigester extrahiert. Die vereinigten organischen Phasen werden mit Natriumchloridlösung neutralgewaschen, mit Natriumsulfat getrocknet und im Vakuum eingeengt. Der Rückstand wird an Aluminiumoxid (neutral, Stufe III) chromatographiert. Es werden 14,8 g obiger Verbindung erhalten.
$[\alpha]_D^{22} = -2°$ ($CHCl_3$) ; c = 0,51)
Fp. : 188-191°C (Ethylacetat)

b) 11β, 19-(o-Phenylen)-3, 3-(2, 2-dimethyltrimethylendioxy)-androstan-5α, 17β-diol

Bei −65°C werden 600 ml wasserfreier Ammoniak unter Feuchtigkeitsausschluß in den Reaktionskolben einkondensiert und mit 970 mg frisch geschnittenen Lithiumspänen versetzt. Unmittelbar nach Auftreten der charakteristischen Blaufärbung wird eine Lösung von 14 g des unter a) erhaltenen Produkts in 450 ml abs. Tetrahydrofuran derart zugetropft, daß ein Wechselspiel zwischen Entfärbung der Reaktionslösung und Blaufärbung entsteht. Nach erfolgter Zugabe wird das überschüssige Lithium durch Zutropfen von Ethanol beseitigt, der überwiegende Teil des Ammoniaks durch Abdampfen entfernt und das Reaktionsgemisch auf Wasser gegossen. Die wäßrige Phase wird mit Essigester extrahiert. Die vereinigten organischen Phasen werden anschließend mit Natriumchloridlösung gewaschen, mit Natriumsulfat getrocknet und im Vakuum eingeengt. Es werden 13,9 g Rohprodukt isoliert. Chromatographie an Aluminiumoxid (neutral, Stufe III) ergibt 10,3 g obiger Verbindung.
$[\alpha]_D^{22} = +13°$ ($CHCl_3$) ; c = 0,52)
Fp. : = 164-167°C (Ethylacetat)

$^1$H-NMR ($CDCl_3$) [δ] : 7,0-7,45 (4H, m, aromatische Protonen) ; 3,13 (1H, d J = 16 Hz, Proton an C-19) ; 2,68 (1H, d J = 16 Hz, Proton an C-19) ; 0,98 (3H, s, Protonen einer Ketalmethylgruppe) ; 0,95 (3H, s, Protonen einer Ketalmethylgruppe) ; 0,25 (3H, s, Protonen an C-18).

Die unter b) angegebene Titelverbindung kann auch auf folgendem Wege hergestellt werden :

α) 19-(2-Bromphenyl)-3, 3-(2, 2-dimethyltrimethylendioxy)-9(11)-androsten-5α, 17β-diol

Analog Beispiel 1 a) werden 5 g 5α,10α-Epoxy-3,3-(2,2-dimethyltrimethylendioxy)-9(11)-estren-17β-ol mit 26,7 g 2-Brombenzylbromid umgesetzt. Nach der Chromatographie werden 5,9 g obiger Verbindung als weißer Schaum isoliert.

$^1$H-NMR ($CDCl_3$) [δ] : 6,95-7,55 (4H, m, Protonen am Aromaten) ; 5,45 (1H, s breit, Proton an C-11) ; 3,7-3,82 (1H, m, Proton an C-17) ; 3,4-3,6 (4H, m, Protonen der Ketalmethylgruppen) ; 3,16 und 3,07 (jeweils [1H, d mit der Aufspaltung von 15 Hz], A,B-System der Protonen an C-19) ; 0,98 (3H, s, Protonen einer Ketalmethylgruppe) ; 0,9 (3H, s, Protonen einer Ketalmethylgruppe) ; 0,55 (3H, s, Protonen an C-18).

β) 11β, 19-(o-Phenylen)-3, 3-(2, 2-dimethyltrimethylendioxy)-androstan-5α, 17β-diol

2,5 g der unter α) hergestellten Verbindung werden in 250 ml absolutem Toluol gelöst, mit 2,25 ml Tributylzinnhydrid und 250 mg α,α-Azoisobutyronitril versetzt und 3 Stunden zum Rückfluß erhitzt. Anschließend

wird das Solvens am Vakuum entfernt, der Rückstand in Tetrahydrofuran aufgenommen und mit 50 ml gesättigter wäßriger Kaliumfluoridlösung 1 Stunde gerührt. Dann wird die wäßrige Phase mit Essigester extrahiert und verworfen. Die organischen Phasen werden vereinigt, über Natriumsulfat getrocknet und im Vakuum eingeengt. Der Rückstand wird an Aluminiumoxid (neutral, Stufe III) chromatographiert. Man erhält 1,75 g der Titelverbindung.

Die unter b) angegebene Titelverbindung kann auch aus folgender Verbindung hergestellt werden :

γ) 19-(2-Fluorphenyl)-3, 3-(2, 2-dimethyltrimethylendioxy)-9(11)-androsten-5α, 17β-diol

Analog Beispiel 1 a) werden 750 mg 5α,10α-Epoxy-3,3-(2,2-dimethyltrimethylendioxy)-9(11)-estren-17β-ol mit 2 g 2-Fluorbenzylchlorid umgesetzt. Nach Chromatographie werden 798 mg obiger Verbindung als weißer Schaum isoliert.

$^1$H-NMR ($CD_2Cl_2$) [δ] : 6,92-7,33 (4H, m, Protonen am Aromaten) ; 5,09 (1H, m, Proton an C-11) ; 3,62-3,72 (1H, m, Proton an C-17) ; 3,45-3,58 (4H, m, Protonen der Ketalmethylengruppen) ; 2,97 und 2,9 (jeweils [1H, d mit der Aufspaltung von 15 Hz], A,B-System der Protonen an C-19) ; 0,99 (3H, s, Protonen einer Ketalmethylgruppe) ; 0,9 (3H, s, Protonen einer Ketalmethylgruppe) ; 0,61 (3H, s, Protonen an C-18).

δ) 11β, 19-(o-Phenylen)-3, 3-(2, 2-dimethyltrimethylendioxy)-androstan-5α, 17β-diol

Analog Beispiel 1 b) werden 750 mg 19-(2-Fluorphenyl)-3,3-(2,2-dimethyltrimethylendioxy)-9(11)-androsten-5α,17β-diol mit 60 mg Lithium umgesetzt. Nach Chromatographie werden 585 mg obiger Verbindung als weißer Schaum isoliert.

c) 17β-Hydroxy-11β, 19-(o-phenylen)-4-androsten-3-on

2 g des unter b) hergestellten Produkts werden in 100 ml Aceton gelöst und mit 5 ml 4 n Salzsäure versetzt. Nach 4 stündigem Nachrühren bei Raumtemperatur wird das Reaktionsgemisch auf gesättigte Natriumhydrogencarbonatlösung gegossen und die wäßrige Phase mit Methylenchlorid extrahiert. Die vereinigten organischen Phasen werden über Natriumsulfat getrocknet und im Vakuum eingeengt. Der Rückstand wird über Kieselgel chromatographiert. Es werden 1,13 g obiger Verbindung erhalten.
$[\alpha]_D^{22}$ = +84° ($CHCl_3$ ; c = 0,5)

$^1$H-NMR ($CDCl_3$) [δ] : 7-7,5 (4H, m, Protonen am Aromaten) ; 5,88 (1H, s, Proton an C-4) ; 3,68 (1H, tr J = 9 Hz, Proton an C-17) ; 3,3 (1H, m Proton an C-11) ; 3,26 (1H, d J = 17 Hz, Proton an C-19) ; 2,74 (1H, d $J_1$ = 17 Hz, Proton an C-19) ; 0,29 (3H, s, Protonen an C-18).

**BEISPIEL 2**

17β-Hydroxy-17-(prop-1-inyl)-11β, 19-(o-phenylen)-4-androsten-3-on

a) 11β, 19-(o-Phenylen)-5α-hydroxy-3, 3-(2, 2-dimethyltrimethylendioxy)-androstan-17-on

Zu einem Gemisch aus 34,3 ml Pyridin und 287 ml Methylenchlorid werden bei 0°C portionsweise 11,28 g Chromtrioxid zugegeben. Anschließend wird das unter Beispiel 1 b) (8 g) erhaltene Steroid gelöst in 50 ml Methylenchlorid, langsam bei der gleichen Temperatur zum Reaktionsgemisch zugetropft und dieses weitere zwei Stunden bei Eisbadtemperatur nachgerührt. Nach Beendigung des Rührens läßt man die festen Reaktionsbestandteile sich absetzen, dekantiert die überstehende Phase ab und wäscht den Niederschlag mehrmals kräftig mit Methylenchlorid aus. Die vereinigten organischen Phasen werden durch Waschen mit wäßriger 0,5 m Kaliumhydroxidlösung von restlichen anorganischen Bestandteilen befreit, mit Wasser neutral gewaschen, über Natriumsulfat getrocknet und im Vakuum eingeengt. Es werden 7 g rohes (11β,19-(o-Phenylen)-5α-hydroxy-3,3-(2,2-dimethyltrimethylendioxy)-androstan-17-on isoliert, dessen Reinheit für weitere Reaktionen ausreichend ist (siehe unten). 500 mg werden für analytische Zwecke durch Chromatographie an Aluminiumoxid (neutral, Stufe III) gereinigt. Es werden 432 mg des gewünschten Produkts isoliert.
$[\alpha]_D^{22}$ = +31° ($CHCl_3$ ; c = 0,505)
Fp. : = 206-210°C (Ethylacetat)

b) 17-(Prop-1-inyl)-11β, 19-(o-phenylen)-3, 3-(2, 2-dimethyltrimethylendioxy)-androstan-5α, 17β-diol

225 ml abs. Tetrahydrofuran werden bei 0°C mit Propin gesättigt. Anschließend wird zu dieser Lösung 1,6 m n-Butyllithiumlösung (Hexan) (27,7 ml) langsam ohne größere Temperaturerhöhung zugetropft. Nach fünfzehnminütigem Nachrühren tropft man langsam eine Lösung von 2 g des unter a) hergestellten Rohprodukts in 45 ml abs. Tetrahydrofuran zu dieser Reaktionsmischung und läßt 30 Minuten nachrühren. Danach wird das Reaktionsgemisch auf Wasser gegossen, die wäßrige Phase mit Essigester extrahiert und die vereinigten organischen Phasen werden mit Natriumchloridlösung gewaschen.

Trocknen über Natriumsulfat und Einengen im Vakuum führen zu 2,44 g Rohprodukt. Durch Chromatographie an Aluminiumoxid (neutral, Stufe III) werden 1,8 g obiger Verbindung erhalten.
IR (KBr) : 2230 $cm^{-1}$ Dreifachbindung

c) 17β-Hydroxy-17-(prop-1-inyl)-11β, 19-(o-phenylen)-4-androsten-3-on

Analog der unter Beispiel 1 c) beschriebenen Sauren Spaltung werden 1,5 g des unter b) erhaltenen Produkts zu 738 mg der Titelverbindung umgesetzt.

**BEISPIEL 3**

17β-Hydroxy-17-(prop-1-inyl)-11β, 19-(o-phenylen)-4, 6-androstadien-3-on

a) 11β, 19-(o-Phenylen)-4-androsten-3, 17-dion

20 g des nach der Sequenz Beispiel 1 a), Beispiel 1 b) und Beispiel 2 a) erhaltenen Produkts werden analog der Vorschrift Beispiel 1 c) zu 8,69 g der Titelverbindung gespalten.
$[\alpha]_D^{22}$ = +116° ($CHCl_3$ ; c = 0,51)
Fp. : = 284-288°C

b) 11β, 19-(o-Phenylen)-3-ethoxy-3, 5-androstadien-17-on

8 g des unter a) erhaltenen Produkts werden in einem Gemisch aus 85 ml abs. Methylenchlorid, 25 ml Ethanol und 6,7 ml Orthoameisensäuretriethylester vorgelegt und bei 0°C mit 170 mg p-Toluolsulfonsäure (Monohydrat) versetzt. Anschließend wird bei Eisbadtemperatur über Nacht nachgerührt, dann mit einem Überschuß an Natriumhydrogencarbonatlösung versetzt und die wäßrige Phase mit Methylenchlorid extrahiert. Die vereinigten organischen Phasen werden mit gesättigter Natriumchloridlösung gewaschen, über Natriumsulfat getrocknet und im Vakuum eingeengt. Es werden 11,3 g verunreinigtes Rohprodukt erhalten. Kristallisation aus Ethanol (versetzt mit einigen Tropfen Pyridin) führt zu 5,43 g kristalliner Titelverbindung.
$[\alpha]_D^{22}$ = +39° ($CHCl_3$ ; c = 0,5)
Fp. = 182-186°C.

c) 17-(Prop-1-inyl)-11β, 19-(o-phenylen)-3-ethoxy-3, 5-androstadien-17β-ol

5 g des nach b) erhaltenen Produkts werden analog Beispiel 2 b) zu 5,4 g Rohprodukt, dessen Reinheit für weitere Umsetzungen ausreichend ist, umgesetzt. Kristallisation von 400 mg des Rohprodukts aus Ethanol führt zu 268 mg kristalliner Titelverbindung.
$[\alpha]_D^{22}$ = −91° ($CHCl_3$ ; c = 0,5)
Fp. = +203-207°C.

d) 17-(Prop-1-inyl)-17β-hydroxy-11β, 19-(o-phenylen)-4, 6-androstadien-3-on

5 g des nach c) erhaltenen Rohprodukts werden in einem Gemisch aus 50 ml 80%iger wäßriger Dioxanlösung und 24 ml 10%iger wäßriger Natriumacetatlösung suspendiert. Zu dieser Suspension werden 1,6 g 1,3-Dibrom-5,5-dimethylhydantoin portionsweise bei 0°C zugegeben. Dabei geht das Steroid langsam in Lösung. Nach zweistündiger Reaktionszeit wird das Reaktionsgemisch auf Wasser gegossen und die wäßrige Phase mit Methylenchlorid extrahiert. Die vereinigten organischen Phasen werden mit gesättigter Natriumthiosulfatlösung und Wasser gewaschen, über Natriumsulfat getrocknet und im Vakuum eingeengt. Das so erhaltene verunreinigte 17-(Prop-1-inyl)-17β-hydroxy-11β,19-(o-phenylen)-6β-brom-4-androsten-3-on wird in 48 ml abs.

Dimethylformamid gelöst, unter Schutzgas mit 2,4 g Lithiumbromid und 1,65 g Lithiumcarbonat versetzt und eine Stunde bei 100°C gerührt. Nach Abkühlen der Reaktionsmischung auf Raumtemperatur wird sie auf Wasser gegossen, die wäßrige Phase mit 4 n Salzsäure neutralisiert, auf Eisbadtemperatur gekühlt, eine Stunde bei dieser Temperatur nachgerührt und das ausgefallene Steroid abgesaugt. Es werden 4,14 g leicht verunreinigtes Rohprodukt erhalten, dessen Reinheit für weitere Umsetzungen ausreicht. Kristallisation aus Diisopropylether führt ausgehend von 1,14 g Rohprodukt zu 638 mg obiger Verbindung.
$[\alpha]_D^{22}$ = +80° ($CHCl_3$ ; c = 0,5)
Fp. = 215-217°C.

**BEISPIEL 4**

17-(Prop-1-inyl)-17β-hydroxy-11β, 19-(4-methoxy-o-phenylen)-4-androsten-3-on

a) 19-(2-Chlor-5-methoxyphenyl)-3, 3-(2, 2-dimethyltrimethylendioxy)-9(11)-androsten-5α, 17β-diol

Analog der Vorschrift von Beispiel 1 a) werden ausgehend von 15 g 5α,10α-Epoxy-3,3-(2,2-dimethyltrimethylendioxy)-9(11)-estren-17β-ol durch Umsetzung mit 2-Chlor-5-methoxybenzylchlorid 15,5 g obiger Verbindung erhalten.

b) 11β, 19-(4-Methoxy-o-phenylen)-3, 3-(2, 2-dimethyltrimethylendioxy)-androstan-5α, 17β-diol

Analog der Vorschrift von Beispiel 1 b) werden ausgehend von 15 g der unter a) erhaltenen Verbindung 11,6 g der Titelverbindung als weißer Schaum hergestellt.
$[\alpha]_D^{22}$ = +21,1° ($CHCl_3$ ; c = 0,52)
Fp. : = 223-224°C (Diisopropylether)
Die Titelverbindung b) kann auch nach folgendem Syntheseweg hergestellt werden :

α) 19-(2-Brom-5-methoxyphenyl)-3, 3-(2, 2-dimethyltrimethylendioxy)-9(11)-androsten-5α, 17β-diol

150 g 2-Brom-5-methoxy-benzylbromid werden in 1 l abs. Diethylether unter Schutzgas suspendiert und mit 13,3 g Magnesiumspänen versetzt. Nachdem die Grignard-Reaktion angesprungen ist, wird die Reaktionstemperatur durch Kühlung unter 30°C gehalten. Nach vollständiger Bildung des Grignard-Reagenzes werden 50 g 5α,10α-Epoxy-3,3-(2,2-dimethyltrimethylendioxy)-9(11)-estren-5α,17β-diol gelöst, in 330 ml abs. Tetrahydrofuran unter Rühren zugetropft. Anschließend wird die Reaktionsmischung 1,5 Stunden nachgerührt und wie unter Beispiel 1 a) beschrieben aufgearbeitet. Nach Chromatographie werden 66,5 g obiger Verbindung als weißer Schaum isoliert.
Fp. : 128-130°C (Diisopropylether/Hexan).

β) 11β, 19-(4-Methoxy-o-phenylen)-3, 3-(2, 2-dimethyltrimethylendioxy)-androstan-5α, 17β-diol

Analog Beispiel 1 β) werden 66 g 19-(2-Brom-5-methoxyphenyl)-3,3-(2,2-dimethyltrimethylendioxy)-9(11)-androsten-5α,17β-diol in 1,7 l absolutem Toluol mit 34 ml Tributylzinnhydrid unter Verwendung von 660 mg 2,2-Azoisobuttersäurenitril als Radikalstarter umgesetzt. Nach vollständigem Umsatz wird das Lösungsmittel am Vakuum abgezogen und der Rückstand aus Diisopropylether kristallisiert. Es werden 49 g obiger Verbindung kristallin erhalten.

c) 11β, 19-(4-Methoxy-o-phenylen)-5α-hydroxy-3, 3-(2, 2-dimethyltrimethylendioxy) androstan-17-on

Analog Beispiel 2 a) werden 11 g der nach b) erhaltenen Verbindung in die entsprechende Ketoverbindung überführt. Es werden 9,53 g obiger Verbindung als weißer Schaum erhalten.
$[\alpha]_D^{22}$ = +33° ($CHCl_3$ ; c = 0,55)
Fp. : = 235-238°C

d) 17-(Prop-1-inyl)-11β, 19-(4-methoxy-o-phenylen)-3, 3-(2, 2-dimethyltrimethylendioxy)-androstan-5α, 17β-diol

Analog Beispiel 2 b) werden 4 g der unter c) erhaltenen Verbindung in die entsprechende 17α-Propinyl-Verbindung überführt. Es werden nach der Chromatographie 3,3 g obiger Verbindung als weißer Schaum isoliert.
IR (KBr) : 2230 $cm^{-1}$ Dreifachbindung

e) 17-(Prop-1-inyl)-17β-hydroxy-11β, 19-(4-methoxy-o-phenylen)-4-androsten-3-on

Analog Beispiel 1 c) werden 3 g der unter d) erhaltenen Verbindung zur entsprechenden 4-En-ketoverbindung umgesetzt. Es werden 1,5 g Titelverbindung als weißer Schaum isoliert.
$[\alpha]_D^{22}$ = +18° ($CHCl_3$ ; c = 0,465).

**BEISPIEL 5**

17-(3-Hydroxyprop-l(Z)-enyl)-17β-hydroxy-11β, 19-(4-methoxy-o-phenylen)-4-androsten-3-on

a) 17-[3-(Tetrahydropyran-2-yloxy)-prop-1-inyl]-11β, 19-(4-methoxy-o-phenylen)-3, 3-(2, 2-dimethyltrimethylendioxy)-androstan-5α, 17β-diol

Zu einer Lösung aus 5,7 g 3-(Tetrahydropyran-2-yloxy)-prop-1-in in 100 ml abs. Tetrahydrofuran werden unter Schutzgas 28,3 ml einer 15%igen Lösung von n-Butyllithium in Hexan bei 0°C langsam zugetropft. Anschließend wird 15 Minuten bei 0°C nachgerührt und dann eine Lösung von 4 g des unter Beispiel 4 c) erhaltenen Produkts in 60 ml abs. Tetrahydrofuran bei 0 bis +5°C zugetropft. Man rührt anschließend 3 Stunden bei Raumtemperatur, gießt danach auf Eiswasser und extrahiert mit Essigester. Nach Trocknen der organischen Phase über Natriumsulfat und Einengen im Vakuum wird das Rohprodukt an Aluminiumoxid (neutral, Stufe III) chromatographiert. Es werden 4,36 g obiger Verbindung als weißer Schaum erhalten.
Fp. : = 150-153°C (Diisopropylether) [als 1 : 1 Epimerengemisch bezüglich des Tetrahydropyranylethers]

b) 17-[3-(Tetrahydropyran-2-yloxy)-prop-1(Z)-enyl]-11β, 19-(4-methoxy-o-phenylen)-3, 3-(2, 2-dimethyltrimethylendioxy)-androstan-5α, 17β-diol

Eine Lösung von 4 g des unter a) erhaltenen Produkts in 75 ml Tetrahydrofuran wird nach Zusatz von 5 ml Pyridin und 400 mg Palladium/Bariumsulfat (10% Pd) bei Raumtemperatur und Normaldruck hydriert. Nach Stillstand der Wasserstoffaufnahme filtriert man vom Katalysator ab und engt das Filtrat ein. Es werden 3,91 g obiger Verbindung als gelblicher Schaum erhalten.

c) 17-[3-Hydroxyprop-1(Z)-enyl]-17β-hydroxy-11β, 19-(4-methoxy-o-phenylen)-4 androsten-3-on

Analog Beispiel 1 c) werden 3,5 g der unter b) dargestellten Verbindung gespalten. Es werden 1,5 g Titelverbindung als weißer Schaum isoliert.
$[\alpha]_D^{22}$ = +60° ($CHCl_3$ ; c = 0,5)

**BEISPIEL 6**

17-(Cyanomethyl)-17β-hydroxy-11β, 19-(4-methoxy-o-phenylen)-4-androsten-3-on

a) 11β, 19-(4-Methoxy-o-phenylen-3, 3-(2, 2-dimethyltrimethylendioxy)-androstan-[17(β-1′)-spiro-3′]-oxiran-5α-ol

Unter Schutzgas werden 1 g des nach Vorschrift 4 c) erhaltenen Ketons in 20 ml absolutem Dimethylformamid gelöst und bei 0°C nacheinander mit 2,04 g Trimethylsulfoniumjodid und 1,40 g Kaliumtertiärbutylat versetzt. Anschließend wird das Reaktionsgemisch langsam unter Rühren über Nacht auf Raumtemperatur erwärmt, dann auf gesättigte Ammoniumchloridlösung gegossen und die wäßrige Phase mit Essigester mehrmals extrahiert. Die vereinigten organischen Phasen trocknet man über Natriumsulfat, engt sie am Vakuum ein und chromatographiert den Rückstand an Aluminiumoxid (neutral/Stufe III). Es werden 895 mg obiger Verbindung als weißer Schaum isoliert.

b) 17-Cyanomethyl-11β, 19-(4-methoxy-o-phenylen)-3, 3-(2, 2-dimethyltrimethylendioxy)-androstan-5α, 17β-diol

Unter Schutzgas werden 850 mg des unter a) hergestellten Epoxids in 17 ml absolutem Ethanol gelöst und mit einer Lösung von 1,7 g Kaliumcyanid in 3,4 ml Wasser versetzt. Das Reaktionsgemisch wird anschließend über Nacht auf 50°C erwärmt, dann auf Eiswasser gegossen und die wäßrige Phase mehrmals mit Essigester extrahiert. Die vereinigten organischen Phasen werden über Natriumsulfat getrocknet und am Vakuum zur Trockne eingeengt. Der Rückstand wird an Aluminiumoxid (neutral/Stufe III) chromatographiert. Man isoliert 815 mg obiger Verbindung.
IR (KBr) : 2250 $cm^{-1}$ C≡N Dreifachbindung.

c) 17-Cyanomethyl-17β-hydroxy-11β, 19-(4-methoxy-o-phenylen)-4-androsten-3-on

800 mg der unter b) erhaltenen Verbindung werden analog Beispiel 1 c) zur entsprechenden 4-En-3-ketoverbindung umgesetzt. Es werden 575 mg Titelverbindung isoliert.
$[\alpha]_D^{22}$ = 59° ($CHCl_3$ ; c = 0,505)
Fp. = 155-156°C (Essigester/Hexan)

**BEISPIEL 7**

17-(Prop-1-inyl)-17β-hydroxy-11β, 19-(4-methoxy-o-phenylen)-4, 6-androstadien-3-on

a) 11β, 19-(4-Methoxy-o-phenylen)-4-androsten-3, 17-dion

Analog Beispiel 1 c) werden 11,2 g der nach Vorschrift c) in Beispiel 4 erhaltenen Substanz zur entsprechenden 4-En-3-Ketoverbindung umgesetzt. Es werden 7,6 g obiger Verbindung isoliert.
$[\alpha]_D^{22}$ = 130° ($CHCl_3$ ; c = 0,5)
Fp. = 184-187°C (Essigester)

b) 11β, 19-(4-Methoxy-o-phenylen)-3-ethoxy-3, 5-androstadien-17-on

Analog Beispiel 3 b) werden 5 g der unter a) erhaltenen Substanz mit Ethanol umgesetzt. Es werden 2,45 g kristalline Titelverbindung erhalten.
$[\alpha]_D^{22}$ = 57° ($CHCl_3$ ; c = 0,5)
Fp. = 174-176°C

c) 17-(Prop-1-inyl)-11β, 19-(4-methoxy-o-phenylen)-3-ethoxy-3, 5-androstadien-17β-ol

Analog Beispiel 3 c) werden 2,4 g der unter b) erhaltenen Ketoverbindung umgesetzt. Es werden 2,45 g Rohprodukt isoliert.
$[\alpha]_D^{22}$ = −86° ($CHCl_3$ ; c = 0,505)
Fp. = 168-171°C (Ethanol)

d) 17-(Prop-1-inyl)-17β-hydroxy-11β, 19-(4-methoxy-o-phenylen)-4, 6-androstadien-3-on

Analog Beispiel 3 d) werden 2,35 g des unter c) erhaltenen Rohprodukts zur entsprechenden 4,6-Dien-3-ketoverbindung umgesetzt. Chromatographie des Rohprodukts an Kieselgel führen zu 1,43 g Titelverbindung.
$[\alpha]_D^{22}$ = 132° ($CHCl_3$ ; c = 0,5)
Fp. = 237-242°C (Essigester)

**BEISPIEL 8**

17-(3-Hydroxyprop-1(Z)-enyl)-17β-hydroxy-11β, 19-(4-methylthio-o-phenylen)-4-androsten-3-on

a) 19-(2-Chlor-5-methylthiophenyl)-3, 3-(2, 2-dimethyltrimethylendioxy)-9(11)-androsten-5α, 17β-diol

Analog Beispiel 1 a) werden 34 g 5α,10α-Epoxy-3,3-(2,2-dimethyltrimethylendioxy)-9(11)-androsten-17β-ol mit 94,5 g 2-Chlor-5-methylthiobenzylchlorid umgesetzt. Nach Chromatographie werden 43,2 g obiger Titelverbindung als weißer Schaum isoliert.

b) 11β, 19-(4-Methylthio-o-phenylen)-3, 3-(2, 2-dimethyltrimethylendioxy)-androstan-5α, 17β-diol

40 g der unter a) erhaltenen Substanz werden gelöst in 750 ml absolutem Tetrahydrofuran bei −78°C zu einem Gemisch aus 3,79 g Lithium und 3,4 l flüssigem Ammoniak zugetropft. Nach 45 minütigem Nachrühren wird langsam ein Gemisch aus 200 ml Methanol, 200 ml Tetrahydrofuran und 4,6 ml Methyljodid bei derselben Temperatur zugetropft. Nach erfolgter Zugabe wird der Ansatz analog Vorschrift b) Beispiel 1 aufgearbeitet. Aus dem Rohprodukt werden direkt 18,37 g saubere Titelverbindung auskristallisiert.
Fp. = 173-176°C (Essigester).

c) 11β, 19-(4-Methylthio-o-phenylen)-5α-hydroxy-3, 3-(2, 2-dimethyltrimethylendioxy)-androstan-17-on

Analog Beispiel 13 c) werden 18 g der unter b) erhaltenen Verbindung mit 21,47 g Aluminiumtriisopropylat und 156 ml Cyclohexanon in 780 ml absolutem Toluol zur entsprechenden 17-Ketoverbindung umgesetzt. Nach Chromatographie an Aluminiumoxid (neutral, Stufe III) werden 13,98 g obiger Verbindung als weißer Schaum isoliert.
$[\alpha]_D^{22}$ = 41,8° ($CHCl_3$ ; c = 0,5)
Fp. = 224-225°C (Essigester/Hexan)

d) 17-[3-(Tetrahydropyran-2-yloxy)-prop-1-inyl]-11β, 19-(4-methylthio-o-phenylen)-3, 3-(2, 2-dimethyltrimethylendioxy)-androstan-5α, 17β-diol

Analog Beispiel 5 a) werden 13,8 g der unter c) erhaltenen Substanz mit 19 g 3-(Tetrahydropyran-2-yloxy)-prop-1-in umgesetzt. Nach Chromatographie werden 15,65 g obiger Verbindung als weißer Schaum erhalten.
IR (KBr) : 2230 $cm^{-1}$ Dreifachbindung.

e) 17-[3-(Tetrahydropyran-2-yloxy)-prop-1(Z)-enyl]-11β, 19-(4-methylthio-o-phenylen)-3, 3-(2, 2-dimethyltrimethylendioxy)-androstan-5α, 17β-diol

Analog Beispiel 5 b) werden 15,5 g der unter d) erhaltenen Substanz mit Wasserstoff unter Verwendung von 1,51 g Palladium auf Bariumsulfat (10% Pd) vergiftet mit 18,9 ml Pyridin als Katalysator hydriert. Nach Chromatographie werden 14,15 g obiger Verbindung als weißer Schaum isoliert.

f) 17-(3-Hydroxyprop-1(Z)-enyl)-17β-hydroxy-11β, 19-(4-methylthio-o-phenylen)-4-androsten-3-on

5,5 g des unter e) erhaltenen Olefins werden analog Beispiel 1 c) mit 5 ml 4 n wäßriger Salzsäure in 200 ml Aceton zur 4-En-3-ketoverbindung umgesetzt. Es werden nach Chromatographie an Kieselgel 2,34 g Titelverbindung als weißer Schaum isoliert.
$[\alpha]_D^{22}$ = 86° ($CHCl_3$ ; c = 0,51)
Fp. = 146-148°C (Essigester/Hexan)

**BEISPIEL 9**

17-(3-Hydroxyprop-1(Z)-enyl)-5α, 17β-dihydroxy-11β, 19-(4-methylthio-o-phenylen)-androstan-3-on

a) 17-(3-Hydroxyprop-1(Z)-enyl)-5α, 17β-dihydroxy-11β, 19-(4-methylthio-o-phenylen)-androstan-3-on

5 g der unter Beispiel 8 e) erhaltenen Substanz werden in 50 ml 70%iger Essigsäure bei Raumtemperatur zur gewünschten 3-Ketoverbindung umgesetzt.

Das Reaktionsgemisch wird anschließend mit Wasser verdünnt und die wäßrige Phase mit Methylenchlorid extrahiert. Die vereinigten organischen Phasen werden nacheinander mit gesättigter Natriumhydrogensulfatlösung und gesättigter Kochsalzlösung gewaschen und anschließend über Natriumsulfat getrocknet. Nach Abzug der Solvenzien am Vakuum wird der Rückstand an Kieselgel chromatographiert. Es werden 2,66 g obiger Verbindung als weißer Schaum isoliert.
$[\alpha]_D^{22}$ = −5° ($CHCl_3$ ; c = 0,5)

Fp. = 193-195°C (Essigester)

**BEISPIEL 10**

17-(3-Hydroxyprop-1(Z)-enyl)-17β-hydroxy-11β, 19-(4-methylsulfinyl-o-phenylen)-4-androsten-3-on

a) 17-[3-(Tetrahydropyran-2-yloxy)-prop-1(Z)-enyl]-11β, 19-(4-methylsulfenyl-o-phenylen)-3, 3-(2, 2-dimethyltrimethylendioxy)-androstan-5α, 17β-diol

5 g der unter Beispiel 8 e) erhaltenen Substanz werden in einem Gemisch aus 45 ml Tetrahydrofuran, 45 ml Methanol und 10 ml Wasser gelöst und mit 5,1 g Natriumperjodat versetzt. Über Nacht wird das Reaktionsgemisch bei Raumtemperatur nachgerührt, dann über Celite filtriert und das Filtrat mit Essigester verdünnt. Die organische Phase wird mit gesättigter Natriumhydrogensulfatlösung gewaschen, über Natriumsulfat getrocknet und am Vakuum eingeengt. Der Rückstand wird an Aluminiumoxid (neutral, Stufe III) chromatographiert. Man erhält 3,94 g obiger Verbindung als weißen Schaum.

b) 17-(3-Hydroxyprop-1-(Z)-enyl)-17β-hydroxy-11β, 19-(4-methylsulfinyl-o-phenylen)-4-androsten-3-on

Analog Beispiel 1 c) werden 3,8 g der unter a) erhaltenen Substanz zur 4-En-3-ketoverbindung umgesetzt. Nach Chromatographie an Kieselgel werden 1,66 g Titelverbindung isoliert.
$[\alpha]_D^{22}$ = 51° ($CHCl_3$ ; c = 0,5).

**BEISPIEL 11**

17-(3-Hydroxyprop-1-inyl)-17β-hydroxy-11β, 19-(4-methylthio-o-phenylen)-4-androsten-3-on

a) 17-(3-Hydroxyprop-1-inyl)-17β-hydroxy-11β, 19-(4-methylthio-o-phenylen)-4-androsten-3-on

Analog Beispiel 1 c) werden 2,5 g der unter Beispiel 8 d) erhaltenen Substanz zur entsprechenden 4-En-3-ketoverbindung gespalten. Es werden nach Chromatographie an Kieselgel 1,13 g Titelverbindung als weißer Schaum isoliert.

**BEISPIEL 12**

17-(3-Hydroxyprop-1(Z)-enyl)-17β-hydroxy-11β, 19-(4-ethylthio-o-phenylen)-4-androsten-3-on

a) 11β, 19-(4-Ethylthio-o-phenylen)-3, 3-(2, 2-dimethyltrimethylendioxy)-androstan-5α, 17β-diol

Analog Beispiel 8 b) werden 10 g der unter Beispiel 8 a) hergestellten Substanz mit 620 mg Lithium und anstelle von Methyljodid mit 14,7 ml Ethyljodid umgesetzt. Aus dem Rohprodukt werden in einem Gemisch aus Essigester/Hexan 4,62 g obiger Verbindung als kristallines Produkt gewonnen.
$[\alpha]_D^{22}$ = 39° ($CHCl_3$ ; c = 0,5)
Fp. = 164°C

b) 11β, 19-(4-Ethylthio-o-phenylen)-5α-hydroxy-3, 3-(2, 2-dimethyltrimethylendioxy)-androstan-17-on

Analog Beispiel 13 c) werden 4,5 g der unter a) hergestellten Substanz in die entsprechende 17-Ketoverbindung überführt. Nach Chromatographie werden 3,4 g obiger Titelverbindung isoliert.
$[\alpha]_D^{22}$ = 44° ($CHCl_3$ ; c = 0,505) ;
IR (KBr) : 1740 $cm^{-1}$ Fünfringkekton.

c) 17-[3-(Tetrahydropyran-2-yloxy)-prop-1-inyl]-11β, 19-(4-ethylthio-o-phenylen)-3, 3-(2, 2-dimethyltrimethylendioxy)-androstan-5α, 17β-diol

Analog Beispiel 5 a) werden 3,2 g des unter b) hergestellten Ketons mit 4,31 g 3-(Tetrahydropyran-2-yloxy)prop-1-in umgesetzt. Nach Chromatographie werden 3,25 g obiger Verbindung als weißer Schaum isoliert.

IR (KBr) : 2230 $cm^{-1}$ Dreifachbindung

d) 17-[3-(Tetrahydropyran-2-yloxy)-prop-1(Z)-enyl]-11β, 19-(4-ethylthio-o-phenylen)-3, 3-(2, 2-dimethyltrimethylendioxy)-androstan-5α, 17β-diol

Analog Beispiel 5 b) werden 3,1 g der unter c) erhaltenen Substanz mit 300 mg Palladium auf Bariumsulfat (10% Pd) vergiftet mit 3,75 ml Pyridin als Katalysator hydriert. Nach Chromatographie werden 2,85 g Titelverbindung als weißer Schaum isoliert.

e) 17-(3-Hydroxyprop-1(Z)-enyl)-17β-hydroxy-11β, 19-(4-ethylthio-o-phenylen)-4-androsten-3-on

2,7 g des unter d) erhaltenen Olefins werden analog Beispiel 1 c) mit 2,5 ml 4 n wäßriger Salzsäure in 100 ml Aceton zur entsprechenden 4-En-3-ketoverbindung umgesetzt. Nach Chromatographie an Kieselgel werden 1,35 g Titelverbindung als gelblicher Schaum isoliert.
$[\alpha]_D^{22}$ = 85° ($CHCl_3$ ; c = 0,5).

**BEISPIEL 13**

17-(Prop-1-inyl)-17β-hydroxy-11β, 19-(4-dimethylamino-o-phenylen)-4-androsten-3-on

a) 19-(2-Chlor-5-dimethylaminophenyl)-3, 3-(2, 2-dimethyltrimethylendioxy)-9(11)-androsten-5α, 17β-diol

Analog Beispiel 1 a) werden ausgehend von 15 g 5α,10α-Epoxy-3,3-(2,2-dimethyltrimethylendioxy)-9(11)-estren-17β-ol durch Umsetzung mit 2-Chlor-5-dimethylaminobenzylchlorid 14,39 g obiger Verbindung erhalten.

b) 11β, 19-(4-Dimethylamino-o-phenylen)-3, 3-(2, 2-dimethyltrimethylendioxy)-androstan-5α, 17β-diol

Analog Beispiel 1 b) werden ausgehend von 14 g der unter a) erhaltenen Verbindung 9,9 g obiger Verbindung als weißer Schaum erhalten.

c) 11β, 19-(4-Dimethylamino-o-phenylen)-5α-hydroxy-3, 3-(2, 2-dimethyltrimethylendioxy)-androstan-17-on

11,5 g der unter b) hergestellten Verbindung werden in 500 ml Toluol gelöst und nacheinander mit 13,8 g Aluminiumtriisopropylat und 100 ml Cyclohexanon versetzt. Anschließend wird das Reaktionsgemisch unter Rückfluß erhitzt und ungefähr ein Drittel des Lösungsmittels abdestilliert. Nach dem Abkühlen gießt man in Eiswasser, filtriert die entstandene Emulsion über Celite, wäscht den Filterrückstand gründlich mit Ethylacetat, trennt die organische Phase des Filtrats ab, trocknet diese über Natriumsulfat und engt sie im Vakuum ein. Nach Chromatographie des Rückstandes an Aluminiumoxid (neutral, Stufe III) werden 8,13 g Titelverbindung erhalten.
Kristallisation von 130 mg dieser Verbindung aus Essigester führen zu 67 mg kristallinem 11β,19-(4-Dimethylamino-o-phenylen)-5α-hydroxy-3,3-(2,2-dimethyltrimethylendioxy)-androstan-17-on.
$[\alpha]_D^{22}$ = 28° ($CHCl_3$ ; c = 0,5)
Fp. = 264-267°C

d) 17-(Prop-1-inyl)-11β, 19-(4-dimethylamino-o-phenylen)-3, 3-(2, 2-dimethyltrimethylendioxy)-androstan-5α, 17β-diol

Analog Beispiel 2b) werden 3 g der unter c) erhaltenen Verbindung in die entsprechende 17α-Propinyl-Verbindung überführt. Es werden nach der Chromatographie 2,6 g obiger Verbindung als gelblicher Schaum isoliert.
IR (KBr) : 2235 $cm^{-1}$ Dreifachbindung

e) 17-(Prop-1-inyl)-17β-hydroxy-11β, 19-(4-dimethylamino-o-phenylen)-4-androsten-3-on

Analog Beispiel 1 c) werden 2,5 g des unter d) erhaltenen Produkts zur entsprechenden 4-En-3-ketoverbindung umgesetzt. Es werden 1,58 g obiger Verbindung als weißer Schaum isoliert.
$[\alpha]_D^{22}$ = +28° ($CHCl_3$, c = 0,51)

Fp. : = 231-234°C (Ethylacetat)

Das für die Reaktionsstufe 13a) benötigte 2-Chlor-5-dimethylaminobenzylchlorid wird auf folgendem Wege hergestellt :

α) 2-Chlor-5-aminobenzylalkohol

Unter Schutzgas werden 300 g Lithiumaluminiumhydrid in 3 l Tetrahydrofuran bei 0°C vorgelegt und portionsweise mit 500 g 5-Amino-2-chlorbenzoesäure (technisch 85%ig) versetzt. Anschließend wird das Reaktionsgemisch langsam auf Raumtemperatur erwärmt und über Nacht bei dieser Temperatur nachgerührt. Zur Aufarbeitung wird das Reaktionsgemisch auf 0°C gekühlt und das überschüssige Lithiumaluminiumhydrid vorsichtig mit gesättigter Ammoniumchloridlösung zersetzt. Danach wird die organische Phase vom Bodensatz abgetrennt und der Bodensatz mehrmals mit Essigester und Methylenchlorid gewaschen. Die vereinigten organischen Phasen werden mit gesättigter Natriumchloridlösung neutral gewaschen, über Natriumsulfat getrocknet und am Vakuum eingeengt. Es werden 242 g roher 2-Chlor-5-aminobenzylalkohol erhalten, dessen Reinheit für die sich anschließenden Reaktionen ausreichend ist.

$^1$H-NMR ($CDCl_3$) [δ] : 6,3-7,15 (3H, m, aromatische Protonen) ;
4,55 (2H, s, benzylische Protonen).

β) 2-Chlor-5-dimethylaminobenzylalkohol

Eine Suspension aus 51,8 g Natriumborhydrid in einer Mischung aus 30 g 2-Chlor-5-aminobenzylalkohol und 1 l Tetrahydrofuran werden so unter Kühlung zu einer gerührten Mischung aus 235 ml 2 m Schwefelsäure und 88 ml einer 38%igen Formalinlösung zugetropft, daß die Temperatur zwischen -10 und 20°C bleibt. Nach erfolgter Zugabe wird die Reaktionsmischung mit festem Natriumhydroxid stark basisch gemacht und mit etwas Wasser versetzt. Die organische Phase wird abgetrennt, die wäßrige mit Methylenchlorid mehrmals extrahiert und die vereinigten organischen Phasen werden mit gesättigter Natriumchloridlösung neutral gewaschen. Anschließend werden sie über Natriumsulfat getrocknet und im Vakuum eingeengt. Es werden 24 g 2-Chlor-5-dimethylaminobenzylalkohol als Öl erhalten.

$^1$H-NMR ($CDCl_3$) [δ] : 6,4-7,25 (3H, m, aromatische Protonen) ;
4,67 (3H, s, benzylische Protonen) ;
2,92 (6H, s, Protonen der beiden Methylgruppen).

γ) 2-Chlor-5-dimethylaminobenzylchlorid

23,8 g N-Chlorsuccinimid werden in 600 ml absolutem Methylenchlorid vorgelegt, auf 0°C gekühlt und langsam mit 15,6 ml Dimethylsulfid versetzt. Anschließend wird die entstandene Suspension auf −30°C abgekühlt und mit 20 g 2-Chlor-5-dimethylaminobenzylalkohol vorsichtig versetzt. Danach wird auf 0°C erwärmt und das Reaktionsgemisch bei dieser Temperatur 3 Stunden nachgerührt. Anschließend wird mit Methylenchlorid verdünnt und das Reaktionsgemisch auf Eiswasser gegossen. Die organische Phase wird abgetrennt, mit gesättigter Natriumchloridlösung gewaschen, über Natriumsulfatlösung getrocknet und im Vakuum eingeengt. Der Rückstand wird an Aluminiumoxid (neutral, Stufe III) mit Hexan chromatographiert. Es werden 17,2 g 2-Chlor-5-dimethylaminobenzylchlorid erhalten.

$^1$H-NMR ($CDCl_3$) [δ] : 6,4-7,3 (3H, m, aromatische Protonen) ;
4,61 (2H, s, benzylische Protonen) ;
2,92 (6H, s, Protonen der beiden Methylgruppen).

**BEISPIEL 14**

17β-Hydroxy-17-methoxymethyl-11β, 19-(4-dimethylamino-o-phenylen)-4-androsten-3-on

a) 11β, 19-(4-Dimethylamino-o-phenylen)-3, 3-(2, 2-dimethyltrimethylendioxy)-androstan-[17(β-1′)-spiro-3′]-oxiran-5α-ol

2,5 g der unter Beispiel 13 c) dargestellten Verbindung werden unter Schutzgas in 50 ml abs. Dimethylformamid gelöst und auf 0°C gekühlt. Zu dieser Lösung werden nacheinander 5 g Trimethylsulfoniumjodid und

3,4 g Kaliumtert.butylat zugegeben. Es wird solange nachgerührt, bis eine vollständige Umsetzung erfolgt ist (DC-Kontrolle). Anschließend wird die Reaktionsmischung auf Eiswasser gegossen, die wäßrige Phase mit Essigester extrahiert, die organische Phase mit Natriumchloridlösung gewaschen und über Natriumsulfat getrocknet. Nach Abzug der Solvenzien wird der Rückstand an Aluminiumoxid (neutral, Stufe III) chromatographiert. Es werden 2,1 g obiger Verbindung als weißer Schaum isoliert.

b) 17-Methoxymethyl-11β, 19-(4-dimethylamino-o-phenylen)-3, 3-(2, 2-dimethyltrimethylendioxy)-androstan-5α, 17β-diol

2 g der unter a) dargestellten Verbindung werden in 40 ml 3 m methanolische Natriummethylatlösung gelöst und anschließend unter Schutzgas 5 Stunden zum Rückfluß erhitzt. Nach Abkühlen wird das Reaktionsgemisch auf Wasser gegossen, die wäßrige Phase mit Methylenchlorid extrahiert und die organische Phase mit Natriumchloridlösung gewaschen. Nach Trocknen der organischen Phase über Natriumsulfat, Einengen im Vakuum und Chromatographie des Rückstandes an Aluminiumoxid (neutral, Stufe III) werden 1,41 g obiger Verbindung als weißer Schaum isoliert.

c) 17β-Hydroxy-17-methoxymethyl-11β, 19-(4-dimethylamino-o-phenylen)-4-androsten-3-on

Analog Beispiel 1 c) werden 1,3 g des unter b) erhaltenen Produkts zur entsprechenden 4-En-3-ketoverbindung umgesetzt. Es werden 0,75 g Titelverbindung als weißer Schaum isoliert.
$[\alpha]_D^{22} = 80°$ ($CHCl_3$ ; c = 0,505),
Fp. = 124-127°C

**BEISPIEL 15**

17-Cyanomethyl-17β-hydroxy-11β, 19-(4-dimethylamino-o-phenylen)-4-androsten-3-on

a) 17-Cyanomethyl-11β, 19-(4-dimethylamino-o-phenylen)-3, 3-(2, 2-dimethyltrimethylendioxy)-androstan-5α, 17β-diol

Analog Beispiel 6 b) werden 2,2 g des nach Vorschrift a) Beispiel 14 hergestellten Epoxids mit einer Lösung von 4,22 g Kaliumcyanid in 8,4 ml Wasser in 42 ml Ethanol umgesetzt. Nach Chromatographie werden 1,95 g obiger Verbindung isoliert.
IR (KBr) : 2245 $cm^{-1}$ C≡N Dreifachbindung

b) 17-Cyanomethyl-17β-hydroxy-11β, 19-(4-dimethylamino-o-phenylen)-4-androsten-3-on

1,9 g des unter a) erhaltenen Cyanids werden analog Beispiel 1 c) zur entsprechenden 4-En-3-ketoverbindung umgesetzt. Aus dem Rohprodukt können direkt 1,23 g Titelverbindung kristallisiert werden. Chromatographie der Mutterlauge führen zu weiteren 138 mg der gewünschten Cyanoverbindung.
$[\alpha]_D^{22} = 77°$ ($CHCl_3$ ; c = 0,5)
Fp. = 172-176°C

**BEISPIEL 16**

17-(3-Hydroxyprop-1-inyl)-17β-hydroxy-11β, 19-(4-dimethylamino-o-phenylen)-4-androsten-3-on

a) 17-[3-(Tetrahydropyran-2-yloxy)-prop-1-inyl]-11β, 19-(4-dimethylamino-o-phenylen)-3, 3-(2, 2-dimethyltrimethylendioxy)-androstan-5α, 17β-diol

Analog Beispiel 5 a) werden 10 g der nach Vorschrift c) Beispiel 13 hergestellten Ketoverbindung mit 55,3 g 3-(Tetrahydropyran-2-yloxy)-propin und 247 ml einer 15%igen Lösung von n-Butyllithium in Hexan umgesetzt. Es werden nach Chromatographie 11,74 g obiger Verbindung als weißer Schaum isoliert.
IR (KBr) : 2230 $cm^{-1}$ Dreifachbindung

b) 17-(3-Hydroxyprop-1-inyl)-17β-hydroxy-11β, 19-(4-dimethylamino-o-phenylen)-4-androsten-3-on

Analog Beispiel 1 c) werden 11,74 g der unter a) hergestellten Verbindung in 6,97 g Titelverbindung überführt.
$[\alpha]_D^{22}$ = 25,6° ($CHCl_3$ ; c = 0,5)
Fp. = 251-253°C (Essigester)

**BEISPIEL 17**

17-(3-Hydroxyprop-1(Z)-enyl)-17β-hydroxy-11β, 19-(4-dimethylamino-o-phenylen)-4-androsten-3-on

a) 17-(3-Hydroxyprop-1(Z)-enyl)-17β-hydroxy-11β, 19-(4-dimethylamino-o-phenylen)-4-androsten-3-on

Analog Beispiel 5 b) werden 6,5 g der nach Beispiel 16 b) hergestellten Acetylenverbindung zum entsprechenden Z-Olefin umgesetzt. Es werden 4,76 g Titelverbindung nach Chromatographie an Kieselgel als weißer Schaum isoliert.
$[\alpha]_D^{22}$ = 71° ($CHCl_3$ ; c = 0,5)

**BEISPIEL 18**

17β-Hydroxy-11β, 19-(4-hydroxy-o-phenylen)-4-androsten-3-on

a) 11β, 19-(4-Hydroxy-o-phenylen)-3, 3-(2, 2-dimethyltrimethylendioxy)-androstan-5α, 17β-diol

50 g der analog Vorschrift β) des Beispiels 4 erhaltenen Methoxyverbindung werden in 500 ml absolutem Dimethylformamid gelöst und unter Schutzgas mit 28,2 g Natriummethanthiolat versetzt. Unter Inertgasatmosphäre wird das Reaktionsgemisch 3 Stunden zum Rückfluß erhitzt, anschließend auf Raumtemperatur abgekühlt und dann auf 8 l Eiswasser gegossen. Es wird solange bei Raumtemperatur nachgerührt, bis das Rohprodukt als feste Substanz ausgeflockt ist. Anschließend wird es abgesaugt, mit Wasser gewaschen und am Vakuum getrocknet. Es werden 49,2 g rohe Titelverbindung als weißer Feststoff isoliert, dessen Qualität für weitere Umsetzungen ausreicht.
$[\alpha]_D^{22}$ = 21° ($CHCl_3$ ; c = 0,5)
Fp. = 267-270°C (Essigester)

b) 17β-Hydroxy-11β, 19-(4-hydroxy-o-phenylen)-4-androsten-3-on

2 g des unter a) erhaltenen Phenols werden analog Beispiel 1 c) mit 3 ml 4 n wäßriger Salzsäure in 60 ml Aceton zur 4-En-3-ketoverbindung umgesetzt. Es werden 1,05 g obiger Verbindung als weißer Schaum isoliert.
Fp. = 242-245°C (Essigester).

**BEISPIEL 19**

17β-Hydroxy-11β, 19-(4-trifluormethylsulfonyloxy-o-phenylen)-4-androsten-3-on

a) 17β-Hydroxy-11β, 19-(4-trifluormethylsulfonyloxy-o-phenylen)-4-androsten-3-on

10 g des nach Beispiel 18 b) hergestellten Phenols werden in 250 ml absolutem Methylenchlorid gelöst und mit 17,3 g 4-Dimethylaminopyridin versetzt. Unter Schutzgas wird die Lösung anschließend auf −50°C gekühlt und langsam durch Zutropfen mit 4,76 ml Trifluormethansulfonsäureanhydrid gelöst in 30 ml absolutem Methylenchlorid versetzt. Nach 15 minütigem Nachrühren bei −50°C wird das Reaktionsgemisch auf gesättigte Natriumhydrogencarbonatlösung gegossen und die wäßrige Phase mit Methylenchlorid extrahiert. Die vereinigten organischen Phasen werden mit gesättigter Natriumchloridlösung gewaschen, über Natriumsulfat getrocknet und am Vakuum eingeengt. Man erhält nach Chromatographie des Rückstandes an Kieselgel 11,37 g Titelverbindung als gelblichen Schaum.
Fp. = 204-205°C (Diisopropylether).

**BEISPIEL 20**

17β-Hydroxy-11β, 19-[4-(2-trimethylsilylethinyl)-o-phenylen]-4-androsten-3-on

a) 17β-Hydroxy-11β, 19-[4-(2-Trimethylsilylethinyl)-o-phenylen]-4-androsten-3-on

1 g der nach Beispiel 19 a) hergestellten Triflatverbindung werden in 10 ml absolutem Dimethylformamid gelöst und unter Schutzgas mit 1,18 ml Triethylamin, 1,39 ml Trimethylsilylacetylen und 49 mg Palladiumtetrakistriphenylphosphin versetzt. Anschließend wird das Reaktionsgemisch 1 Stunde auf 110°C erhitzt, dann auf Raumtemperatur abgekühlt und mit Essigester verdünnt. Nach Filtration über Celite wird das Filtrat mehrmals mit gesättigter Natriumchloridlösung gewaschen, die organische Phase abgetrennt, über Natriumsulfat getrocknet und am Vakuum eingeengt. Chromatographie des Rückstandes an Kieselgel ergeben 656 mg Titelverbindung als weißen Schaum.
Fp. = 267-271°C (Diisopropylether).

**BEISPIEL 21**

17-(Prop-1-inyl)-17β-hydroxy-11β, 19-[4-(2-trimethylsilylethinyl)-o-phenylen]-4-androsten-3-on

a) 11β, 19-(4-Trifluormethylsulfonyloxy-o-phenylen)-3, 3-(2, 2-dimethyltrimethylendioxy)-androstan-5α, 17β-diol

40 g des nach Beispiel 18 a) hergestellten Phenols werden analog Beispiel 19 a) mit 14,93 ml Trifluormethansulfonsäureanhydrid umgesetzt. Nach Chromatographie werden 37,3 g obiger Verbindung als weißer Schaum isoliert.

b) 11β, 19-[4-(2-Trimethylsilylethinyl)-o-phenylen]-3, 3-(2, 2-dimethyltrimethylendioxy)-androstan-5α, 17β-diol

15 g der nach a) hergestellten Verbindung werden analog Beispiel 20 a) mit 17,3 ml Trimethylsilylacetylen umgesetzt. Nach Chromatographie an Aluminiumoxid (neutral, Stufe III) werden 11.7 g obiger Verbindung als weißer Schaum isoliert.
IR (KBr) : 2150 $cm^{-1}$ Dreifachbindung am Aromaten

c) 11β, 19-[4-(Trimethylsilylethinyl)-o-phenylen]-5α-hydroxy-3, 3-(2, 2-dimethyltrimethylendioxy)-androstan-17-on

11,2 g der unter b) erhaltenen Substanz werden analog Beispiel 2 a) mit 11,69 g Chromtrioxid zur entsprechenden 17-Ketoverbindung oxidiert. Chromatographie an Aluminiumoxid (neutral, Stufe III) führen zu 9,05 g Titelverbindung als weißem Schaum.
$[\alpha]_D^{22} = 51°$ ($CHCl_3$ ; c = 0,5) ;
Fp. = 245-248°C (Diisopropylether).

d) 17-(Prop-1-inyl)-11β, 19-[4-(2-Trimethylsilylethinyl)-o-phenylen]-3, 3-(2, 2-dimethyltrimethylendioxy)-androstan-5α, 17β-diol

1 g des unter c) hergestellten Ketons werden analog Beispiel 2 b) mit Propin umgesetzt. Nach Chromatographie an Aluminiumoxid (neutral, Stufe III) werden 956 mg obiger Verbindung als weißer Schaum isoliert.

IR (KBr) : 2250 $cm^{-1}$ Dreifachbindung am Aromaten
2235 $cm^{-1}$ Dreifachbindung

e) 17-(Prop-1-inyl)-17β-hydroxy-11β, 19-[4-(2-trimethylsilylethinyl)-o-phenylen]-4-androsten-3-on

900 mg der unter d) hergestellten Verbindung werden analog Beispiel 1 c) zur entsprechenden 4-En-3-ketoverbindung gespalten. Nach Chromatographie an Kieselgel werden 471 mg Titelverbindung als weißer Schaum isoliert.

$[\alpha]_D^{22}$ = 59° ($CHCl_3$ ; c = 0,505).

**BEISPIEL 22**

17-(Prop-1-inyl)-17β-hydroxy-11β, 19-(4-ethinyl-o-phenylen)-4-androsten-3-on

a) 11β, 19-(4-Ethinyl-o-phenylen)-5α-hydroxy-3, 3-(2, 2-dimethyltrimethylendioxy)-androstan-17-on

1,5 g des nach Beispiel 21 c) hergestellten Ketons werden in 26 ml absolutem Methanol gelöst und mit 1,1 g wasserfreiem Kaliumcarbonat versetzt. Unter Schutzgas wird das Reaktionsgemisch bei Raumtemperatur 3 Stunden nachgerührt, anschließend auf gesättigte Natriumchloridlösung gegossen und die wäßrige Phase mehrmals mit Methylenchlorid extrahiert. Die vereinigten organischen Phasen wäscht man mit gesättigter Natriumchloridlösung, trocknet sie über Natriumsulfat und engt sie am Vakuum ein. Die Qualität des Rohprodukts (1,31 g) ist ausreichend für weitere Umsetzungen. Chromatographie von 100 mg des Rohprodukts an Aluminiumoxid (neutral, Stufe III) führen zu 67 mg reiner Titelverbindung als weißem Schaum.

b) 17-(Prop-1-inyl)-11β, 19-(4-ethinyl-o-phenylen)-3, 3-(2, 2-dimethyltrimethylendioxy)-androstan-5α, 17β-diol

1,2 g des unter a) erhaltenen Produkts werden analog Beispiel 2 b) mit Propin umgesetzt. Nach Chromatographie an Alumiumoxid (neutral, Stufe III) werden 1,12 g obiger Verbindung als weißer Schaum isoliert.

IR (KBr) : 2110 $cm^{-1}$ Dreifachbindung am Aromaten
2235 $cm^{-1}$ Dreifachbindung

c) 17-(Prop-1-inyl)-17β-hydroxy-11β, 19-(4-ethinyl-o-phenylen)-4-androsten-3-on

1,1 g der nach b) hergestellten Substanz werden analog Beispiel 1 c) zur entsprechenden 4-En-3-ketoverbindung umgesetzt. Nach Chromatographie an Kieselgel werden 612 mg Titelverbindung als weißer Schaum isoliert.
$[\alpha]_D^{22}$ = 41° ($CHCl_3$ ; c = 0,5).

**BEISPIEL 23**

17-(Prop-1-inyl)-17β-hydroxy-11β, 19-[4-(2-trimethylsilylethyl)-o-phenylen]-4-androsten-3-on

a) 11β, 19-[4-(2-Trimethylsilylethyl)-o-phenylen]-5α-hydroxy-3, 3-(2, 2-dimethyltrimethylendioxy)-androstan-17-on

1 g des nach Beispiel 21 c) hergestellten Ketons werden in 10 ml absolutem Ethanol gelöst und mit 100 mg Palladium auf Kohle (10%ig) als Katalysator bei Normaldruck hydriert. Nach der Aufnahme von 2 Äquivalenten Wasserstoff wird das Reaktionsgemisch über Celite abgesaugt, der Filterrückstand mit Essigester nachgewaschen und das Filtrat um Vakuum eingeengt. Nach Chromatographie des Rückstandes an Aluminiumoxid (neutral, Stufe III) werden 884 mg obiger Verbindung als weißer Schaum isoliert.

b) 17-(Prop-1-inyl)-11β, 19-[4-(2-trimethylsilylethyl)-o-phenylen]-3, 3-(2, 2-dimethyltrimethylendioxy)-androstan-5α, 17β-diol

850 mg des unter a) hergestellten Ketons werden analog Beispiel 2 b) mit Propin umgesetzt. Nach Chromatographie an Aluminiumoxid (neutral, Stufe III) werden 845 mg obiger Verbindung als weißer Schaum isoliert.
IR (KBr) : 2235 $cm^{-1}$ Dreifachbindung.

c) 17-(Prop-1-inyl)-17β-hydroxy-11β, 19-[4-(2-trimethylsilylethyl-o-phenylen]-4-androsten-3-on

800 mg der unter b) hergestellten Substanz werden analog Beispiel 1 c) zur entsprechenden 4-En-3-ketoverbindung umgesetzt. Nach Chromatographie an Kieselgel werden 512 mg obiger Verbindung als weißer

Schaum isoliert.
$[\alpha]_D^{22} = 23°$ ($CHCl_3$ ; c = 0,505).

**BEISPIEL 24**

17-(Prop-1-inyl)-17β-hydroxy-11β, 19-(4-ethyl-o-phenylen)-4-androsten-3-on

a) 11β, 19-(4-Ethyl-o-phenylen)-5α-hydroxy-3, 3-(2, 2-dimethyltrimethylendioxy)-androstan-17-on

4 g der nach Beispiel 22 a) erhaltenen Acetylenverbindung werden analog Beispiel 23 a) zur entsprechenden Ethylverbindung hydriert. Nach Chromatographie an Aluminiumoxid (neutral, Stufe III) werden 3,63 g obiger Verbindung als weißer Schaum isoliert.

b) 17-(Prop-1-inyl)-11β, 19-(4-ethyl-o-phenylen)-3, 3-(2, 2-dimethyltrimethylendioxy)-androstan-5α, 17β-diol

1,5 g der unter a) hergestellten Verbindung werden analog Beispiel 2 b) mit Propin umgesetzt. Nach Chromatographie an Aluminiumoxid (neutral, Stufe III) werden 1,43 g obiger Verbindung als weißer Schaum erhalten.
IR (KBr) : 2240 $cm^{-1}$ Dreifachbindung

c) 17-(Prop-1-inyl)-17β-hydroxy-11β, 19-(4-ethyl-o-phenylen)-4-androsten-3-on

1,3 g der unter b) erhaltenen Substanz werden analog Beispiel 1 c) zur entsprechenden 4-En-3-ketoverbindung umgesetzt. Nach Chromatographie an Kieselgel werden 879 mg obiger Verbindung als weißer Schaum erhalten.
$[\alpha]_D^{22} = 18°$ ($CHCl_3$ ; c = 0,5)
Fp. = 283-285°C (Essigester)

**BEISPIEL 25**

17-(3-Hydroxy-prop-1(Z)-enyl)-17β-hydroxy-11β, 19-(4-ethyl-o-phenylen)-4-androsten-3-on

a) 17-[3-(Tetrahydropyran-2-yloxy)-prop-1-inyl]-11β, 19-(4-ethyl-o-phenylen)-3, 3-(2, 2-dimethyltrimethylendioxy)-androstan-5α, 17β-diol

2 g der unter Beispiel 24 a) hergestellten Ethylverbindung werden analog der unter Beispiel 5 a) beschriebenen Vorschrift mit 3-(Tetrahydropyran-2-yloxy)-prop-1-in umgesetzt. Nach Chromatographie an Aluminiumoxid werden 2,29 g der Titelverbindung als weißer Schaum isoliert.
IR (KBr) : 2240 $cm^{-1}$ Dreifachbindung

b) 17-[3-(Tetrahydropyran-2-yloxy)-prop-1(Z)-enyl]-11β, 19-(4-ethyl-o-phenylen)-3, 3-(2, 2-dimethyltrimethylendioxy)-androstan-5α, 17β-diol

2,2 g der unter a) erhaltenen Acetylenverbindung werden analog Beispiel 5 b) zum Z-Olefin hydriert. Nach Chromatographie an Aluminiumoxid (neutral, Stufe III) werden 1,95 g der Titelverbindung als weißer Schaum isoliert.

c) 17-(3-Hydroxyprop-1(Z)-enyl)-17β-hydroxy-11β, 19-(4-ethyl-o-phenylen)-4-androsten-3-on

1,9 g der unter b) hergestellten Verbindung werden analog Beispiel 1 c) zur entsprechenden 4-En-3-ketoverbindung gespalten. Nach Chromatographie an Kieselgel werden 706 mg obiger Titelverbindung als weißer Schaum isoliert.
$[\alpha]_D^{22} = 62°$ ($CHCl_3$ ; c = 0,505)
Fp. = 127-129°C (Essigester)

**BEISPIEL 26**

17-(Prop-1-inyl)-17β-hydroxy-11β, 19-(4-vinyl-o-phenylen)-4-androsten-3-on

a) 11β, 19-(4-Hydroxy-o-phenylen)-5α-hydroxy-3, 3-(2, 2-dimethyltrimethylendioxy)-androstan-17-on

20 g des nach Beispiel 4 c) hergestellten 11β,19-(4-Methoxy-o-phenylen)-5α-hydroxy-3,3(2,2-dimethyltrimethylendioxy)-androstan-17-ons werden analog Beispiel 18 a) in das entsprechende Phenol umgewandelt. Es werden 16,8 g als Rohprodukt isoliert, dessen Reinheit für weitere Umsetzungen ausreichend ist. 500 mg des Rohprodukts werden an Aluminiumoxid für analytische Zwecke chromatographiert und ergeben 412 mg der Titelverbindung.

b) 11β, 19-(4-Trifluormethylsulfonyloxy-o-phenylen)-5α-hydroxy-3, 3-(2, 2-dimethyltrimethylendioxy)-androstan-17-on

16,3 g des unter a) erhaltenen Phenols werden analog Beispiel 19 a) in das entsprechende Triflat umgewandelt. Nach Chromatographie an Kieselgel werden 15,1 g der Titelverbindung als weißer Schaum isoliert.

IR (KBr) : 1740 $cm^{-1}$ Fünfringketon
1215 und 1420 $cm^{-1}$ Triflat

c) 11β, 19-(4-Vinyl-o-phenylen)-5α-hydroxy-3, 3-(2, 2-dimethyltrimethylendioxy)-androstan-17-on

1,5 g der unter b) hergestellten Substanz werden in 18 ml absolutem Dimethylformamid gelöst und mit 146 mg Palladiumtetrakistriphenylphosphin und 207 mg Lithiumchlorid unter Schutzgas versetzt. Nach 5minütigem Nachrühren wird das Reaktionsgemisch mit 0,89 ml Tri-n-butylvinylzinn versetzt und auf 110°C erhitzt. Nach 1 Stunde wird das Reaktionsgemisch auf Raumtemperatur abgekühlt, mit Essigester verdünnt und über Celite abgesaugt. Das Filtrat wird mit gesättigter Natriumchloridlösung gewaschen, die organische Phase über Natriumsulfat getrocknet und am Vakuum eingeengt. Der Rückstand wird an Aluminiumoxid (neutral, Stufe III) chromatographiert, und man erhält 1,1 g der Titelverbindung als weißen Schaum.

d) 17-(Prop-1-inyl)-11β, 19-(4-vinyl-o-phenylen)-3, 3-(2, 2-dimethyltrimethylendioxy)-androstan-5α, 17β-diol

1 g des unter a) hergestellten Ketons werden analog Beispiel 2 b) mit Propin umgesetzt. Nach Chromatographie an Aluminiumoxid (neutral, Stufe III) werden 912 mg der Titelverbindung als weißer Schaum isoliert.
IR (KBr) : 2240 $cm^{-1}$ Dreifachbindung

e) 17-(Prop-1-inyl)-17β-hydroxy-11β, 19-(4-vinyl-o-phenylen)-4-androsten-3-on

850 mg der unter d) erhaltenen Verbindung werden analog Beispiel 1 c) in die entsprechende 4-En-3-ketoverbindung umgewandelt. Nach Chromatographie an Kieselgel werden 485 mg der Titelverbindung als weißer Schaum isoliert.
$[\alpha]_D^{22}$ = 50° ($CHCl_3$, c = 0,505)
Fp. = 243-245°C (Diisopropylether)

**BEISPIEL 27**

17-(Prop-1-inyl)-17β-hydroxy-11β, 19-[4-(2-propenyl)-o-phenylen]-4-androsten-3-on

a) 11β, 19-[4-(2-Propenyl)-o-phenylen]-5α-hydroxy-3, 3-(2, 2-dimethyltrimethylendioxy)-androstan-17-on

1,5 g der nach Beispiel 26 b) hergestellten Substanz werden analog Beispiel 26 c) mit 0,36 ml Tetraallylzinn umgesetzt. Nach Chromatographie an Kieselgel werden 1,06 g der Titelverbindung als weißer Schaum isoliert.

b) 17-(Prop-1-inyl)-11β, 19-[4-(2-propenyl)-o-phenylen]-3, 3-(2, 2-dimethyltrimethylendioxy)-androstan-5α, 17β-diol

1 g der nach a) erhaltenen Substanz werden analog Beispiel 2 b) mit Propin umgesetzt. Nach Chromatographie an Aluminiumoxid (neutral, Stufe III) werden 942 mg der Titelverbindung als weißer Schaum isoliert.
IR (KBr) : 2240 $cm^{-1}$ Dreifachbindung

c) 17-(Prop-1-inyl)-17β-hydroxy-11β, 19-[4-(2-propenyl)-o-phenylen]-4-androsten-3-on

900 mg der nach b) hergestellten Substanz werden analog Beispiel 1 c) zur entsprechenden 4-En-3-ketoverbindung umgesetzt. Chromatographie an Kieselgel ergibt 397 mg der Titelverbindung als weißen Schaum.
$[\alpha]_D^{22}$ = 18° ($CHCl_3$ ; c = 0,5)
Fp. = 275-277°C (Methylenchlorid)

**BEISPIEL 28**

17β-Hydroxy-11β, 19-(4-acetyl-o-phenylen)-4-androsten-3-on

a) 11β, 19-(4-Acetly-o-phenylen)-3, 3-(2, 2-dimethyltrimethylendioxy)-androstan-5α, 17β-diol

30 g des nach Beispiel 21 a) hergestellten Triflats werden analog Beispiel 26 c) mit 22,06 g 1-Ethoxyvinyl-tributylzinn umgesetzt. Nach Chromatographie an Kieselgel werden 18,75 g der Titelverbindung als weißer Schaum isoliert.
$[\alpha]_D^{22}$ = 146° ($CHCl_3$ ; c = 0,5)
Fp. = 179-181°C (Diisopropylether)

b) 17β-Hydroxy-11β, 19-(4-acetyl-o-phenylen)-4-androsten-3-on

18 g der nach a) erhaltenen Substanz werden analog Beispiel 1 c) zur entsprechenden 4-En-3-ketoverbindung umgesetzt. Es werden nach Chromatographie an Kieselgel 10,8 g der Titelverbindung als gelblicher Schaum isoliert.
Fp. = 135-138°C (Essigester/Hexan)

**BEISPIEL 29**

17-(Prop-1-inyl)-17β-hydroxy-11β, 19-(4-acetyl-o-phenylen)-4-androsten-3-on

a) 11β, 19-{4-[1,1-(2,2-Dimethyltrimethylendioxy)-ethyl]-o-phenylen}-3, 3-(2, 2-dimethyltrimethylendioxy)-5-androsten-17β-ol

10 g der nach 28 b) erhaltenen Substanz werden in 250 ml absolutem Toluol gelöst und nacheinander mit 25,7 g 1,3-Dimethylpropandiol und 1,86 g Pyridiniumparatoluolsulfonat versetzt. Das Reaktionsgemisch wird anschließend 4 Stunden unter Rückfluß erhitzt und gleichzeitig entstehendes Wasser azeotrop entfernt. Dann wird das Reaktionsgemisch auf Raumtemperatur abgekühlt, auf eiskalte 5%ige wäßrige Natriumhydroxidlösung gegossen und die wäßrige Phase mit Essigester extrahiert. Die vereinigten organischen Phasen werden mit gesättigter Natriumchloridlösung gewaschen, über Natriumsulfat getrocknet und am Vakuum eingeengt. Chromatographie des Rückstandes an Aluminiumoxid (neutral, Stufe III) führt zu 10,7 g der Titelverbindung als weißem Schaum.

b) 11β, 19-{4-[1, 1-(2, 2-Dimethyltrimethylendioxy)-ethyl]-o-phenylen}-3, 3-(2, 2-dimethyltrimethylendioxy)-5-androsten-17-on

10,5 g der nach a) erhaltenen Substanz werden analog Beispiel 2 a) zur entsprechenden 17-Ketoverbindung umgesetzt. Es werden 10,2 g Rohprodukt isoliert, dessen Reinheit für weitere Reaktionen ausreichend ist. 500 mg werden für analytische Zwecke an Aluminiumoxid (neutral, Stufe III) chromatographiert und ergeben 443 mg der Titelverbindung als weißen Schaum.
$[\alpha]_D^{22}$ = 43° ($CHCl_3$ ; c = 0,5) ;

Fp. = 244-246°C (Essigester)

c) 17-(Prop-1-inyl)-11β, 19-{4-[1, 1-(2, 2-dimethyltrimethylendioxy)-ethyl]-o-phenylen}-3, 3-(2, 2-dimethyltrimethylendioxy)-5-androsten-17β-ol

1,5 g des nach b) erhaltenen Rohprodukts werden analog Beispiel 2 b) mit Propin umgesetzt. Nach Chromatographie an Aluminiumoxid (neutral, Stufe III) werden 1,35 g der Titelverbindung als weißer Schaum erhalten.
IR (KBr) : 2240 $cm^{-1}$ Dreifachbindung

d) 17-(Prop-1-inyl)-17β-hydroxy-11β, 19-(4-acetyl-o-phenylen)-4-androsten-3-on

1,3 g der nach c) hergestellten Substanz werden analog Beispiel 1 c) zur entsprechenden 4-En-3-ketoverbindung gespalten. Nach Chromatographie an Kieselgel werden 747 mg der Titelverbindung als gelblicher Schaum isoliert.
$[\alpha]_D^{22}$ = 36° ($CHCl_3$ ; c = 0,5)
Fp. = 186-187°C (Essigester)

**BEISPIEL 30**

17-(Prop-2-inyl)-17β-hydroxy-11β, 19-(4-acetyl-o-phenylen)-4-androsten-3-on

a) 17-(3-Trimethylsilylprop-2-inyl)-11β, 19-{4-[1, 1-(2, 2-dimethyltrimethylendioxy)-ethyl]-o-phenylen}-3, 3-(2, 2-dimethyltrimethylendioxy)-5-androsten-17β-ol

1,5 g der nach Beispiel 29 b) hergestellten Verbindung werden analog Beispiel 5 a) mit 2,3 ml 1-Trimethylsilylprop-1-in umgesetzt. Nach Chromatographie an Aluminiumoxid (neutral, Stufe III) werden 1,31 g der Titelverbindung als weißer Schaum isoliert.
IR (KBr) : 2170 $cm^{-1}$ Dreifachbindung

b) 17-(Prop-2-inyl)-17β-hydroxy-11β, 19-(4-acetyl-o-phenylen)-4-androsten-3-on

1,2 g der nach a) hergestellten Substanz werden analog Beispiel 1 c) in die entsprechende 4-En-3-ketoverbindung überführt. Nach Chromatographie an Kieselgel werden 547 mg der Titelverbindung als weißer Schaum isoliert.
$[\alpha]_D^{22}$ = 91° ($CHCl_3$ ; c = 0,5)
Fp. = 257-259°C

**BEISPIEL 31**

17-(Prop-1-inyl)-17β-hydroxy-11β, 19-(4-acetyl-o-phenylen)-4, 15-androstadien-3-on

a) 11β, 19-{4-[1, 1-(2, 2-dimethyltrimethylendioxy)-ethyl]-o-phenylen}-3, 3-(2, 2-dimethyltrimethylendioxy)-5, 15-androstadien-17-on

1,89 g der nach Beispiel 29 b) hergestellten Verbindung werden zu einer Lösung von 9,9 mmol Lithiumdiisopropylamid in 40 ml absolutem Tetrahydrofuran gelöst in 20 ml absolutem Tetrahydrofuran unter Schutzgas bei 0°C zugetropft. Anschließend wird zum Reaktionsgemisch Chlortrimethylsilan (2,39 ml) zugetropft. Nach 30 minütigem Nachrühren wird die Reaktionslösung auf eiskalte gesättigte Natriumhydrogencarbonatlösung gegossen, die wäßrige Phase mit Essigester extrahiert und die organische Phase mit Wasser und gesättigter Ammoniumchloridlösung gewaschen. Nach Trocknen über Natriumsulfat wird die organische Phase im Vakuum eingeengt. Es werden 1,96 g 17-Trimethylsilyloxy-11β,19-{4-[1,1-(2,2-dimethyltrimethylendioxy)-ethyl]-o-phenylen}-3,3-(2,2-dimethyltrimethylendioxy)5,16-androstadien roh als gelblicher Schaum isoliert. Dieses Rohprodukt wird in 23 ml absolutem Acetonitril suspendiert und mit 1 g Palladium(II)acetat versetzt. Nach 2 stündigem Nachrühren bei Raumtemperatur wird das Reaktionsgemisch über Celite abgesaugt, der Filterrückstand mit Essigester nachgewaschen und das Filtrat am Vakuum eingeengt. Der Rückstand wird an Aluminiumoxid (neutral, Stufe III) chromatographiert, und man erhält 1,33 g der Titelverbindung als weißen

Schaum.
IR (KBr) : 1710 $cm^{-1}$ ungesättigtes Fünfringketon

b) 17-(Prop-1-inyl)-11β, 19-{4-[1, 1-(2, 2-dimethyltrimethylendioxy)-ethyl]-o-phenylen}-3, 3-(2, 2-dimethyltrimethylendioxy)-5, 15-androstadien-17β-ol

1,3 g der nach a) hergestellten Substanz werden analog Beispiel 2 b) mit Propin umgesetzt. Nach Chromatographie an Aluminiumoxid (neutral, Stufe III) werden 1,23 g der Titelverbindung als weißer Schaum isoliert.
IR (KBr) : 2230 $cm^{-1}$ Dreifachbindung

c) 17-(Prop-1-inyl)-17β-hydroxy-11β, 19-(4-acetyl-o-phenylen)-4, 15-androstadien-3-on

1,1 g der nach b) hergestellten Substanz werden analog Beispiel 1 c) zur entsprechenden 4-En-3-ketoverbindung gespalten. Nach Chromatographie an Kieselgel werden 617 mg der Titelverbindung als gelblicher Schaum isoliert.
$[\alpha]_D^{22}$ = 114° ($CHCl_3$ ; c = 0,5)
Fp. = 189-191°C (Essigester)

**BEISPIEL 32**

17-Methoxymethyl-17β-hydroxy-11β, 19-(4-acetyl-o-phenylen)-4-androsten-3-on

a) 11β, 19-{4-[1, 1-(2, 2-dimethyltrimethylendioxy)-ethyl]-o-phenylen}-3, 3-(2, 2-dimethyltrimethylendioxy)-5-androsten-[17(β-1')-spiro-3']-oxiran

4 g der nach Beispiel 29 b) hergestellten Verbindung werden analog Beispiel 14 a) mit 7,13 g Trimethylsulfoniumjodid umgesetzt. Nach Chromatographie an Aluminiumoxid (neutral, Stufe III) werden 3,76 g der Titelverbindung als weißer Schaum isoliert.

b) 17-Methoxymethyl-11β, 19-{4-[1, 1-(2, 2-dimethyltrimethylendioxy)-ethyl]-o-phenylen}-3, 3-(2, 2-dimethyltrimethylendioxy)-5-androsten-17β-ol

1,8 g der nach a) hergestellten Substanz werden analog Beispiel 14 b) mit Natriummethylat umgesetzt. Nach Chromatographie an Aluminiumoxid (neutral, Stufe III) werden 1,55 g der Titelverbindung als weißer Schaum isoliert.

c) 17-Methoxymethyl-17β-hydroxy-11β, 19-(4-acetyl-o-phenylen)-4-androsten-3-on

1,5 g der nach b) erhaltenen Substanz werden analog Beispiel 1 c) zur entsprechenden 4-En-3-ketoverbindung gespalten. Nach Chromatographie an Kieselgel werden 561 mg der Titelverbindung als gelblicher Schaum isoliert.
$[\alpha]_D^{22}$ = 76° ($CHCl_3$ ; c = 0,5)

**BEISPIEL 33**

17-Cyanomethyl-17β-hydroxy-11β, 19-(4-acetyl-o-phenylen)-4-androsten-3-on

a) 17-Cyanomethyl-11β, 19-{4-[1, 1-(2, 2-dimethyltrimethylendioxy)-ethyl]-o-phenylen}-3, 3-(2, 2-dimethyltrimethylendioxy)-5-androsten-17β-ol

1,8 g der nach Beispiel 32 a) erhaltenen Substanz werden analog Beispiel 6 b) mit Kaliumcyanid umgesetzt. Nach Chromatographie an Aluminiumoxid (neutral, Stufe III) werden 1,67 g der Titelverbindung als weißer Schaum isoliert.
IR (KBr) : 2250 $cm^{-1}$ C≡N Dreifachbindung

b) 17-Cyanomethyl-17β-hydroxy-11β, 19-(4-acetyl-o-phenylen)-4-androsten-3-on

1,5 g der nach a) erhaltenen Substanz werden analog Beispiel 1 c) zur entsprechenden 4-En-3-ketoverbindung gespalten. Nach Chromatographie an Kieselgel werden 732 mg der Titelverbindung als weißer Schaum isoliert.
$[\alpha]_D^{22}$ = 83° ($CHCl_3$ ; c = 0,5)
Fp. = 184-185°C (Methylenchlorid)

**BEISPIEL 34**

17-(Prop-1-inyl)-17β-hydroxy-11β, 19-(4-isopropenyl-o-phenylen)-4-androsten-3-on

a) 11β, 19-(4-Isopropenyl-o-phenylen)-3, 3-(2, 2-dimethyltrimethylendioxy)-androstan-5α, 17ß-diol

Unter Schutzgas werden 1,94 g Natriumhydrid in 20 ml absolutem Dimethylsulfoxid solange bei 70°C erwärmt, bis keine Gasentwicklung mehr zu erkennen ist. Dann wird die Lösung auf 0°C abgekühlt und tropfenweise mit Methyl-triphenylphosphoniumbromid gelöst in 61 ml absolutem Dimethylsulfoxid versetzt. Nach 1 stündigem Nachrühren bei Raumtemperatur werden 10,3 g der nach Beispiel 28 a) hergestellten Verbindung gelöst in 10 ml absolutem Dimethylsulfoxid zugetropft und das Reaktionsgemisch 3 Stunden nachgerührt. Anschließend wird das Reaktionsgemisch auf kalte gesättigte Natriumhydrogencarbonatlösung gegossen, die wäßrige Phase mit Essigester extrahiert und die organischen Phasen mit gesättigter Natriumchloridlösung gewaschen. Die vereinigten organischen Phasen werden über Natriumsulfat getrocknet und im Vakuum eingeengt. Der Rückstand wird an Aluminiumoxid (neutral, Stufe III) chromatographiert. Man erhält 8,3 g der Titelverbindung als weißen Schaum.
Fp. = 155-157°C (Diisopropylether)

b) 11β, 19-(4-Isopropenyl-o-phenylen)-5α-hydroxy-3, 3-(2, 2-dimethyltrimethylendioxy)-androstan-17-on

8,1 g der nach a) hergestellten Substanz werden analog Beispiel 2 a) mit Chromtrioxid oxidiert. Nach Chromatographie an Aluminiumoxid (neutral, Stufe III) werden 7,8 g der Titelverbindung als weißer Schaum isoliert.
Fp. = 238-240°C (Diisopropylether)

c) 17-(Prop-1-inyl)-11β, 19-(4-isopropenyl-o-phenylen)-3, 3-(2, 2-dimethyltrimethylendioxy)-androstan-5α, 17β-diol

1,5 g der nach b) erhaltenen Substanz werden analog Beispiel 2 b) mit Propin umgesetzt. Nach Chromatographie an Aluminiumoxid (neutral, Stufe III) werden 1,34 g der Titelverbindung als weißer Schaum isoliert.
IR (KBr) : 2240 $cm^{-1}$ Dreifachbindung

d) 17-(Prop-1-inyl)-17β-hydroxy-11β, 19-(4-isopropenyl-o-phenylen)-4-androsten-3-on

1,3 g der nach c) erhaltenen Verbindung werden analog Beispiel 1 c) zur 4-En-3-ketoverbindung umgesetzt. Nach Chromatographie an Kieselgel werden 706 mg der Titelverbindung als weißer Schaum isoliert.
$[\alpha]_D^{22}$ = 41° ($CHCl_3$ ; c = 0,5)
Fp. = 247-250°C (Diisopropylether)

Als Nebenprodukt wird 17-(Prop-1-inyl)-17β-hydroxy-11β,19-[4-(1-methylhydroxyethyl)-o-phenylen]-4-androsten-3-on (354 mg) als weißer Schaum isoliert.
$[\alpha]_D^{22}$ = 17° ($CHCl_3$ ; c = 0,5)
Fp. = 222-224°C

**BEISPIEL 35**

17-(Prop-1-inyl)-17β-hydroxy-11β, 19-(4-isopropyl-o-phenylen)-4-androsten-3-on

a) 11β, 19-(4-Isopropyl-o-phenylen)-5α-hydroxy-3, 3-(2, 2-dimethyltrimethylendioxy)-androstan-17-on

2 g der nach Beispiel 34 b) erhaltenen Substanz werden wie unter Beispiel 23 a) beschrieben, aber nur

bis zur Aufnahme von einem Äquivalent Wasserstoff, mit Palladium als Katalysator hydriert. Nach Chromatographie an Aluminiumoxid (neutral, Stufe III) werden 1,83 g der Titelverbindung als weißer Schaum isoliert.

b) 17-(Prop-1-inyl)-11β, 19-(4-isopropyl-o-phenylen)-3, 3-(2, 2-dimethyltrimethylendioxy)-androstan-5α, 17β-diol

1,8 g der nach a) hergestellten Verbindung werden analog Beispiel 2 b) mit Propin umgesetzt. Nach Chromatographie an Aluminiumoxid (neutral, Stufe III) werden 1,81 g der Titelverbindung als weißer Schaum isoliert.

c) 17-(Prop-1-inyl)-17β-hydroxy-11β, 19-(4-isopropyl-o-phenylen)-4-androsten-3-on

1,7 g der nach b) hergestellten Verbindung werden analog Beispiel 1 c) zur 4-En-3-ketoverbindung gespalten. Nach Chromatographie an Kieselgel werden 932 mg der Titelverbindung als weißer Schaum isoliert.
$[\alpha]_D^{22}$ = 21° ($CHCl_3$ ; c = 0,505)
Fp. = 240-243°C (Essigester)

**BEISPIEL 36**

17-(3-Hydroxyprop-1(Z)-enyl)-17β-hydroxy-11β, 19-(4-isopropenyl-o-phenylen)-4-androsten-3-on

a) 17-[3-(Tetrahydropyran-2-yloxy)-prop-1-inyl]-11β, 19-(4-isopropenyl-o-phenylen)-3, 3-(2, 2-dimethyltrimethylendioxy)-androstan-5α, 17β-diol

2 g der nach Beispiel 34 b) erhaltenen Substanz werden analog Beispiel 5 a) mit 3-(Tetrahydropyran-2-yloxy)-prop-1-in umgesetzt. Nach Chromatographie an Aluminiumoxid (neutral, Stufe III) werden 2,1 g der Titelverbindung als weißer Schaum isoliert.

b) 17-[3-(Tetrahydropyran-2-yloxy)-prop-1(Z)-enyl]-11β, 19-(4-isopropenyl-o-phenylen]-3, 3-(2, 2-dimethyltrimethylendioxy)-androstan-5α, 17β-diol

2 g der nach a) erhaltenen Verbindung werden analog Beispiel 5 b) mit Lindlarkatalysator hydriert. Nach Chromatographie an Aluminiumoxid (neutral, Stufe III) werden 1,78 g der Titelverbindung als weißer Schaum isoliert.

c) 17-(3-Hydroxyprop-1(Z)-enyl)-17β-hydroxy-11β, 19-(4-isopropenyl-o-phenylen)-4-androsten-3-on

1,7 g der nach b) erhaltenen Verbindung werden analog Beispiel 1 c) zur 4-En-3-ketoverbindung gespalten. Nach Chromatographie an Kieselgel werden 567 mg der Titelverbindung als weißer Schaum isoliert.
$[\alpha]_D^{22}$ = 79° ($CHCl_3$ ; c = 0,5)
Fp. = 143-145°C (Essigester)
Als Nebenprodukt werden 178 mg 17-(3-Hydroxyprop-1(Z)-enyl)-17β-hydroxy-11β,19-[4-(1-methyl-1-hydroxyethyl)-o-phenylen]-4-androsten-3-on als weißer Schaum isoliert.
$[\alpha]_D^{22}$ = 61° ($CHCl_3$ ; c = 0,5)
Fp. = 208-211°C (Essigester)

**BEISPIEL 37**

17-(4-Hydroxybut-1(Z)-enyl)-17β-hydroxy-11β, 19-(4-isopropenyl-o-phenylen)-4-androsten-3-on

a) 17-(4-Hydroxybut-1-inyl)-11β, 19-(4-isopropenyl-o-phenylen)-3, 3-(2, 2-dimethyltrimethylendioxy)-androstan-5α, 17β-diol

2 g der nach Beispiel 34 b) erhaltenen Substanz werden in 60 ml absolutem Tetrahydrofuran gelöst und unter Schutzgas nacheinander mit 2,27 ml n-But-1-in-4-ol und 4,09 g Kaliumethylat versetzt. Während der Zugabe und dem nachfolgenden 14 stündigem Nachrühren wird das Reaktionsgemisch bei einer Temperatur von 0°C gehalten. Anschließend wird es auf Wasser gegossen und die wäßrige Phase mit Essigester extrahiert. Die vereinigten organischen Phasen werden mit gesättigter Natriumchloridlösung gewaschen, über Natriumsulfat getrocknet und am Vakuum eingeengt. Der Rückstand wird an Aluminiumoxid (neutral, Stufe III) chromatographiert, und es werden 1,2 g der Titelverbindung als weißer Schaum isoliert.
IR (KBr) : 2240 $cm^{-1}$ Dreifachbindung

b) 17-(4-Hydroxybut-1(Z)-enyl)-11β, 19-(4-isopropenyl-o-phenylen)-3, 3-(2, 2-dimethyltrimethylendioxy)-androstan-5α, 17β-diol

1 g der nach a) erhaltenen Substanz werden analog Beispiel 5 b) unter Verwendung von Lindlarkatalysator hydriert. Nach Chromatographie an Aluminiumoxid (neutral, Stufe III) werden 836 mg der Titelverbindung als weißer Schaum erhalten.

c) 17-(4-Hydroxybut-1(Z)-enyl)-17β-hydroxy-11β, 19-(4-isopropenyl-o-phenylen)-4-androsten-3-on

800 mg der nach b) erhaltenen Substanz werden analog Beispiel 1 c) zur 4-En-3-ketoverbindung gespalten. Nach Chromatographie an Kieselgel werden 306 mg der Titelverbindung als weißer Schaum isoliert.
$[\alpha]_D^{22}$ = 78° ($CHCl_3$ ; c = 0,515)

**BEISPIEL 38**

17-(Prop-1-inyl)-17β-hydroxy-11β, 19-(4-diethoxyphosphoryl-o-phenylen)-4-androsten-3-on

a) 11β, 19-(4-Diethoxyphosphoryl-o-phenylen)-5α-hydroxy-3, 3-(2, 2-dimethyltrimethylendioxy)-androstan-17-on

1,5 g des nach Beispiel 26 b) erhaltenen Triflats werden in 10 ml absolutem Triethylamin gelöst und unter Schutzgas mit 122 mg Tetrakistriphenylphosphinpalladium und 0,46 ml Phosphorigsäurediethylester versetzt. Anschließend wird das Reaktionsgemisch 1,5 Stunden unter Rückfluß erhitzt. Nach Zugabe von weiteren 100 mg Tetrakistriphenylphosphinpalladium und 0,46 ml Phosphorigsäurediethylester erhitzt man das Reaktionsgemisch weitere 1,5 Stunden zum Rückfluß, läßt es dann auf Raumtemperatur abkühlen und engt es am Vakuum ein. Der Rückstand wird an Kieselgel chromatographiert und ergibt 1,05 g der Titelverbindung als weißen Schaum.

b) 17-(Prop-1-inyl)-11β, 19-(4-diethoxyphosphoryl)-o-phenylen)-3, 3-(2, 2-dimethyltrimethylendioxy)-androstan-5α, 17β-diol

1 g der nach a) hergestellten Verbindung werden analog Beispiel 2 b) mit Propin umgesetzt. Chromatographie an Aluminiumoxid (neutral, Stufe III) führen zu 832 mg der Titelverbindung als weißem Schaum.
IR (KBr) : 2240 $cm^{-1}$ Dreifachbindung

c) 17-(Prop-1-inyl)-17β-hydroxy-11β, 19-(4-diethoxyphosphoryl-o-phenylen)-4-androsten-3-on

800 mg der nach b) hergestellten Substanz werden analog Beispiel 1 c) zur 4-En-3-ketoverbindung gespalten. Nach Chromatographie an Kieselgel werden 440 mg der Titelverbindung als weißer Schaum isoliert.
$[\alpha]_D^{22}$ = 17° ($CHCl_3$ ; c = 0,5)

**BEISPIEL 39**

17-(Prop-1-inyl)-17β-hydroxy-11β, 19-[4-(2-thienyl)-o-phenylen]-4-androsten-3-on

a) 11β, 19-[4-(2-Thienyl)-o-phenylen]-3, 3-(2, 2-dimethyltrimethylendioxy)-androstan-5α, 17β-diol

1,26 g der nach Beispiel 21 a) hergestellten Substanz werden in 36 ml absolutem Dioxan gelöst und unter Schutzgas mit 3,04 ml Hexa-n-butyldizinn, 254 mg Lithiumchlorid und 100 mg Tetrakistriphenylphosphinpalladium versetzt. Anschließend wird das Reaktionsgemisch auf 110°C erwärmt und 1 Stunde bei dieser Temperatur gehalten, bevor 1,94 ml 2-Bromthiophen zugegeben werden und weitere 18 Stunden bei 110°C nachgerührt wird. Dann wird das Reaktionsgemisch auf Raumtemperatur gebracht und über Celite filtriert. Nach Einengen des Filtrats wird der Rückstand an Kieselgel chromatographiert. Man erhält 545 mg der Titelverbindung als gelblichen Schaum.

Exemplarisch für diese Art der Kupplung wurde folgende Zinnverbindung isoliert :

α) 11β, 19-(4-Tri-n-butylstannyl-o-phenylen)-3, 3-(2, 2-dimethyltrimethylendioxy)-androstan-5α, 17β-diol

1,26 g der nach Beispiel 21 a) hergestellten Substanz werden analog den unter a) beschriebenen Bedingungen mit 3,04 ml Hexa-n-butyldizinn umgesetzt. Nach dem 1 stündigen Erwärmen auf 110°C wird das Reaktionsgemisch normal aufgearbeitet. Chromatographie an Kieselgel führt zu 625 mg der Titelverbindung als weißem Schaum.

$[\alpha]_D^{22}$ = 25° ($CHCl_3$ ; c = 0,5)

Fp. = 137-139°C (Diisopropylether)

b) 11β, 19-[4-(2-Thienyl)-o-phenylen)-5α-hydroxy-3, 3-(2, 2-dimethyltrimethylendioxy)-androstan-17-on

400 mg N-Chlorsuccinimid werden bei 0°C in 5 ml absolutem Methylenchlorid vorgelegt. Nach Zutropfen von 0,3 ml Dimethylsulfid wird das Gemisch 30 Minuten bei 0°C nachgerührt. Anschließend werden 510 mg der nach a) erhaltenen Substanz gelöst in 5 ml absolutem Methylenchlorid langsam zugetropft. Nach 2 stündigem Nachrühren unter Feuchtigkeitsausschluß werden 0,6 ml Triethylamin zum Reaktionsgemisch zugetropft. Dann wird es auf Wasser gegossen, die wäßrige Phase mit Methylenchlorid extrahiert und die organische Phase mit gesättigter Natriumchloridlösung gewaschen. Anschließend wird sie über Natriumsulfat getrocknet und am Vakuum eingeengt. Nach Chromatographie des Rückstandes an Aluminiumoxid (neutral, Stufe III) werden 387 mg der Titelverbindung als gelblicher Schaum isoliert.

IR (KBr) : 1740 $cm^{-1}$ Fünfringketon

c) 17-(Prop-1-inyl)-11β, 19-[4-(2-thienyl)-o-phenylen]-3, 3-(2, 2-dimethyltrimethylendioxy)-androstan-5α, 17β-diol

350 mg der nach b) erhaltenen Substanz werden analog Beispiel 2 b) mit Propin umgesetzt. Nach Chromatographie an Aluminiumoxid (neutral, Stufe III) werden 343 mg der Titelverbindung als gelblicher Schaum isoliert.

IR (KBr) : 2240 $cm^{-1}$ Dreifachbindung

d) 17-(Prop-1-inyl)-17β-hydroxy-11β, 19-[4-(2-thienyl)-o-phenylen]-4-androsten-3-on

320 mg der nach d) erhaltenen Verbindung werden analog Beispiel 1 c) zur 4-En-3-ketoverbindung gespalten. Nach Chromatographie an Kieselgel werden 234 mg der Titelverbindung als gelblicher Schaum isoliert.

$[\alpha]_D^{22}$ = 65° ($CHCl_3$ ; c = 0,5)

**BEISPIEL 40**

17-(Prop-1-inyl)-17β-hydroxy-11β, 19-[4-(3-thienyl)-o-phenylen]-4-androsten-3-on

a) 11β, 19-[4-(3-Thienyl)-o-phenylen]-5α-hydroxy-3, 3-(2, 2-dimethyltrimethylendioxy)-androstan-17-on

1,26 der nach Beispiel 26 b) erhaltenen Substanz werden analog den unter Vorschrift Beispiel 39 a) genannten Bedingungen mit 2 ml 3-Bromthiophen umgesetzt. Nach Chromatographie an Aluminiumoxid (neutral, Stufe III) werden 546 mg der Titelverbindung als gelblicher Schaum isoliert.

b) 17-(Prop-1-inyl)-11β, 19-[4-(3-thienyl)-o-phenylen]-3, 3-(2, 2-dimethyltrimethylendioxy)-androstan-5α, 17β-diol

520 mg der nach a) hergestellten Substanz werden analog Beispiel 2 b) mit Propin umgesetzt. Nach Chromatographie an Aluminiumoxid (neutral, Stufe III) werden 462 mg der Titelverbindung als gelblicher Schaum isoliert.

IR (KBr) : 2250 $cm^{-1}$ Dreifachbindung

c) 17-(Prop-1-inyl)-17β-hydroxy-11β, 19-[4-(3-thienyl)-o-phenylen]-4-androsten-3-on

410 mg der nach b) erhaltenen Substanz werden analog Beispiel 1 c) zur 4-En-3-ketoverbindung gespalten. Nach Chromatographie an Kieselgel werden 287 mg der Titelverbindung als gelblicher Schaum isoliert.
$[\alpha]_D^{22}$ = 61° ($CHCl_3$ ; c = 0,51)

**BEISPIEL 41**

17-(Prop-1-inyl)-17β-hydroxy-11β, 19-[4-(3-furyl)-o-phenylen]-4-androsten-3-on

a) 11β, 19-[4-(3-Furyl)-o-phenylen]-5α-hydroxy-3, 3-(2, 2-dimethyltrimethylendioxy)-androstan-17-on

1,26 g der nach Beispiel 26 b) hergestellten Substanz werden analog Beispiel 39 a) mit 1,8 ml 3-Bromfuran umgesetzt. Nach Chromatographie an Kieselgel werden 660 mg der Titelverbindung als weißer Schaum isoliert.
Fp. = 240-243°C (Essigester)

b) 17-(Prop-1-inyl)-11β, 19-[4-(3-furyl)-o-phenylen]-3, 3-(2, 2-dimethyltrimethylendioxy)-androstan-5α, 17β-diol

630 mg der nach a) erhaltenen Substanz werden analog Beispiel 2 b) mit Propin umgesetzt. Nach Chromatographie an Aluminiumoxid (neutral, Stufe III) werden 615 mg der Titelverbindung als weißer Schaum isoliert.
IR (KBr) : 2240 $cm^{-1}$ Dreifachbindung

c) 17-(Prop-1-inyl)-17β-hydroxy-11β, 19-[4-(3-furyl)-o-phenylen]-4-androsten-3-on

590 mg der nach b) erhaltenen Substanz werden analog Beispiel 1 c) zur 4-En-3-ketoverbindung umgesetzt. Nach Chromatographie an Kieselgel werden 289 mg der Titelverbindung als weißer Schaum isoliert.
$[\alpha]_D^{22}$ = 49° ($CHCl_3$ ; c = 0,51)

**BEISPIEL 42**

17-(Prop-1-inyl)-17β-hydroxy-11β, 19-[4-(3-methoxyphenyl)-o-phenylen]-4-androsten-3-on

a) 11β, 19-[4-(3-Methoxyphenyl)-o-phenylen]-5α-hydroxy-3, 3-(2, 2-dimethyltrimethylendioxy)-androstan-17-on

1,26 g der nach Beispiel 26 b) hergestellten Substanz werden analog Beispiel 39 a) mit 2,53 ml 3-Bromanisol umgesetzt. Nach Chromatographie an Kieselgel werden 685 mg der Titelverbindung als weißer Schaum isoliert.

b) 17-(Prop-1-inyl)-11β, 19-[4-(3-methoxyphenyl)-o-phenylen]-3, 3-(2, 2-dimethyltrimethylendioxy)-androstan-5α, 17β-diol

650 mg der unter a) hergestellten Substanz werden analog der unter Beispiel 2 b) beschriebenen Vorschrift mit Propin umgesetzt. Nach Chromatographie an Aluminiumoxid (neutral, Stufe III) werden 635 mg der Titelverbindung als weißer Schaum isoliert.
IR (KBr) : 2230 $cm^{-1}$ Dreifachbindung

c) 17-(Prop-1-inyl)-17β-hydroxy-11β, 19-[4-(3-methoxyphenyl)-o-phenylen]-4-androsten-3-on

600 mg der unter b) dargestellten Substanz werden analog den unter Beispiel 1 c) beschriebenen Bedingungen zur 4-En-3-ketoverbindung umgesetzt. Nach Chromatographie an Kieselgel werden 366 mg der Titelverbindung als weißer Schaum isoliert.
$[\alpha]_D^{22}$ = 66° ($CHCl_3$ ; c = 0,5)
Fp. = 158-162°C (Essigester/Hexan)

**BEISPIEL 43**

17-(Prop-1-inyl)-17β-hydroxy-11β, 19-[4-(4-methoxyphenyl)-o-phenylen]-4-androsten-3-on

a) 11β, 19-[4-(4-Methoxyphenyl)-o-phenylen]-5α-hydroxy-3, 3-(2, 2-dimethyltrimethylendioxy)-androstan-17-on

1,26 der nach Vorschrift Beispiel 26 b) hergestellten Substanz werden analog Beispiel 39 a) mit 2,53 ml 4-Bromanisol umgesetzt. Nach Chromatographie an Kieselgel werden 522 mg der Titelverbindung als weißer Schaum isoliert.
$[\alpha]_D^{22} = 48°$ ($CHCl_3$ ; c = 0,5)
Fp. = 171-173°C (Essigester)

b) 17-(Prop-1-inyl)-11β, 19-[4-(4-methoxyphenyl)-o-phenylen]-3, 3-(2, 2-dimethyltrimethylendioxy)-androstan-5α, 17β-diol

500 mg der unter a) hergestellten Substanz werden analog der unter Beispiel 2 b) beschriebenen Vorschrift mit Propin umgesetzt. Nach Chromatographie an Aluminiumoxid (neutral, Stufe III) werden 498 mg der Titelverbindung als weißer Schaum isoliert.
IR (KBr) : 2240 $cm^{-1}$ Dreifachbindung

c) 17-(Prop-1-inyl)-17β-hydroxy-11β, 19-[4-(4-methoxyphenyl)-o-phenylen]-4-androsten-3-on

450 mg der unter b) hergestellten Substanz werden analog Beispiel 1 c) zur 4-En-3-ketoverbindung umgesetzt. Nach Chromatographie an Kieselgel werden 276 mg der Titelverbindung als weißer Schaum isoliert.
$[\alpha]_D^{22} = 70°$ ($CHCl_3$ ; c = 0,505)
Fp. = 165-169°C (Essigester/Hexan)

**BEISPIEL 44**

17-(Prop-1-inyl)-17β-hydroxy-11β, 19-[4-(2-methoxyphenyl)-o-phenylen]-4-androsten-3-on

a) 11β, 19-[4-(2-Methoxyphenyl)-o-phenylen]-5α-hydroxy-3, 3-(2, 2-dimethyltrimethylendioxy)-androstan-17-on

1,26 g der nach Beispiel 26 b) erhaltenen Substanz werden analog Beispiel 39 a) mit 2,53 ml 2-Bromanisol umgesetzt. Nach Chromatographie an Kieselgel werden 448 mg der Titelverbindung als weißer Schaum isoliert.

b) 17-(Prop-1-inyl)-11β, 19-[4-(2-methoxyphenyl)-o-phenylen]-3, 3-(2, 2-dimethyltrimethylendioxy)-androstan-5α, 17β-diol

410 mg der nach a) hergestellten Substanz werden analog Beispiel 2 b) mit Propin umgesetzt. Nach Chromatographie an Aluminiumoxid (neutral, Stufe III) werden 405 mg der Titelverbindung als weißer Schaum isoliert.
IR (KBr) : 2240 $cm^{-1}$ Dreifachbindung

c) 17-(Prop-1-inyl)-17β-hydroxy-11β, 19-[4-(2-methoxyphenyl)-o-phenylen]-4-androsten-3-on

380 mg der nach b) hergestellten Substanz werden analog Beispiel 1 c) zur 4-En-3-ketoverbindung umgesetzt. Nach Chromatographie an Kieselgel werden 205 mg der Titelverbindung als weißer Schaum isoliert.
$[\alpha]_D^{22} = 49°$ ($CHCl_3$ ; c = 0,51)

**BEISPIEL 45**

17-(Prop-1-inyl)-17β-hydroxy-11β, 19-(4, 5-methylendioxy-o-phenylen)-4-androsten-3-on

a) 19-(2-Chlor-4,5-methylendioxyphenyl)-3, 3-(2, 2-dimethyltrimethylendioxy)-9(11)-androsten-5α, 17β-diol

Analog Beispiel 1 a) werden aus 10 g 5α,10α-Epoxy-3,3-(2,2-dimethyltrimethylendioxy)-9(11)-estren-17β-ol durch Umsetzung mit 6-Chlorpiperonylchlorid 10,3 g 19-(2-Chlor-4,5-methylendioxyphenyl)-3,3-(2,2-dimethyltrimethylendioxy)-9(11)-androsten-5α,17β-diol erhalten.

b) 11β, 19-(4, 5-Methylendioxy-o-phenylen)-3, 3-(2, 2-dimethyltrimethylendioxy)-androstan-5α, 17β-diol

Analog Beispiel 1 b) werden aus 10 g der unter a) erhaltenen Verbindung 5,9 g 11β,19-(4,5-Methylendioxy-o-phenylen)-3,3-(2,2-dimethyltrimethylendioxy)-androstan-5α,17β-diol als weißer Schaum hergestellt.

c) 11β, 19-(4, 5-Methylendioxy-o-phenylen)-5α-hydroxy-3, 3-(2, 2-dimethyltrimethylendioxy)-androstan-17-on

Analog Beispiel 2 a) werden 5,5 g der unter b) erhaltenen Verbindung in die entsprechende Ketoverbindung überführt. Es werden 4,2 g 11β,19-(4,5-Methylendioxy-o-phenylen)-5α-hydroxy-3,3-(2,2-dimethyltrimethylendioxy)-androstan-17-on als weißer Schaum erhalten.
$[\alpha]_D^{22}$ = +45° ($CHCl_3$ ; c = 0,525)
Fp. : = 219-222°C (Ethylacetat)

d) 17-(Prop-1-inyl)-11β, 19-(4, 5-methylendioxy-o-phenylen)-3, 3-(2, 2-dimethyltrimethylendioxy)-androstan-5α, 17β-diol

Analog Beispiel 2 b) werden 4 g der unter c) erhaltenen Verbindung in die entsprechende 17α-Propinylverbindung überführt. Es werden nach der Chromatographie 3,5 g 17-(Prop-1-inyl)-11β,19-(4,5-methylendioxy-o-phenylen)-3,3-(2,2-dimethyltrimethylendioxy)-androstan-5α,17β-diol als weißer Schaum isoliert.
IR (KBr) : 2230 $cm^{-1}$ Dreifachbindung

e) 17-(Prop-1-inyl)-17β-hydroxy-11β, 19-(4, 5-methylendioxy-o-phenylen)-4-androsten-3-on

Analog Beispiel 1 c) werden 3 g der unter d) erhaltenen Verbindung zur entsprechenden 4-En-3-ketoverbindung umgesetzt. Es werden 1,36 g 17-(Prop-1-inyl)-17β-hydroxy-11β,19-(4,5-methylendioxy-o-phenylen)-4-androsten-3-on isoliert.
$[\alpha]_D^{22}$ = +2° ($CHCl_3$ ; c = 0,485)

**Patentansprüche**

1. 19,11β-Überbruckte Steroide der allgemeinen Formel I

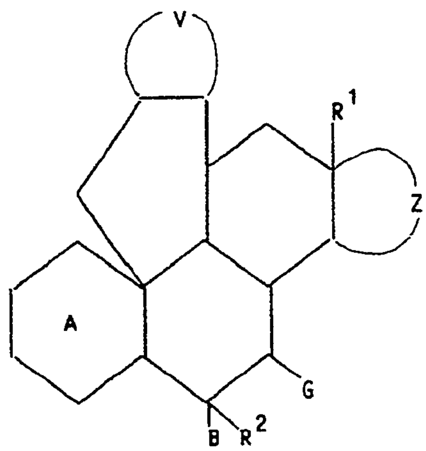

(I),

worin

| | |
|---|---|
| $R^1$ | für einen Methyl- oder Ethylrest, |
| $R^2$ | für ein Wasserstoff-, Chloratom, einen $C_1$-$C_4$-Alkylrest, |
| B und G, | die gleich oder verschieden sind, jeweils für ein Wasserstoffatom, einen $C_1$-$C_4$-Alkylrest oder gemeinsam für eine zweite Bindung zwischen den Kohlenstoffatomen 6 und 7, |
| B und $R^2$ | gemeinsam für eine Methylen- oder Ethylengruppe, |
| Z | für den Rest eines Ringes der Formel |

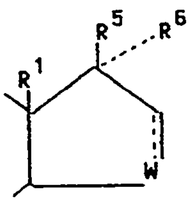

steht, worin

| | |
|---|---|
| $R^1$ | die in Formel I genannte Bedeutung hat, die von W ausgehende gestrichelte Linie die etwaige Anwesenheit einer Doppelbindung bedeutet, |

W einen $CH_2$-, CH-, $CH_2CH_2$- oder $CHCH_2$-Rest,

$R^5/R^6$

$-OR^7/-C \equiv C-U$

$-OR^7/-\underset{\underset{O}{\|}}{C}-CH_2-R^8$

$-\underset{\underset{O}{\|}}{C}-CH_2-R^8/-OR^7$

$-\underset{\underset{O}{\|}}{C}-CH_2-R^8/-CH_3$

$-\underset{\underset{O}{\|}}{C}-CH_2-R^8/-H$

$-OR^7/-(CH_2)_m-CH_2-R^9$

$-OR^7/-CH=CH(CH_2)_k-CH_2-R^9$

$-OR^{10}/-H$

$-OR^{10}/-(CH_2)_k-C \equiv C-U$

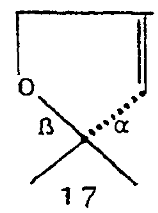

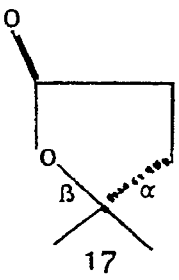

mit

$R^7$ in der Bedeutung eines Wasserstoffatoms oder Acylrestes mit 1 bis 4 Kohlenstoffatomen,

U in der Bedeutung eines Wasserstoffatoms, einer Alkyl-, Hydroxyalkyl-, Alkoxyalkyl-, Acyloxyalkylgruppe mit Jeweils 1 bis 4 Kohlenstoffatomen im Alkyl- bzw. Acylrest oder eines Halogenatoms,

$R^8$ in der Bedeutung eines Wasserstoffatoms, einer Hydroxygruppe, einer Alkyl-, O-Alkyl- oder O-Acylgruppe mit jeweils 1 bis 4 Kohlenstoffatomen,

$R^9$ in der Bedeutung eines Wasserstoffatoms, eines Hydroxy- oder Cyanidrestes, einer O-Alkyl- oder O-Acylgruppe mit jeweils 1 bis 4 Kohlenstoffatomen,

$R^{10}$ in der Bedeutung eines Wasserstoffatoms, einer Alkyl- oder Acylgruppe mit jeweils 1 bis 10 Kohlenstoffatomen,

m in der Bedeutung 0, 1, 2 oder 3,

k in der Bedeutung 0, 1 oder 2, bedeuten

V für den Rest eines Phenylrings der Formel

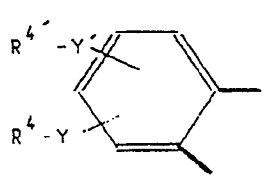

oder für den Rest eines fünf- oder sechsgliedrigen heteroaromtischen Rings mit 1 bis 2 N—, O—oder S-Atomen der Formel

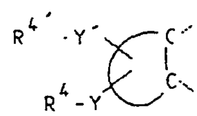

,

worin

$R^4$ und $R^{4'}$, die gleich oder verschieden sind, jeweils ein Wasserstoffatom, einen Cyanidrest, eine $—OR^{11}—$, $—S(O)_kR^{11}—$, $—N(O)_nR^{11}R^{12}—$, $—O—SO_2R^{13}—$, $—P(O)(OR^{14})_2$, $—SiR^{14}_3$ oder $—SnR^{14}_3$-Gruppe mit k in der Bedeutung der Ziffern 0, 1 oder 2, n in der Bedeutung der Ziffern 0 oder 1,

$R^{11}$ in der Bedeutung eines Wasserstoffatoms oder eines $C_1$-$C_8$-Alkylrestes,

$R^{12}$ in der Bedeutung von $R^{11}$, eines Cyanid- oder eines $C_1$-$C_{10}$-Acylrestes,

$R^{13}$ in der Bedeutung eines perfluorierten $C_1$-$C_4$-Alkylrestes,

$R^{14}$ in der Bedeutung eines $C_1$-$C_4$-Alkylrestes oder

$R^{11}$ und $R^{12}$ innerhalb einer $—N(O)_nR^{11}R^{12}$-Gruppe gemeinsam unter Einschluß von N einen 5- oder 6gliedrigen heterocyclischen Ring, wobei im Ring noch ein weiteres Heteroatom N, O oder S enthalten sein kann,

Y und Y', die gleich oder verschieden sind, jeweils für Eine direkte Bindung, eine geradkettige oder verzweigte, gegebenenfalls Doppel- oder Dreifachbindung(en) aufweisende Alkylengruppe mit bis zu 20 Kohlenstoffatomen, die gegebenenfalls substituiert ist durch eine oder mehrere Oxo-, $C_1$-$C_{10}$-Acyloxy-, $—OR^{11}—$, $—S(O)_kR^{11}—$ und/oder $—N(O)_nR^{11}R^{12}$-Gruppe(n), ein gegebenenfalls substituierter Arylenrest oder

$R^4$-Y und $R^{4'}$-Y' gemeinsam den Rest eines, gegebenenfalls substituierten gesättigten, ungesättigten

oder aromatischen 5- oder 6-gliedrigen Ringes mit 0 bis 2 Sauerstoff-, Schwefelatomen und/oder $NR^{11}$-Gruppen bedeuten, mit der Maßgabe, daß nur dann k und n größer als 0 sind, wenn $R^{11}$ für einen $C_1$-$C_8$-Alkylrest steht und der Ring A für

a)

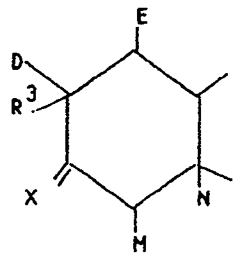

,

wobei

M und N gemeinsam eine zweite Bindung oder M ein Wasserstoffatom und N eine Hydroxygruppe, wobei dann B, $R^2$, G, $R^3$ D und E Wasserstoffatome sind und
X ein Sauerstoffatom, zwei Wasserstoffatome oder eine Hydroxyiminogruppierung N ~OH,
$R^3$ und D, die gleich oder verschieden sind, jeweils ein Wasserstoffatom, einen Nitrilrest oder einen $C_1$-$C_4$-Alkylrest oder gemeinsam eine Methylen- oder Ethylengruppe,
E ein Wasserstoffatom, einen $C_1$-$C_4$-Alkylrest,
D und E gemeinsam eine zweite Bindung zwischen den Kohlenstoffatomen 1 und 2 oder gemeinsam eine Methylengruppe bedeuten
oder

b)

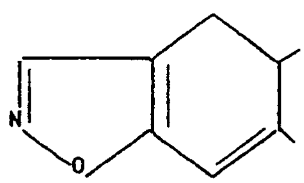

oder

c)

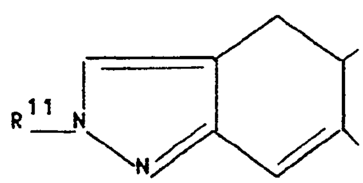

mit $R^{11}$ in der Bedeutung eines Wasserstoffatoms oder eines $C_1$-$C_8$-Alkylrestes stehen, sowie gegebenenfalls deren pharmazeutisch verträgliche Additionssalze mit Säuren.

2. Verbindungen nach Anspruch 1, dadurch gekennzeichnet, daß $R^2$, B und G jeweils für ein Wasserstoffatom stehen.

3. Verbindungen nach Anspruch 1, dadurch gekennzeichnet, daß B und G gemeinsam für eine zweite Bindung und $R^2$ für ein Wasserstoffatom stehen.

4. Verbindungen nach Anspruch 1, dadurch gekennzeichnet, daß Y und Y' jeweils für eine direkte Bindung und $R^4$ und $R^{4'}$ jeweils für ein Wasserstoffatom stehen.

5. Verbindungen nach Anspruch 1, dadurch gekennzeichnet, daß Y und Y' jeweils für eine direkte Bindung, $R^4$ für ein Wasserstoffatom und $R^{4'}$ für ein Stickstoffatom, substituiert mit zwei $C_1$-$C_8$-Alkylresten, stehen.

6. Verbindungen nach Anspruch 1, dadurch gekennzeichnet, daß Y und Y' jeweils für eine direkte Bindung, $R^4$ für ein Wasserstoffatom und $R^{4'}$ für eine $C_1$-$C_8$-Alkoxygruppe stehen.

7. Verbindungen nach Anspruch 1, dadurch gekennzeichnet, daß Y für eine direkte Bindung, $R^4$ und $R^{4'}$

jeweils für ein Wasserstoffatom und Y' für eine geradkettige oder verzweigte, gegebenenfalls Doppel- und/oder Dreifachbindung(en) aufweisende Alkylengruppe mit bis zu 20 Kohlenstoffatomen, die durch eine Oxo- oder $OR^{11}$-Gruppe mit $R^{11}$ in der Bedeutung eines Wasserstoffatoms oder eines $C_1$-$C_8$-Alkylrestes, substituiert ist, stehen.

8. Verbindungen nach Anspruch 1, dadurch gekennzeichnet, daß $R^4$-Y und $R^{4'}$-Y' gemeinsam für den Rest eines gesättigten, ungesättigten oder aromatischen 5- oder 6-gliedrigen Ringes mit 0 bis 2 Sauerstoff-, Schwefelatomen und/oder $NR^{11}$-Gruppen mit $R^{11}$ in der Bedeutung eines Wasserstoffatoms oder eines $C_1$-$C_8$Alkylrestes stehen.

9. Verbindungen nach Anspruch 1, dadurch gekennzeichnet, daß Y'-$R^{4'}$ ein Wasserstoffatom und Y-$R^4$ eine Ethyl-, Vinyl-, Isopropyl-,Isopropenyl-, Prop-1(Z)-enyl-, Prop-1(E)-enyl-, Prop-2-enyl-, Ethinyl-. Propinyl-, Prop-2-inylMethoxy-, Thiomethyl-, Thioethyl-, 1-Hydroxyethyl- oder Diethoxyphosphorylgruppe, ein substituierter oder unsubstituierter carbocyclischer oder heterocyclischer Arylrest ist.

10. Verbindungen nach Anspruch 9, dadurch gekennzeichnet, daß der Arylrest ein Phenyl-, Naphthyl-, 2-Methoxyphenyl-, 3-Methoxyphenyl-, 4-Methoxyphenyl-, 2-Tolyl-, 3-Tolyl-, 4-Tolyl-, 2-Dimethylamino-, 3-Dimethylamino-, 4-Dimethylamino-, Furyl-2-, Furyl-3-, Thienyl-2-, Thienyl-3-, Pyridyl-2-, Pyridyl-3-, Pyridyl-4-, Thiazolyl-, Imidazolyl-Rest ist.

11. Verbindungen nach Anspruch 1, dadurch gekennzeichnet, daß der 5- oder 6gliedrige heteroaromatische Ring ein Furyl-, Thienyl-, Pyridyl-, Pyrazolyl-, Pyrrolyl-, Pyrimidinyl-, Oxazolyl-, Pyridazinyl-, Pyrazinyl-Rest ist.

12. Verbindungen der allgemeinen Formel II

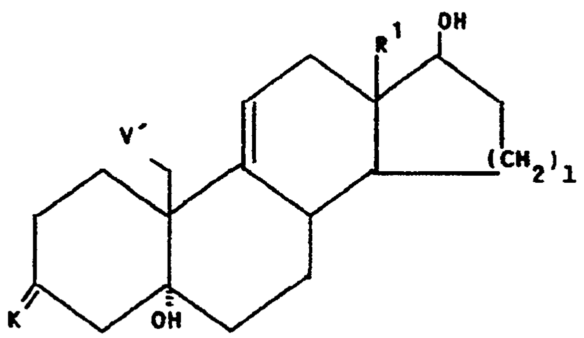

(II),

worin

| | |
|---|---|
| $R^1$ | einen Methyl- oder Ethylrest |
| l | die Ziffern 1 oder 2, |
| K | eine in Form des Ketals oder Thioketals blockierte Ketogruppe bedeuten und |
| V' | für einen Phenylring der Formel |

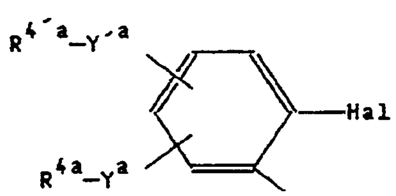

oder für einen 5- oder 6gliedrigen heteroaromatischen Ring mit 1-2 N—, O— oder S-Atomen der Formel

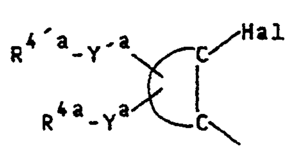

steht,
worin

Hal ein Fluor-, Chlor-, Brom- oder Jodatom bedeutet,

$R^{4a}$, $R^{4'a}$, $Y^a$, $Y'^a$ unter Ausschluß des Cyanidrestes die gleiche Bedeutung wie $R^4$, $R^{4'}$, Y und Y' in Formel I haben, wobei gegebenenfalls vorhandene Hydroxy-, Mercapto-, Amino-, Oxo- und/oder terminale Acetylengruppen geschützt sind.

13. 19,11β-Überbrückte Steroide der allgemeinen Formel IV

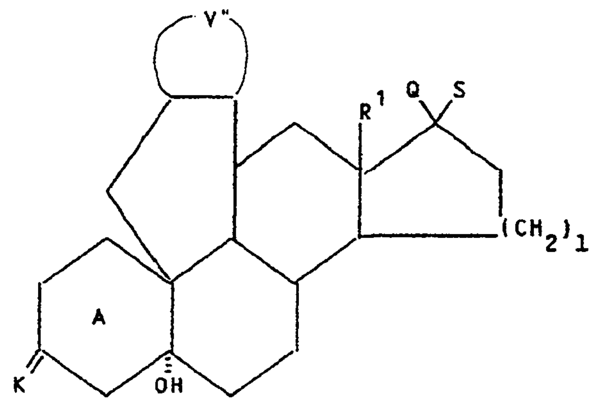

(IV),

worin

R¹ einen Methyl- oder Ethylrest,
l die Ziffern 1 oder 2,
K eine in Form des Ketals oder Thioketals blockierte Ketogruppe bedeuten und
V'' für einen Phenylring der Formel

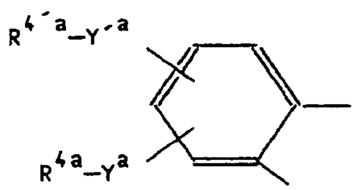

oder für einen 5- oder 6gliedrigen heteroaromatischen Ring mit 1-2 N—, O— oder S-Atomen der Formel

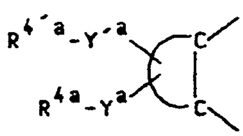

steht,

worin

$R^{4a}$, $R^{4'a}$, $Y^a$ und $Y'^a$ unter Ausschluß des Cyanidrestes die gleiche Bedeutung wie $R^4$, $R^{4'}$, Y und Y' in Formel I haben, wobei gegebenenfalls vorhandene Hydroxy-, Mercapto-, Amino-, Oxo- und/oder terminale Acetylengruppen geschützt sind, sowie Q für $R^5$ und S für $R^6$ oder aber gemeinsam für ein Ketosauerstoffatom stehen.

14. Verfahren zur Herstellung von Verbindungen der allgemeinen Formel I

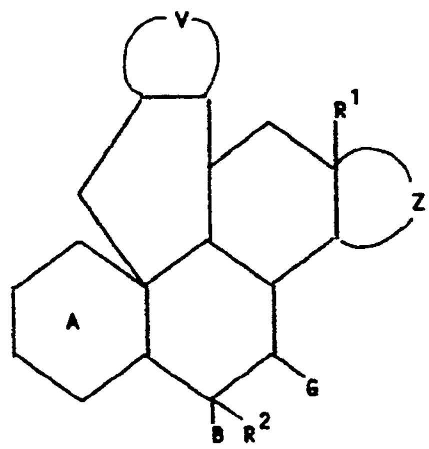

(I),

worin

| | |
|---|---|
| $R^1$ | für einen Methyl- oder Ethylrest, |
| $R^2$ | für ein Wasserstoff-, Chloratom, einen $C_1$-$C_4$-Alkylrest, |
| B und G, | die gleich oder verschieden sind, jeweils für ein Wasserstoffatom, einen $C_1$-$C_4$-Alkylrest oder gemeinsam für eine zweite Bindung zwischen den Kohlenstoffatomen 6 und 7, |
| B und $R^2$ | gemeinsam für eine Methylen- oder Ethylengruppe, |
| Z | für den Rest eines Ringes der Formel |

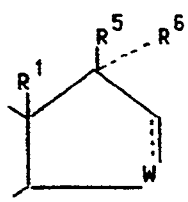

steht, worin

| | |
|---|---|
| $R^1$ | die in Formel I genannte Bedeutung hat, die von W ausgehende gestrichelte Linie die etwaige Anwesenheit einer Doppelbindung bedeutet, |

W einen $CH_2$-, CH-, $CH_2CH_2$- oder $CHCH_2$-Rest,

$R^5/R^6$
$-OR^7/-C\equiv C-U$
$-OR^7/-\underset{\overset{\|}{O}}{C}-CH_2-R^8$
$-\underset{\overset{\|}{O}}{C}-CH_2-R^8/-OR^7$
$-\underset{\overset{\|}{O}}{C}-CH_2-R^8/-CH_3$
$-\underset{\overset{\|}{O}}{C}-CH_2-R^8/-H$
$-OR^7/-(CH_2)_m-CH_2-R^9$
$-OR^7/-CH=CH(CH_2)_k-CH_2-R^9$
$-OR^{10}/-H$
$-OR^{10}/-(CH_2)_k-C\equiv C-U$

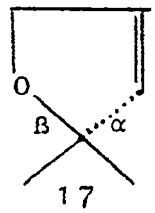

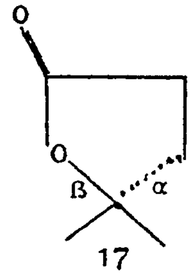

mit

R7 in der Bedeutung eines Wasserstoffatoms oder Acylrestes mit 1 bis 4 Kohlenstoffatomen, U in der Bedeutung eines Wasserstoffatoms, einer Alkyl-, Hydroxyalkyl-, Alkoxyalkyl-, Acyloxyalkylgruppe mit jeweils 1 bis 4 Kohlenstoffatomen im Alkyl- bzw. Acylrest oder eines Halogenatoms,

$R^8$ in der Bedeutung eines Wasserstoffatoms, einer Hydroxygruppe, einer Alkyl-, O-Alkyl- oder O-Acylgruppe mit jeweils 1 bis 4 Kohlenstoffatomen,

$R^9$ in der Bedeutung eines Wasserstoffatoms, eines Hydroxy- oder Cyanidrestes, einer O-Alkyl- oder O-Acylgruppe mit jeweils 1 bis 4 Kohlenstoffatomen,

$R^{10}$ in der Bedeutung eines Wasserstoffatoms, einer Alkyl- oder Acylgruppe mit jeweils 1 bis 10 Kohlenstoffatomen,

m in der Bedeutung 0, 1, 2 oder 3,

k in der Bedeutung 0, 1 oder 2 bedeuten,

V für den Rest eines Phenylrings der Formel

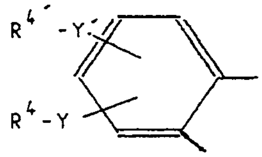

oder für den Rest eines fünf- oder sechsgliedrigen heteroaromatischen Rings mit 1 bis 2 N—, O— oder S-Atomen der Formel

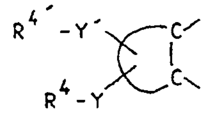,

worin

$R^4$ und $R^{4'}$, die gleich oder verschieden sind, jeweils ein Wasserstoffatom einen Cyanidrest, eine $—OR^{11}—$, $—S(O)_kR^{11}—$, $—N(O)_nR^{11}R^{12}—$, $—O\text{-}SO_2R^{13}—$, $—P(O)(OR^{14})_2$, $—SiR^{14}_3$ oder $—SnR^{14}_3$-Gruppe mit k in der Bedeutung der Ziffern 0, 1 oder 2, n in der Bedeutung der Ziffern 0 oder 1,

$R^{11}$ in der Bedeutung eines Wasserstoffatoms oder eines $C_1$-$C_8$-Alkylrestes

$R^{12}$ in der Bedeutung von $R^{11}$, eines Cyanid- oder eines $C_1$-$C_{10}$-Acylrestes,

$R^{13}$ in der Bedeutung eines perfluorierten $C_1$-$C_4$-Alkylrestes,

$R^{14}$ in der Bedeutung eines $C_1$-$C_4$-Alkylrestes oder

$R^{11}$ und $R^{12}$ innerhalb einer $—N(O)_nR^{11}R^{12}$-Gruppe gemeinsam unter Einschluß von N einen 5- oder 6gliedrigen heterocyclischen Ring, wobei im Ring noch ein weiteres Heteroatom N, O oder S enthalten sein kann,

Y und Y′, die gleich oder verschieden sind, jeweils für eine direkte Bindung, eine geradkettige oder verzweigte, gegebenenfalls Doppel- oder Dreifachbindung(en) aufweisende Alkylengruppe mit bis zu 20 Kohlenstoffatomen, die gegebenenfalls substituiert ist durch eine oder mehrere Oxo-, $C_1$-$C_{10}$-Acyloxy-, $—OR^{11}—$, $—S(O)_kR^{11}—$ und/oder $—N(O)_nR^{11}R^{12}$-Gruppe(n), ein gegebenenfalls substituierter Arylenrest oder

$R^4$-Y und $R^{4'}$-Y' gemeinsam den Rest eines, gegebenenfalls substituierten gesättigten, ungesättigten oder aromatischen 5- oder 6-gliedrigen Ringes mit 0 bis 2 Sauerstoff-, Schwefelatomen und/oder $NR^{11}$-Gruppen bedeuten, mit der Maßgabe, daß nur dann k und n größer als 0 sind, wenn $R^{11}$ für einen $C_1$-$C_8$-Alkylrest steht und der Ring A für

a)

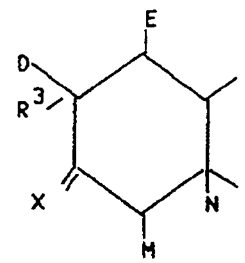

,

wobei

M und N gemeinsam eine zweite Bindung oder M ein Wasserstoffatom und N eine Hydroxygruppe, wobei dann B, $R^2$, G, $R^3$ D und E Wasserstoffatome sind und

X ein Sauerstoffatom, zwei Wasserstoffatome oder eine Hydroxyiminogruppierung N ~OH,

$R^3$ und D, die gleich oder verschieden sind, jeweils ein Wasserstoffatom, einen Nitrilrest oder einen $C_1$-$C_4$-Alkylrest oder gemeinsam eine Methylen- oder Ethylengruppe,

E ein Wasserstoffatom, einen $C_1$-$C_4$-Alkylrest,

D und E gemeinsam eine zweite Bindung zwischen den Kohlenstoffatomen 1 und 2 oder gemeinsam eine Methylengruppe bedeuten

oder

b)

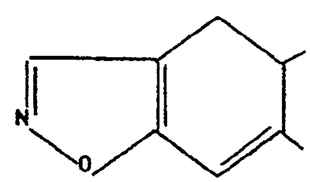

oder

c)

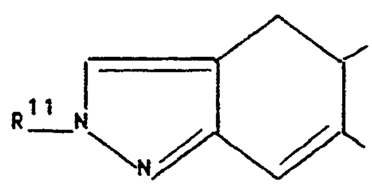

mit $R^{11}$ in der Bedeutung eines Wasserstoffatoms oder eines $C_1$-$C_8$-Alkylrestes stehen, sowie gegebenenfalls deren pharmazeutisch verträgliche Additionssalze mit Säuren, dadurch gekennzeichnet, daß man Verbindungen der allgemeinen Formel II

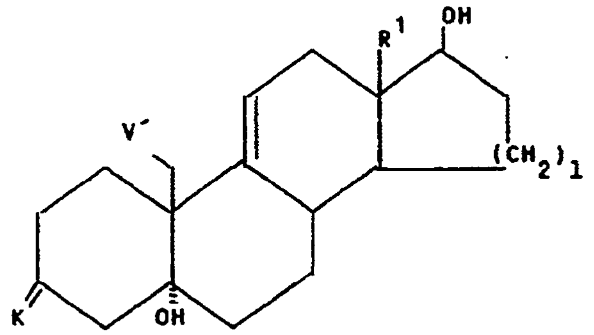

(II),

worin

- $R^1$ eines Methyl- oder Ethylrest
- l die Ziffern 1 oder 2,
- K eine in Form des Ketals oder Thioketals blockierte Ketogruppe bedeuten und
- V' für einen Phenylring der Formel

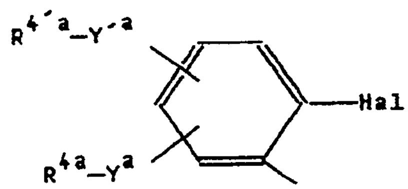

oder für einen 5- oder 6gliedrigen heteroaromatischen Ring mit 1-2 N—, O— oder S-Atomen der Formel

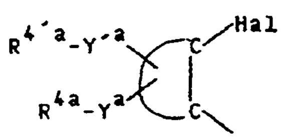

steht,
worin

Hal ein Fluor-, Chlor-, Brom- oder Jodatom bedeutet,

$R^{4a}$, $R^{4'a}$, $Y^a$ und $Y'^a$ unter Ausschluß des Cyanidrestes die gleiche Bedeutung wie $R^4$, $R^{4'}$, Y und Y' haben, wobei gegebenenfalls vorhandene Hydroxy-, Mercapto-, Amino-, Oxo- und/oder terminale Acetylengruppen geschützt sind, durch Behandlung mit einem elektropositiven Metall in flüssigem Ammoniak, gemischt mit einem oder mehreren organischen Lösungsmitteln, oder wenn der n-Halogensubstituent in der Verbindung der allgemeinen Formel II ein Brom- oder Jodatom ist, auch reduktiv radikalisch zu den Zwischenprodukten der allgemeinen Formel IVa cyclisiert

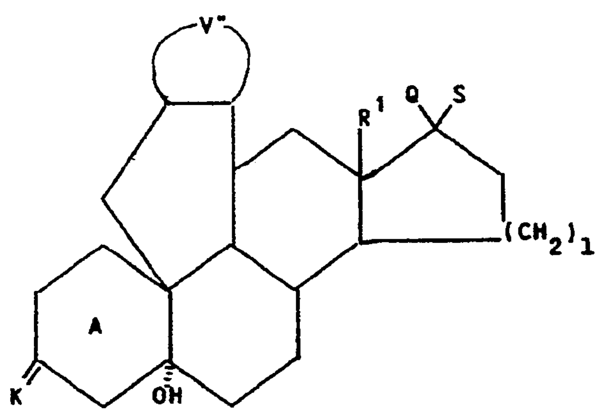

(IVa),

worin

V″ für einen Phenylring der Formel

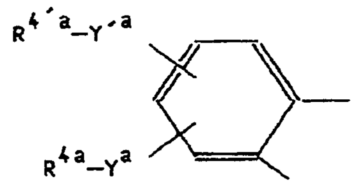

oder für einen 5- oder 6gliedrigen heteroaromatischen Ring mit 1-2 N—, O— oder S-Atomen der Formel

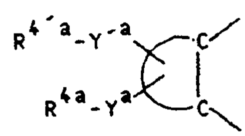

steht,

worin

$R^{4a}$, $R^{4'a}$, $Y^a$ und $Y'^a$ unter Ausschluß des Cyanidrestes die gleiche Bedeutung wie $R^4$, $R^{4'}$, Y und Y′ haben, wobei gegebenenfalls vorhandene Hydroxy-, Mercapto-, Amino-, Oxo- und/oder terminale Acetylengruppen geschützt sind sowie Q ausschließlich eine β-Hydroxygruppe und S ausschließlich ein α-Wasserstoffatom bedeuten, und dann entweder zuerst

a) die C-17-Hydroxygruppe gegebenenfalls oxidiert und anschließend

b) gegebenenfalls eine eine Schutzgruppe aufweisende Hydroxygruppe in V′ von dieser Schutzgruppe befreit, gewünschtenfalls aus der Hydroxyverbindung ein entsprechendes Perfluoralkylsulfonat herstellt, gegebenenfalls das Perfluoralkylsulfonat entweder direkt oder durch Austausch der Perfluoralkylsulfonatabgangsgruppe gegen eine Zinntrialkylgruppe über die entsprechende Zinntrialkylverbindung in eine Verbindung überführt, die in V″, gegebenenfalls nach weiteren Reaktionen, das gewünschte Substitutionsmuster aufweist,

oder zuerst b) und dann a) ausführt und danach

c) den Ring D in gewünschter Weise nach an sich bekannten Methoden funktionalisiert, das so erhaltene Produkt der Einwirkung eines Dehydratationsmittels, das auch zur Freisetzung der 3-Oxogruppe befähigt ist, zur Wasserabspaltung unter gleichzeitiger Ausbildung der 4(5)-Doppelbindung unterwirft und anschließend, gegebenenfalls nach erneutem Schutz von intermediär freigesetzten, in V und/oder Z enthaltenen funktionellen Gruppen, die gewünschten Funktionen der Ringe A und B des Steroidgerüstes einführt oder

d) das so erhaltene Produkt der Einwirkung eines Dehydratationsmittels, das auch zur Freisetzung der 3-Oxogruppe befähigt ist zur Wasserabspaltung unter gleichzeitiger Ausbildung der 4(5)-Doppelbindung unterwirft, die gewünschten Funktionen der Ringe A und B des Steroidgerüstes einführt und anschließend, nach Schutz der 3-Oxogruppe, den Ring D in gewünschter Weise funktionalisiert,

oder die Schritte a) und b) nach dem Schritt c) oder d) ausführt,

das so erhaltene Produkt gegebenenfalls von Schutzgruppen befreit, gewünschtenfalls die in V gegebenenfalls enthaltene(n) Hydroxy-, Mercapto- und/oder Aminogruppe(n) alkyliert bzw. acyliert, gewünschtenfalls einen Cyanidrest in den/die Arylsubstituenten einführt, gewünschtenfalls die in dem(n) Arylsubstituenten gegebenenfalls enthaltene(n) Amino- und/oder Sulfidgruppe(n) oxidiert, gewünschtenfalls mit Hydroxylamin-hydrochlorid zum Produkt der allgemeinen Formel I mit X in der Bedeutung der Hydroxyiminogruppierung N ~OH umsetzt sowie gegebenenfalls ein pharmazeutisch verträgliches Säureadditionssalz herstellt.

15. Verfahren nach Anspruch 14, dadurch gekennzeichnet, daß mit Natrium, Lithium, Kalium oder Calcium als elektropositiven Metall cyclisiert wird.

16. Pharmazeutische Präparate, gekennzeichnet durch einen Gehalt an Verbindungen gemäß den Ansprüchen 1 bis 11.

17. Verwendung von Verbindungen gemäß den Ansprüchen 1 bis 13 zur Herstellung von Arzneimitteln.

**Claims**

1. 19,11β-bridged steroids of the general formula I

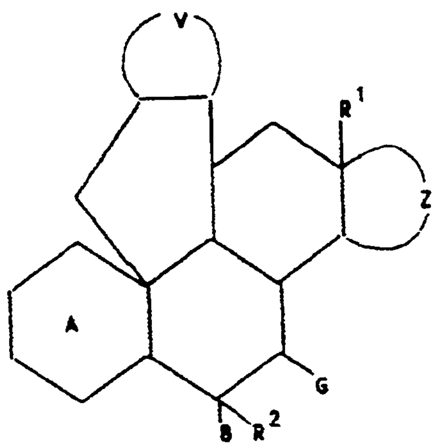

(I),

in which

| | |
|---|---|
| $R^1$ | represents a methyl or ethyl radical, |
| $R^2$ | represents a hydrogen atom, a chlorine atom or a $C_1$-$C_4$alkyl radical, |
| B and G, | which are the same or different, each represents a hydrogen atom or a $C_1$-$C_4$alkyl radical, or together represent a second bond between carbon atoms 6 and 7 |
| B and $R^2$ | together represent a methylene or ethylene group, |
| Z | represents the radical of a ring of the formula |

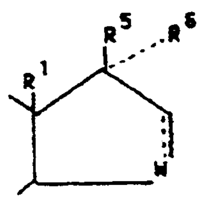

in which

| | |
|---|---|
| $R^1$ | is as defined for formula I, the broken line from W represents the possible presence of a double bond, |
| W | represents a $CH_2$, CH, $CH_2CH_2$ or $CHCH_2$ radical, $R^5$/$R^6$ represent |

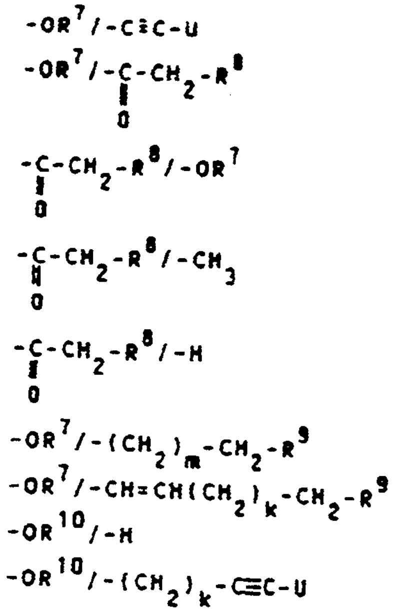

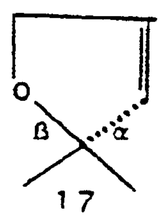

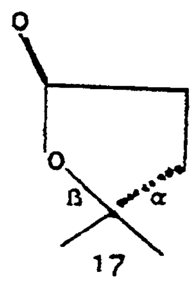

wherein

R^7 represents a hydrogen atom or an acyl radical having from 1 to 4 carbon atoms,

U represents a hydrogen atom, or an alkyl, hydroxyalkyl, alkoxyalkyl or acyloxyalkyl group each having from 1 to 4 carbon atoms in the alkyl or acyl moiety, as the case may be, or a halogen atom,

R^8 represents a hydrogen atom, a hydroxy group, or an alkyl, O-alkyl or O-acyl group each having from 1 to 4 carbon atoms,

R^9 represents a hydrogen atom, a hydroxy or cyanide radical, or an O-alkyl or O-acyl group each having from 1 to 4 carbon atoms,

R^10 represents a hydrogen atom, or an alkyl or acyl group each having from 1 to 10 carbon atoms,

m represents 0, 1, 2 or 3,

k represents 0, 1 or 2,

V represents the radical of a phenyl ring of the formula

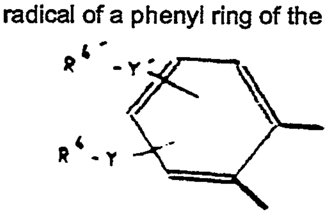

or the radical of a five- or six-membered heteroaromatic ring having 1 or 2 N, O or S atoms of the formula

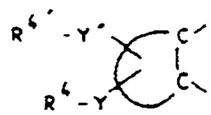

wherein

| | |
|---|---|
| $R^4$ and $R^{4'}$, | which are the same or different, each represents a hydrogen atom, a cyanide radical, an $-OR^{11}$, $-S(O)_kR^{11}$, $-N(O)_nR^{11}R^{12}$, $-O-SO_2R^{13}$, $-P(O)(OR^{14})_2$, $-SiR^{14}_3$ or $-SnR^{14}_3$ group in which k represents the number 0, 1 or 2 and n represents the number 0 or 1, |
| $R^{11}$ | represents a hydrogen atom or a $C_1$-$C_8$alkyl radical, |
| $R^{12}$ | represents $R^{11}$, a cyanide or a $C_1$-$C_{10}$acyl radical, |
| $R^{13}$ | represents a perfluorinated $C_1$-$C_4$alkyl radical, |
| $R^{14}$ | represents a $C_1$-$C_4$alkyl radical or |
| $R^{11}$ and $R^{12}$ | together, within an $-N(O)_nR^{11}R^{12}$ group, represent including N a 5- or 6-membered heterocyclic ring that may contain an additional hetero atom N, O or S, |
| Y and Y', | which are the same or different, each represents a direct bond, a straight-chain or branched alkylene group having up to 20 carbon atoms and optionally containing double or triple bond(s), which is optionally substituted by one or more oxo, $C_1$-$C_{10}$acyloxy, $-OR^{11}$, $-S(O)_kR^{11}$ and/or $-N(O)_nR^{11}R^{12}$ group(s), or an optionally substituted arylene radical or |
| $R^4$-Y and $R^{4'}$-Y' | together represent the radical of an optionally substituted, saturated, unsaturated or aromatic 5- or 6-membered ring having from 0 to 2 oxygen atoms, sulphur atoms and/or $NR^{11}$ groups, with the proviso that k and n are greater than 0 only when $R^{11}$ represents a $C_1$-$C_8$alkyl radical and the ring A represents |

a)

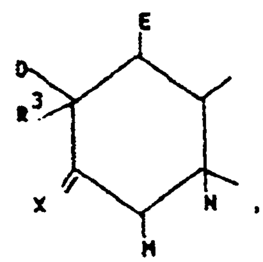

wherein

| | |
|---|---|
| M and N | together represent a second bond, or M represents a hydrogen atom and N represents a hydroxy group, in which case B, $R^2$, G, $R^3$, D and E are hydrogen atoms, and |
| X | represents an oxygen atom, two hydrogen atoms or a hydroxyimino grouping N ~OH, |
| $R^3$ and D, | which are the same or different, each represents a hydrogen atom, a nitrile radical or a $C_1$-$C_4$alkyl radical, or together represent a methylene or ethylene group, |
| E | represents a hydrogen atom or a $C_1$-$C_4$alkyl radical, |
| D and E | together represent a second bond between carbon atoms 1 and 2, or together represent a methylene group, or |

b)

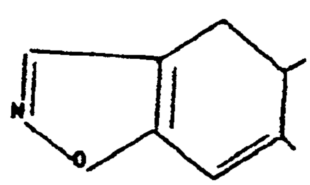

or

c)

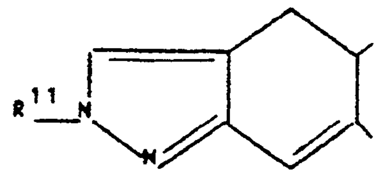

in which $R^{11}$ represents a hydrogen atom or a $C_1$-$C_8$alkyl radical, and optionally their pharmaceutically acceptable addition salts with acids.

2. Compounds according to claim 1, characterised in that each of $R^2$, B and G represents a hydrogen atom.

3. Compounds according to claim 1, characterised in that B and G together represent a second bond and $R^2$ represents a hydrogen atom.

4. Compounds according to claim 1, characterised in that each of Y and Y′ represents a direct bond and each of $R^4$ and $R^{4'}$ represents a hydrogen atom.

5. Compounds according to claim 1, characterised in that each of Y and Y′ represents a direct bond, $R^4$ represents a hydrogen atom and $R^{4'}$ represents a nitrogen atom, substituted by two $C_1$-$C_8$alkyl radicals.

6. Compounds according to claim 1, characterised in that each of Y and Y′ represents a direct bond, $R^4$ represents a hydrogen atom and $R^{4'}$ represents a $C_1$-$C_8$alkoxy group.

7. Compounds according to claim 1, characterised in that Y represents a direct and, each of $R^4$ and $R^{4'}$ represents a hydrogen atom and Y′ represents a straight-chain or branched alkylene group having up to 20 carbon atoms and optionally containing double and/or triple bond(s), which is substituted by an oxo or $OR^{11}$ group in which $R^{11}$ represents a hydrogen atom or a $C_1$-$C_8$alkyl radical.

8. Compounds according to claim 1, characterised in that $R^4$-Y and $R^{4'}$-Y′ together represent the radical of a saturated, unsaturated or aromatic 5- or 6-membered ring having from 0 to 2 oxygen atoms, sulphur atoms and/or $NR^{11}$ groups in which $R^{11}$ represents a hydrogen atom or a $C_1$-$C_8$alkyl radical.

9. Compounds according to claim 1, characterised in that Y′-$R^{4'}$ represents a hydrogen atom and Y-$R^4$ represents an ethyl, vinyl, isopropyl, isopropenyl, prop-1(Z)-enyl, prop-1(E)-enyl, prop-2-enyl, ethynyl, propynyl, prop-2-ynyl, methoxy, thiomethyl, thioetbyl, 1-hydroxyethyl or diethoxypbosphoryl group, or a substituted or unsubstituted carboyclic or heterocyclic aryl radical.

10. Compounds according to claim 9, characterised in that the aryl radical is a phenyl, naphthyl, 2-methoxyphenyl, 3-methoxyphenyl, 4-metboxyphenyl, 2-tolyl, 3-tolyl, 4-tolyl, 2-dimethylamino, 3-dimethylamino, 4-dimethylamino, 2-furyl, 3-furyl, 2-thienyl, 3-thienyl, 2-pyridyl, 3-pyridyl, 4-pyridyl, thiazolyl or imidazolyl radical.

11. Compounds according to claim 1, characterised in that the 5- or 6-membered heteroaromatic ring is a furyl, thienyl, pyridyl, pyrazolyl, pyrrolyl, pyrimidinyl, oxazolyl, pyridazinyl or pyrazinyl radical.

12. Compounds of the general formula II

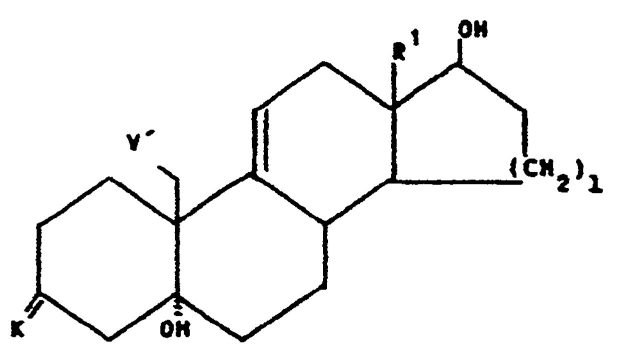

(II)

in which

- $R^1$ represents a methyl or ethyl radical,
- l represents the number 1 or 2,
- K represents a keto group blocked in the form of the ketal or thioketal, and
- V′ represents a phenyl ring of the formula

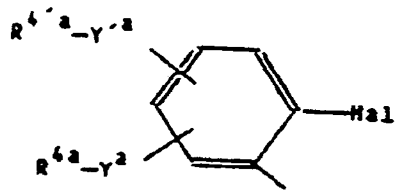

or a 5- or 6-membered heteroaromatic ring having 1 or 2 N, O or S atoms of the formula

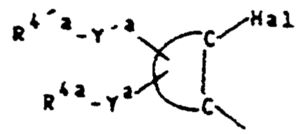

wherein

Hal represents a fluorine, chlorine, bromine or iodine atom,

$R^{4a}$, $R^{4'a}$, $Y^a$ and $Y'^a$ have the same meaning as $R^4$, $R^{4'}$, Y and Y′ in the formula I, with the exclusion of the cyanide radical, wherein hydroxy, mercapto, amino, oxo and/or terminal acetylene groups that may be present are protected.

13. 19,11β-bridged steroids of the general formula IV

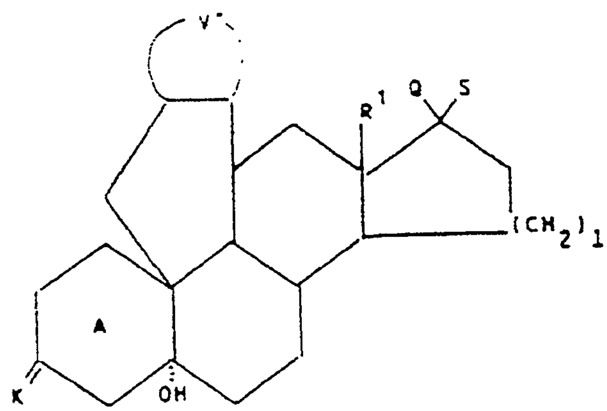

(IV)

in which

$R^1$ represents a methyl or ethyl radical,

l represents the number 1 or 2,

K represents a keto group blocked in the form of the ketal or thioketal, and

V″ represents a phenyl ring of the formula

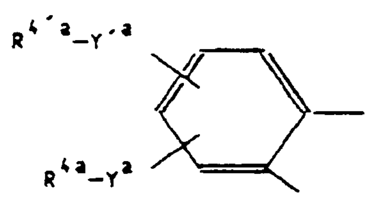

or a 5- or 6-membered heteroaromatic ring having 1 or 2 N, O or S atoms of the formula

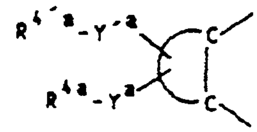

in which

$R^{4a}$, $R^{4'a}$, $Y^a$ and $Y'^a$ have the same meaning as $R^4$, $R^{4'}$, Y and Y' in the formula I, with the exclusion of the cyanide radical, wherein hydroxy, mercapto, amino, oxo and/or terminal acetylene groups that may be present are protected, and Q represents $R^5$ and S represents $R^6$ or Q and S together represent a keto oxygen atom.

14. Process for the manufacture of compounds of the general formula I

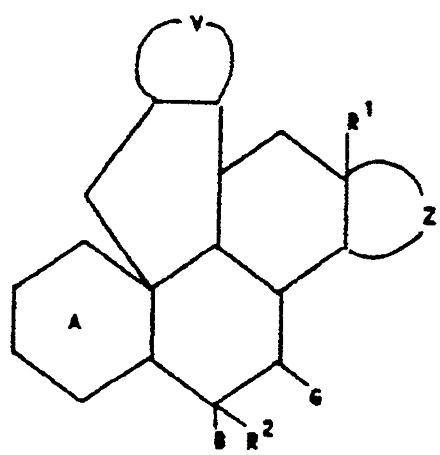

(I),

in which

| | |
|---|---|
| $R^1$ | represents a methyl or ethyl radical, |
| $R^2$ | represents a hydrogen atom, a chlorine atom or a $C_1$-$C_4$alkyl radical, |
| B and G, | which are the same or different, each represents a hydrogen atom or a $C_1$-$C_4$alkyl radical, or together represent a second bond between carbon atoms 6 and 7, |
| B and $R^2$ | together represent a methylene or ethylene group, |
| Z | represents the radical of a ring of the formula |

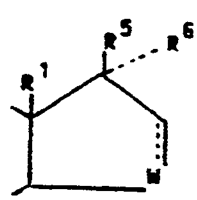

in which

| | |
|---|---|
| $R^1$ | is as defined for formula I, the broken line from W represents the possible presence of a double bond, |
| W | represents a $CH_2$, CH, $CH_2CH_2$ or $CHCH_2$ radical, $R^5$/$R^6$ represent |

$-OR^7/-C\equiv C-U$

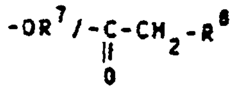

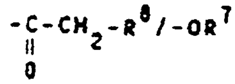

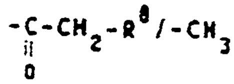

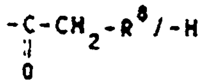

$-OR^7/-(CH_2)_m-CH_2-R^9$

$-OR^7/-CH=CH(CH_2)_k-CH_2-R^9$

$-OR^{10}/-H$

$-OR^{10}/-(CH_2)_k-C\equiv C-U$

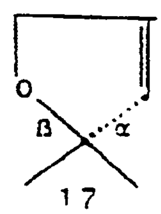

17

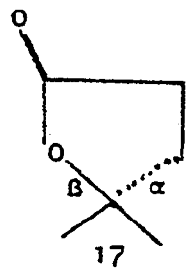

17

wherein

| | |
|---|---|
| $R^7$ | represents a hydrogen atom or an acyl radical having from 1 to 4 carbon atoms, |
| U | represents a hydrogen atom, or an alkyl, hydroxyalkyl, alkoxyalkyl or acyloxyalkyl group each having from 1 to 4 carbon atoms in the alkyl or acyl moiety, as the case may be, or a halogen atom, |
| $R^8$ | represents a hydrogen atom, a hydroxy group, or an alkyl, O-alkyl or O-acyl group each having from 1 to 4 carbon atoms, |
| $R^9$ | represents a hydrogen atom, a hydroxy or cyanide radical, or an O-alkyl or O-acyl group each having from 1 to 4 carbon atoms, |
| $R^{10}$ | represents a hydrogen atom, or an alkyl or acyl group each having from 1 to 10 carbon atoms, |
| m | represents 0, 1, 2 or 3, |
| k | represents 0, 1 or 2, |
| V | represents the radical of a phenyl ring of the formula |

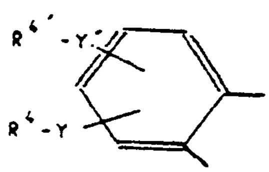

or the radical of a five- or six-membered heteroaromatic ring having 1 or 2 N, O or S atoms of the formula

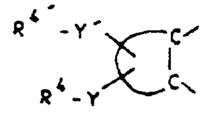,

wherein

R⁴ and R⁴′, — which are the same or different, each represents a hydrogen atom, a cyanide radical, an $—OR^{11}$, $—S(O)_kR^{11}$, $—N(O)_nR^{11}R^{12}$, $—O\text{-}SO_2R^{13}$, $—P(O)(OR^{14})2$, $—SiR^{14}_3$ or $—SnR^{14}_3$ group in which <u>k</u> represents the number 0, 1 or 2 and <u>n</u> represents the number 0 or 1,

$R^{11}$ — represents a hydrogen atom or a $C_1$-$C_8$alkyl radical,

$R^{12}$ — represents $R^{11}$, a cyanide or a $C_1$-$C_{10}$acyl radical,

$R^{13}$ — represents a perfluorinated $C_1$-$C_4$alkyl radical,

$R^{14}$ — represents a $C_1$-$C_4$alkyl radical or

$R^{11}$ and $R^{12}$ — together, within an $—N(O)_nR^{11}R^{12}$ group, represent including N a 5- or 6-membered heterocyclic ring that may contain an additional hetero atom N, O or S,

Y and Y′, — which are the same or different, each represents a direct bond, a straight-chain or branched alkylene group having up to 20 carbon atoms and optionally containing double or triple bond(s), which is optionally substituted by one or more oxo, $C_1$-$C_{10}$acyloxy, $—OR^{11}$, $—S(O)_kR^{11}$ and/or $—N(O)_nR^{11}R^{12}$ group(s), or an optionally substituted arylene radical or

$R^4$-Y and $R^{4\prime}$-Y′ — together represent the radical of an optionally substituted, saturated, unsaturated or aromatic 5- or 6-membered ring having from 0 to 2 oxygen atoms, sulphur atoms and/or $NR^{11}$ groups, with the proviso that <u>k</u> and <u>n</u> are greater than 0 only when $R^{11}$ represents a $C_1$-$C_8$alkyl radical and the ring A represents

a)

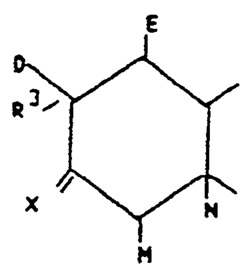

wherein

M and N — together represent a second bond, or M represents a hydrogen atom and N represents a hydroxy group, in which case B, $R^2$, G, $R^3$, D and E are hydrogen atoms, and

X — represents an oxygen atom, two hydrogen atoms or a hydroxyimino grouping N ~OH,

$R^3$ and D, — which are the same or different, each represents a hydrogen atom, a nitrile radical or a $C_1$-$C_4$alkyl radical, or together represent a methylene or ethylene group,

E — represents a hydrogen atom or a $C_1$-$C_4$alkyl radical,

D and E — together represent a second bond between carbon atoms 1 and 2, or together represent a methylene group, or

b)

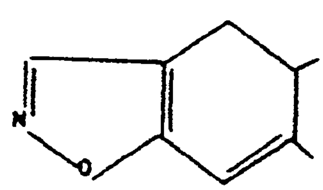

or

c)

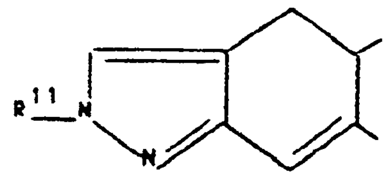

in which $R^{11}$ represents a hydrogen atom or a $C_1$-$C_8$alkyl radical, and optionally their pharmaceutically acceptable addition salts with acids, characterised in that compounds of the general formula II

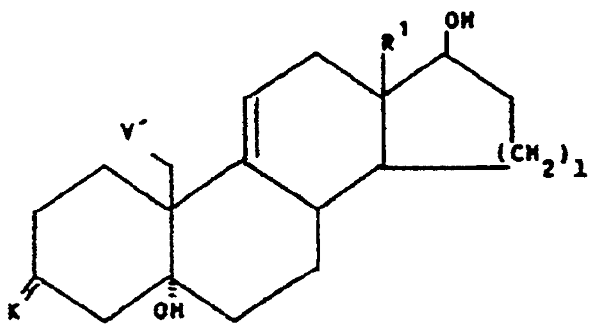

(II),

in which

- $R^1$ represents a methyl or ethyl radical
- l represents the number 1 or 2,
- K represents a keto group blocked in the form of the ketal or thioketal, and and
- V′ represents a phenyl ring of the formula

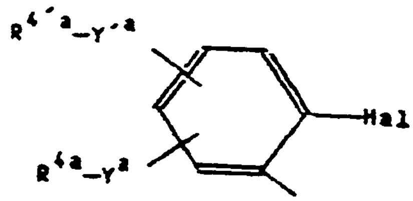

or a 5- or 6-membered heteroaromatic ring having 1 or 2 N, O or S atoms of the formula

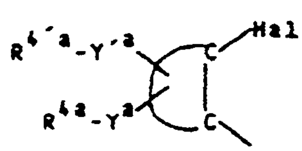

wherein

Hal represents a fluorine, chlorine, bromine or iodine atom,

$R^{4a}$, $R^{4'a}$, $Y^a$ and $Y'^a$ have the same meaning as $R^4$, $R^{4'}$, Y and Y′, with the exclusion of the cyanide radical, wherein hydroxy, mercapto, amino, oxo and/or terminal acetylene groups that may be present are protected, are cyclised by treatment with an electropositive metal in liquid ammonia, mixed with one or more organic solvents, or, if the a-halogen substituent in the compound of the general formula II is a bromine or iodine atom, also by radical reduction, to form the intermediates of the general formula IVa

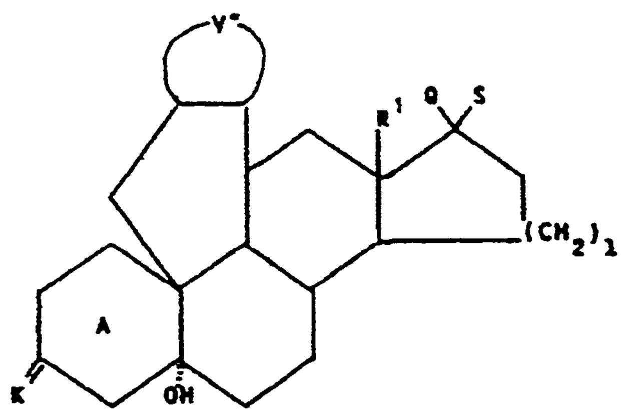

(IVa),

in which

V″ represents a phenyl ring of the formula

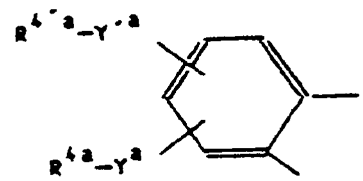

or a 5- or 6-membered heteroaromatic ring having 1 or 2 N, O or S atoms of the formula

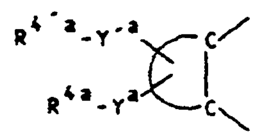

in which

$R^{4a}$, $R^{4'a}$, $Y^a$ and $Y'^a$ have the same meaning as $R^4$, $R^{4'}$, Y and Y′, with the exclusion of the cyanide radical, wherein hydroxy, mercapto, amino, oxo and/or terminal acetylene groups that may be present are protected, and Q represents exclusively a β-hydroxy group and S exclusively an α-hydrogen atom and then, either first

a) the C-17 hydroxy group is optionally oxidised and then

b) optionally a hydroxy group in V′ having a protecting group is freed from that protecting group, if desired a corresponding perfluoroalkyl sulphonate is produced from the hydroxy compound, optionally the perfluoroalkyl sulphonate is converted, either directly or by exchange of the perfluoroalkyl sulphonate leaving group for a tin trialkyl group by way of the corresponding tin trialkyl compound, into a compound that contains in V″, optionally after further reactions, the desired substitution pattern,
or first b) is carried out and then a), and subsequently

c) the ring D is functionalised in the desired manner according to methods known per se, the resulting product is subjected to the action of a dehydration agent that is also capable of freeing the 3-oxo group, for the removal of water with the simultaneous formation of the 4(5) double bond and then, optionally after renewed protection of intermediately freed functional groups contained in V and/or Z, the desired functions of rings A and B of the steroid structure are introduced, or

d) the resulting product is subjected to the action of a dehydrating agent that is also capable of freeing the 3-oxo group, for the removal of water with the simultaneous formation of the 4(5) double bond, the desired functions of rings A and B of the steroid structure are introduced and then, after protection of the 3-oxo group, ring D is functionalised in desired manner,

or steps a) and b) are carried out after step c) or d),

the resulting product is optionally freed from protecting groups, if desired the hydroxy, mercapto and/or amino group(s) that may be contained in V are alkylated or acylated, if desired a cyanide radical is introduced into the aryl substituent(s), if desired the amino and/or sulphide group(s) that may be contained in the aryl substituent(s) are oxidised, if desired reacted with hydroxylamine hydrochloride to form a product of the general

formula I in which X represents the hydroxyimino grouping N ~OH, and optionally a pharmaceutically acceptable acid addition salt is manufactured.

15. Process according to claim 14, characterised in that the cyclisation is carried out with sodium, lithium, potassium or calcium as the electropositive metal.

16. Pharmaceutical preparations, characterised by a content of compounds according to claims 1 to 11.

17. Use of compounds according to claims 1 to 13 for the manufacture of medicaments.

## Revendications

1. Stéroïdes à pont 19,11β répondant à la formule générale I :

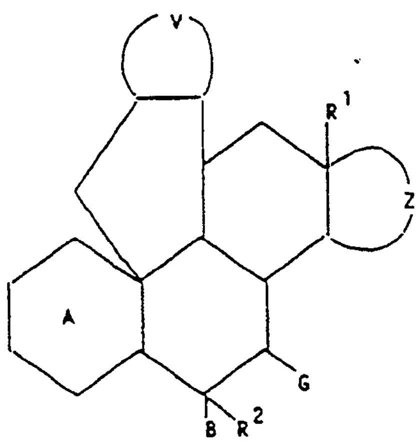

(I), (I)

dans laquelle :

| | |
|---|---|
| $R^1$ | représente un radical méthyle ou éthyle, |
| $R^2$ | représente un atome d'hydrogène ou de chlore ou un radical alkyle en $C_1$-$C_4$, |
| B et G, | qui sont identiques ou différents, représentent chacun un atome d'hydrogène ou un alkyle en $C_1$-$C_4$, ou forment ensemble une deuxième liaison entre les atomes de carbone 6 et 7, |
| B et $R^2$ | représentent ensemble un radical méthylène ou éthylène, |
| Z | représente le radical d'un cycle de formule |

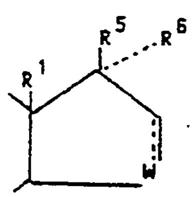

dans laquelle :

| | |
|---|---|
| $R^1$ | a la signification qui lui a été donnée à propos de la formule I, le trait pointillé partant de W traduit la présence éventuelle d'une double liaison, |

W représente un radical $CH_2$, CH, $CH_2CH_2$ ou $CHCH_2$,

$R^5/R^6$ représentent $-OR^7/-C\equiv C-U$,

$-OR^7/-\underset{\underset{O}{||}}{C}-CH_2-R^8$,

$-\underset{\underset{O}{||}}{C}-CH_2-R^8/-OR^7$,

$-\underset{\underset{O}{||}}{C}-CH_2-R^8/-CH_3$,

$-\underset{\underset{O}{||}}{C}-CH_2-R^8/-H$,

$-OR^7/-(CH_2)_m-CH_2-R^9$,

$-OR^7/-CH=CH(CH_2)_k-CH_2-R^9$,

$-OR^{10}/-H$,

$-OR^{10}/-(CH_2)_k-C\equiv C-U$,

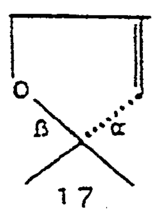
17

ou

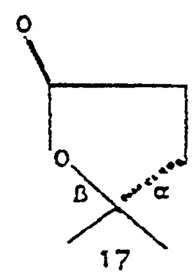
17

radicaux dans lesquels

$R^7$ représente un atome d'hydrogène ou un acyle contenant de 1 à 4 atomes de carbone,

U représente un atome d'hydrogène ou un alkyle, un hydroxyalkyle, un alcoxyalkyle ou un acyloxyalkyle contenant chacun de 1 à 4 atomes de carbone dans la partie alkyle ou acyle, ou représente un atome d'halogène,

$R^8$ représente un atome d'hydrogène, un hydroxy ou un alkyle, un alcoxy ou un acyloxy contenant chacun de 1 à 4 atomes de carbone,

$R^9$ représente un atome d'hydrogène, un hydroxy, un cyano ou un alcoxy ou un acyloxy contenant chacun de 1 à 4 atomes de carbone,

$R^{10}$ représente un atome d'hydrogène ou un alkyle ou un acyle contenant chacun de 1 à 10 atomes de carbone,

m est égal à 0, à 1, à 2 ou à 3,

k est égal à 0, à 1 ou à 2,

V représente le radical d'un cycle phényle de formule :

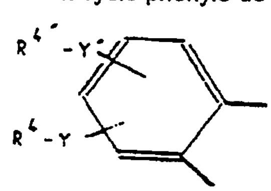

ou le radical d'un cycle hétéro-aromatique pentagonal ou hexagonal renfermant de 1 à 2 atomes N, O ou S et répondant à la formule :

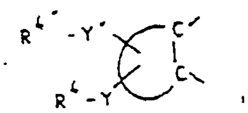

formules dans lesquelles :

R⁴ et R⁴′, qui peuvent être identiques ou différents, représentent chacun un atome d'hydrogène, un radical cyano ou un radical $—OR^{11}$, $—S(O)_kR^{11}$, $—N(O)_nR^{11}R^{12}$, $—O\text{-}SO_2R^{13}$, $—P(O)(OR^{14})_2$, $—SiR^{14}_3$ ou $—SnR^{14}_3$, l'indice k désignant un nombre égal à 0, à 1 ou à 2 et l'indice n un nombre égal à 0 ou à 1,

$R^{11}$ représente un atome d'hydrogène ou un alkyle en $C_1$-$C_8$,

$R^{12}$ a la signification de $R^{11}$ ou représente un radical cyano ou un radical acyle en $C_1$-$C_{10}$,

$R^{13}$ représente un alkyle en $C_1$-$C_4$ perfluoré,

$R^{14}$ représente un alkyle en $C_1$-$C_4$, ou

$R^{11}$ et $R^{12}$, à l'intérieur d'un radical $—N(O)_nR^{11}R^{12}$ forment ensemble et avec N un hétérocycle pentagonal ou hexagonal qui peut contenir un hétéro-atome supplémentaire N, O ou S,

Y et Y′, qui sont identiques ou différents, représentent chacun une liaison directe, un alkylène linéaire ou ramifié, éventuellement à une ou plusieurs liaisons doubles ou triples et à au plus 20 atomes de carbone, qui peut porter un ou plusieurs substituants pris dans l'ensemble constitué par les radicaux oxo, acyloxy en $C_1$-$C_{10}$, $—OR^{11}$, $—S(O)_kR^{11}$ et $—N(O)_nR^{11}R^{12}$, ou un arylène éventuellement substitué, ou

$R^4$-Y et $R^{4'}$-Y′ représentent ensemble le radical d'un cycle pentagonal ou hexagonal saturé, insaturé ou aromatique, éventuellement substitué, qui contient de 0 à 2 atomes d'oxygène, atomes de soufre et/ou radicaux $NR^{11}$, avec la condition que k et n ne soient supérieurs à 0 que lorsque $R^{11}$ représente un radical alkyle en $C_1$-$C_8$, et

le cycle A est :

a) un cycle répondant à la formule :

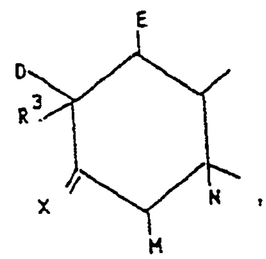

dans laquelle :

M et N représentent ensemble une deuxième liaison ou M représente un atome d'hydrogène et N un radical hydroxy, auquel cas B, $R^2$, G, $R^3$, D et E représentent des atomes d'hydrogène,

X représente un atome d'oxygène, deux atomes d'hydrogène ou un radical hydroxy-imino N ~OH,

$R^3$ et D, qui sont identiques ou différents, représentent chacun un atome d'hydrogène, un radical cyano ou un radical alkyle en $C_1$-$C_4$, ou forment ensemble un radical méthylène ou éthylène,

E représente un atome d'hydrogène ou ensemble un radical alkyle en $C_1$-$C_4$,

D et E représentent ensemble une deuxième liaison entre les atomes de carbone 1 et 2 ou ensemble un radical méthylène,

ou

b) un cycle répondant à la formule :

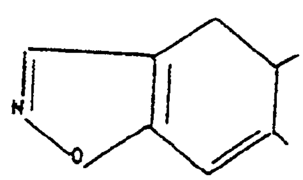

ou

c) un cycle répondant à la formule :

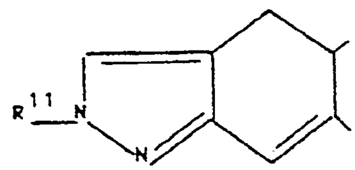

dans laquelle $R^{11}$ représente un atome d'hydrogène ou un radical alkyle en $C_1$-$C_8$, et éventuellement les sels d'addition acceptables du point de vue pharmaceutique qu'ils forment avec des acides.

2. Composés selon la revendication 1 caractérisés en ce que $R^2$, B et G représentent chacun un atome d'hydrogène.

3. Composés selon la revendication 1 caractérisés en ce que B et G représentent ensemble une deuxième liaison et $R^2$ représente un atome d'hydrogène.

4. Composés selon la revendication 1 caractérisés en ce que Y et Y′ représentent chacun une liaison directe, et $R^4$ et $R^{4'}$ représentent chacun un atome d'hydrogène.

5. Composés selon la revendication 1 caractérisés en ce que Y et Y′ représentent chacun une liaison directe, $R^4$ représente un atome d'hydrogène et $R^{4'}$ représente un atome d'azote porteur de deux alkyles en $C_1$-$C_8$.

6. Composés selon la revendication 1 caractérisés en ce que Y et Y′ représentent chacun une liaison directe, $R^4$ représente un atome d'hydrogène et $R^{4'}$un alcoxy en $C_1$-$C_8$.

7. Composés selon la revendication 1 caractérisés en ce que Y représente une liaison directe, $R^4$ et $R^{4'}$ représentent chacun un atome d'hydrogène, et Y′ représente un radical alkylène linéaire ou ramifié, éventuellement à une ou plusieurs liaisons doubles ou triples et à au plus 20 atomes de carbone, qui peut porter un radical oxo ou un radical —$OR^{11}$ dans lequel $R^{11}$ représente un atome d'hydrogène ou un alkyle en $C_1$-$C_8$.

8. Composés selon la revendication 1 caractérisés en ce que $R^4$-Y et $R^{4'}$-Y′ représentent ensemble le radical d'un cycle pentagonal ou hexagonal saturé, insaturé ou aromatique qui contient de 0 à 2 atomes d'oxygène ou de soufre et/ou radicaux —$NR^{11}$— dans lesquels $R^{11}$ représente un atome d'hydrogène ou un alkyle en $C_1$-$C_8$.

9. Composés selon la revendication 1 caractérisés en ce que Y′-$R^{4'}$ représente un atome d'hydrogène et Y-$R^4$ représente un radical éthyle, vinyle, isopropyle, isopropényle, propène-1(Z) yle, propène-1(E) yle, propène-2 yle, éthynyle, propynyle, propyne-2 yle, méthoxy, thiométhyle, thio-éthyle, hydroxy-1 éthyle ou diéthoxyphosphoryle, ou un radical aryle carbocyclique ou hétérocyclique, substitué ou non.

10. Composés selon la revendication 9 caractérisés en ce que le radical aryle est un radical phényle, naphtyle, méthoxy-2 phényle, méthoxy-3 phényle, méthoxy-4 phényle, tolyle-2, tolyle-3, tolyle-4, diméthylamino-2 phényle, diméthylamino-3 phényle, diméthylamino-4 phényle, furyle-2, furyle-3, thiényle-2, thiényle-3, pyridyle-2, pyridyle-3, pyridyle-4, thiazolyle ou imidazolyle.

11. Composés selon la revendication 1 caractérisés en ce que le cycle hétéro-atomique pentagonal ou hexagonal est un radical furyle, thiényle, pyridyle, pyrazolyle, pyrrolyle, pyrimidinyle, oxazolyle, pyridazinyle ou pyrazinyle.

12. Composés répondant à la formule générale II :

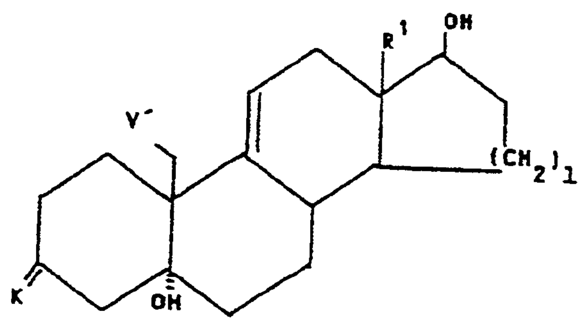

(II),

dans laquelle :

$R^1$ représente un radical méthyle ou éthyle,
l représente le nombre 1 ou le nombre 2,
K représente un radical oxo bloqué sous la forme d'un acétal ou d'un thio-acétal, et
V' représente un cycle phényle de formule :

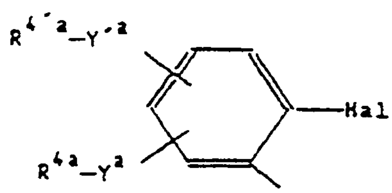

ou un cycle hétéro-aromatique pentagonal ou hexagonal contenant un ou deux atomes N, O ou S et répondant à la formule :

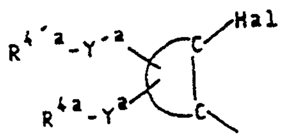

formules dans lesquelles Hal représente un atome de fluor, de chlore, de brome ou d'iode, $R^{4a}$, $R^{4'a}$, $Y^a$ et $Y'^a$ ont les mêmes significations que $R^4$, $R^{4'}$, Y et Y' dans la formule I mais ne peuvent représenter un radical cyano, et les éventuels radicaux hydroxy, mercapto, amino et oxo et/ou les éventuels radicaux acétylènes terminaux sont protégés.

13. Stéroïdes à pont en 19,11β qui répondent à la formule générale IV :

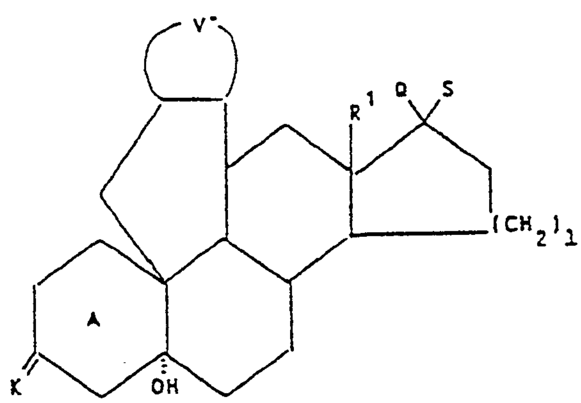

(IV)

dans laquelle :

$R^1$ représente un radical méthyle ou éthyle,
l représente le nombre 1 ou le nombre 2,
K représente un radical bloqué oxo sous la forme d'un acétal ou d'un thio-acétal, et
V'' représente un cycle phényle de formule :

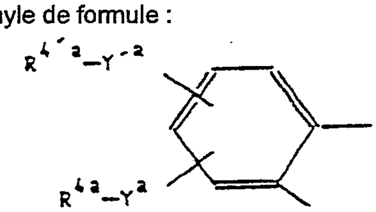

ou un cycle hétéro-aromatique pentagonal ou hexagonal contenant un ou deux atomes N, O ou S et répondant à la formule :

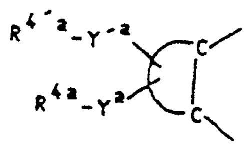

formules dans lesquelles $R^{4a}$, $R^{4'a}$, $Y^a$ et $Y'^a$ ont les mêmes significations que $R^4$, $R^{4'}$, Y et Y' dans la formule I mais ne peuvent représenter un radical cyano, et les éventuels radicaux hydroxy, mercapto, amino et oxo et/ou les éventuels radicaux acétylènes terminaux sont protégés,

Q représente un radical $R^5$ et
S représente un radical $R^6$, ou
Q et S représentent ensemble un atome d'oxygène cétonique.

14. Procédé pour préparer des composés répondant à la formule générale I :

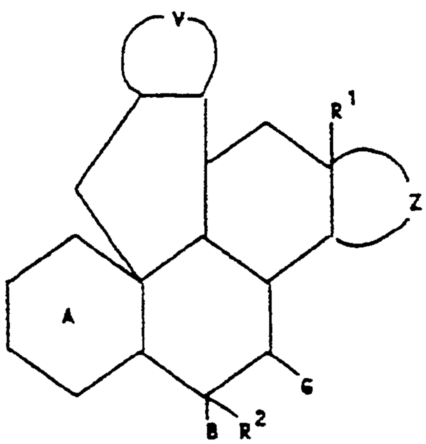

(I)

dans laquelle :

$R^1$ représente un radical méthyle ou éthyle,
$R^2$ représente un atome d'hydrogène ou de chlore ou un radical alkyle en $C_1$-$C_4$,
B et G, qui sont identiques ou différents, représentent chacun un atome d'hydrogène ou un alkyle en $C_1$-$C_4$, ou forment ensemble une deuxième liaison entre les atomes de carbone 6 et 7,
B et $R^2$ représentent ensemble un radical méthylène ou éthylène,
Z représente le radical d'un cycle de formule :

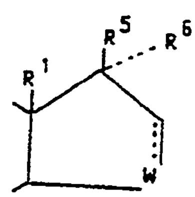

dans laquelle :

$R^1$ a la signification qui lui a été donnée à propos de la formule I, le trait pointillé partant de W traduit la présence éventuelle d'une double liaison,

W représente un radical $CH_2$, CH, $CH_2CH_2$ ou $CHCH_2$,

$R^5/R^6$ représentent $-OR^7/-C\equiv C-U$,

$-OR^7/-\underset{\underset{O}{\|}}{C}-CH_2-R^8$,

$-\underset{\underset{O}{\|}}{C}-CH_2-R^8/-OR^7$,

$-\underset{\underset{O}{\|}}{C}-CH_2-R^8/-CH_3$,

$-\underset{\underset{O}{\|}}{C}-CH_2-R^8/-H$,

$-OR^7/-(CH_2)_m-CH_2-R^9$,

$-OR^7/-CH=CH(CH_2)_k-CH_2-R^9$,

$-OR^{10}/-H$,

$-OR^{10}/-(CH_2)_k-C\equiv C-U$,

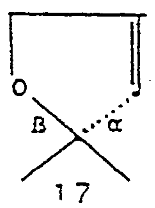
17

ou

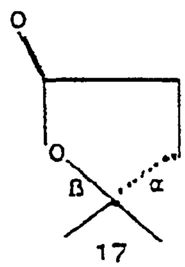
17

radicaux dans lesquels

$R^7$ représente un atome d'hydrogène ou un acyle contenant de 1 à 4 atomes de carbone,

U représente un atome d'hydrogène ou un alkyle, un hydroxyalkyle, un alcoxyalkyle ou un acyloxyalkyle contenant chacun de 1 à 4 atomes de carbone dans la partie alkyle ou acyle, ou représente un atome d'halogène,

$R^8$ représente un atome d'hydrogène, un hydroxy ou un alkyle, un alcoxy ou un acyloxy contenant chacun de 1 à 4 atomes de carbone,

$R^9$ représente un atome d'hydrogène, un hydroxy, un cyano ou un alcoxy ou un acyloxy contenant chacun de 1 à 4 atomes de carbone,

$R^{10}$ représente un atome d'hydrogène ou un alkyle ou un acyle contenant chacun de 1 à 10 atomes de carbone,

m est égal à 0, à 1, à 2 ou à 3,

k est égal à 0, à 1 ou à 2,

V représente le radical d'un cycle phényle de formule :

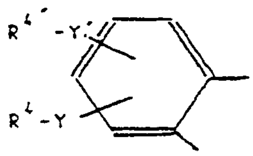

ou le radical d'un cycle hétéro-aromatique pentagonal ou hexagonal renfermant de 1 à 2 atomes N, O ou S et répondant à la formule :

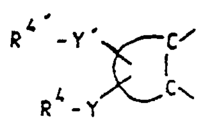

,

formules dans lesquelles

$R^4$ et $R^{4'}$, qui peuvent être identiques ou différents, représentent chacun un atome d'hydrogène, un radical cyano ou un radical $—OR^{11}$, $—S(O)_kR^{11}$, $—N(O)_nR^{11}R^{12}$, $—O\text{-}SO_2R^{13}$, $—P(O)(OR^{14})_2$, $—SiR^{14}{}_3$ ou $—SnR^{14}{}_3$, l'indice k désignant un nombre égal à 0, à 1 ou à 2 et l'indice n un nombre égal à 0 ou à 1,

$R^{11}$ représente un atome d'hydrogène ou un alkyle en $C_1$-$C_8$,

$R^{12}$ a la signification de $R^{11}$ ou représente un radical cyano ou un radical acyle en $C_1$-$C_{10}$,

$R^{13}$ représente un alkyle en $C_1$-$C_4$ perfluoré,

$R^{14}$ représente un alkyle en $C_1$-$C_4$, ou

$R^{11}$ et $R^{12}$, à l'intérieur d'un radical $—N(O)_nR^{11}R^{12}$, forment ensemble et avec N un hétérocycle pentagonal ou hexagonal qui peut contenir un hétéro-atome supplémentaire N, O ou S,

Y et Y', qui sont identiques ou différents, représentent chacun une liaison directe, un alkylène linéaire ou ramifié, éventuellement à une ou plusieurs liaisons doubles ou triples et à au plus 20 atomes de carbone, qui peut porter un ou plusieurs substituants pris dans l'ensemble constitué par les radicaux oxo, acyloxy en $C_1$-$C_{10}$, $—OR^{11}$, $—S(O)_kR^{11}$ et $—N(O)_nR^{11}R^{12}$, ou un arylène éventuellement substitué, ou

$R^4$-Y et $R^{4'}$-Y' représentent ensemble le radical d'un cycle pentagonal ou hexagonal saturé, insaturé ou aromatique, éventuellement substitué, qui contient de 0 à 2 atomes d'oxygène, atomes de soufre et/ou radicaux $NR^{11}$, avec la condition que k et n ne soient supérieurs à 0 que lorsque $R^{11}$ représente un radical alkyle en $C_1$-$C_8$, et

le cycle A est :

a) un cycle répondant à la formule :

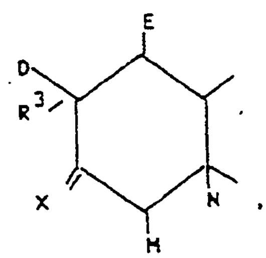

,

dans laquelle :

M et N représentent ensemble une deuxième liaison ou M représente un atome d'hydrogène et N un radical hydroxy, auquel cas B, $R^2$, G, $R^3$, D et E représentent des atomes d'hydrogène,

X représente un atome d'oxygène, deux atomes d'hydrogène ou un radical hydroxy-imino N ~OH,

$R^3$ et D, qui sont identiques ou différents, représentent chacun un atome d'hydrogène, un radical cyano ou un radical alkyle en $C_1$-$C_4$, ou forment ensemble un radical méthylène ou éthylène,

E représente un atome d'hydrogène ou un radical alkyle en $C_1$-$C_4$,

D et E représentent ensemble une deuxième liaison entre les atomes de carbone 1 et 2 ou ensemble un radical méthylène,

ou

b) un cycle répondant à la formule :

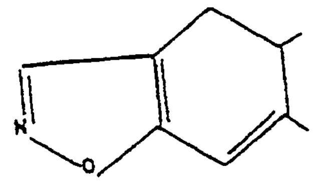

ou

c) un cycle répondant à la formule :

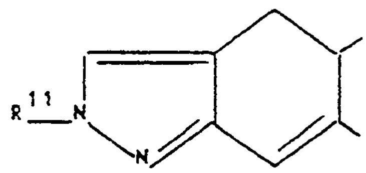

dans laquelle $R^{11}$ représente un atome d'hydrogène ou un radical alkyle en $C_1$-$C_8$, et éventuellement des sels d'addition acceptables du point de vue pharmaceutique qu'ils forment avec des acides, procédé caractérisé en ce qu'on cyclise des composés répondant à la formule générale II :

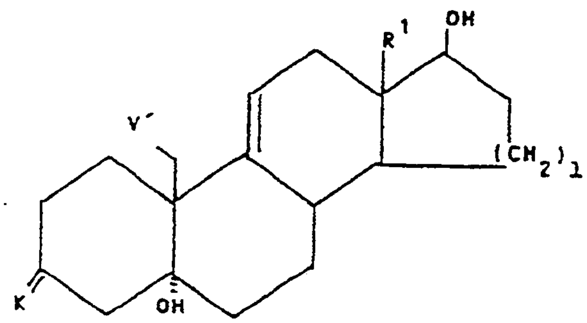

(II)

dans laquelle :

- $R^1$ représente un radical méthyle ou éthyle,
- l représente le nombre 1 ou le nombre 2,
- K représente un radical oxo bloqué sous la forme d'un acétal ou d'un thio-acétal, et
- V' représente un cycle phényle de formule :

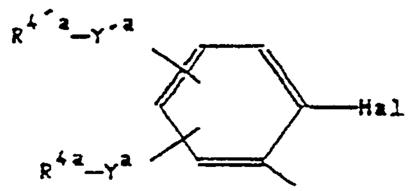

ou un cycle hétéro-aromatique pentagonal ou hexagonal contenant un ou deux atomes N, O ou S et répondant à la formule :

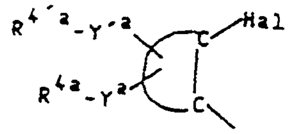

formules dans lesquelles Hal représente un atome de fluor, de chlore, de brome ou d'iode, $R^{4a}$, $R^{4'a}$, $Y^a$ et $Y'^a$ ont les mêmes significations que $R^4$, $R^{4'}$, Y et Y' mais ne peuvent représenter un radical cyano, les éventuels radicaux hydroxy, mercapto, amino et oxo et/ou les éventuels radicaux acétylènes terminaux étant protégés, par traitement avec un métal électropositif dans de l'ammoniac liquide, mélangé avec un ou plusieurs solvants organiques, ou, lorsque l'halogène présent en a comme substituant dans le composé de formule générale II est un atome de brome ou d'iode, également par voie radicalaire réductrice, de manière à obtenir les corps intermédiaires répondant à la formule générale IVa :

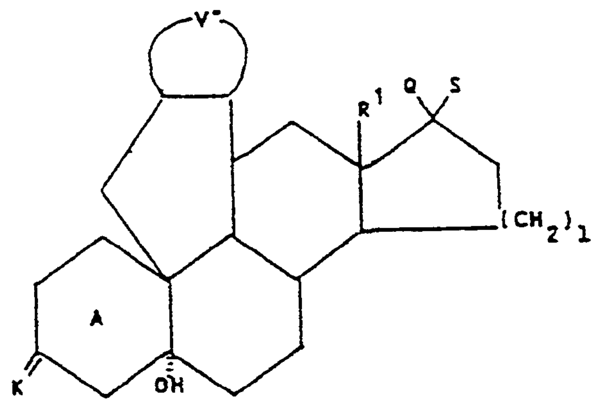

(IVa)

dans laquelle :

V" représente un cycle phényle de formule :

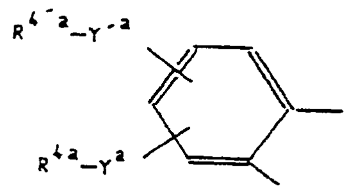

ou un cycle hétéro-aromatique pentagonal ou hexagonal contenant un ou deux atomes N, O ou S et répondant à la formule :

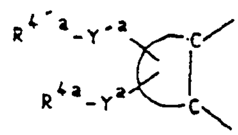

formules dans lesquelles $R^{4a}$, $R^{4'a}$, $Y^a$ et $Y'^a$ ont les mêmes significations que $R^4$, $R^{4'}$, Y et Y' mais ne peuvent représenter un radical cyano, les éventuels radicaux hydroxy, mercapto, amino et oxo et/ou les éventuels radicaux acétylènes terminaux étant protégés,

Q représente exclusivement un radical hydroxy ayant la configuration β et

S représente exclusivement un atome d'hydrogène ayant la configuration α,

et ensuite ou bien

a) on oxyde éventuellement le radical hydroxy porté par le carbone 17, puis

b) on débarrasse éventuellement un radical hydroxy protégé, dans V", du radical qui le protège, si on le désire, on prépare, à partir du composé hydroxylé, un perfluoralkyl-sulfonate correspondant, on transforme éventuellement le perfluoralkyl-sulfonate, soit directement, soit par échange du radical perfluoralkyl-sulfonate éliminable contre un radical trialkyl-étain en passant par le composé trialkyl-étain correspondant, en un composé qui comporte, dans V", éventuellement après des réactions ultérieures, le modèle de substitution souhaité,

ou bien on exécute d'abord b), puis a), après quoi :

c) on fonctionnalise de la façon souhaitée le cycle D par des méthodes connues, on soumet le produit ainsi obtenu à l'action d'un agent déshydratant qui est également capable de libérer le radical oxo en 3, pour provoquer l'enlèvement d'eau en même temps que la formation de la double liaison 4(5), et ensuite,

éventuellement après avoir protégé à nouveau des radicaux fonctionnels, libérés intermédiairement, contenus dans V et/ou Z, on introduit les fonctions voulues dans les cycles A et B du squelette stéroïdique, ou

d) on soumet le produit ainsi obtenu à l'action d'un agent déshydratant qui est également capable de libérer le radical oxo en 3, pour provoquer l'enlèvement d'eau et, en même temps, la formation de la double liaison 4(5), on introduit les fonctions voulues dans les cycles A et B du squelette stéroïdique, puis, après avoir protégé le radical oxo en 3, on fonctionnalise le cycle D de la façon souhaitée,

ou on exécute les étapes a) et b) après les étapes c) ou d),

on élimine éventuellement les radicaux protecteurs du produit ainsi obtenu, si on le désire on alkyle ou on acyle le ou les radicaux hydroxy, mercapto et/ou amino éventuellement contenu(s) dans V, si on le désire on introduit un radical cyano dans le ou les substituants aryles, si on le désire on oxyde le ou les radicaux amino et/ou sulfure(s) éventuellement contenu(s) dans le ou les substituants aryles, si on le désire on fait réagir avec le chlorhydrate d'hydroxylamine pour obtenir le produit de formule générale I dans lequel X représente le radical hydroxy-imino N ~OH, et éventuellement on prépare un sel d'addition d'acide acceptable du point de que pharmaceutique.

15. Procédé selon la revendication 19 caractérisé en ce qu'on cyclise en utilisant, comme métal électropositif, le sodium, le lithium, le potassium ou le calcium.

16. Compositions pharmaceutiques caractérisées en ce qu'elles contiennent des composés selon l'une quelconque des revendications 1 à 11.

17. Application de composés selon l'une quelconque des revendications 1 à 13 à la fabrication de médicaments.

Fig. 1

Influence of selected compounds given s.c. upon dexamethasone-induced thymus suppression in adrenalectomized ♂ - rats (b.w. 100 - 130 g). -

Treatment for 4 days (dose mg/d).
Thymus weight determined on day 5.

( )= n rats/group
| = 95% confidence-interval for %-abolition

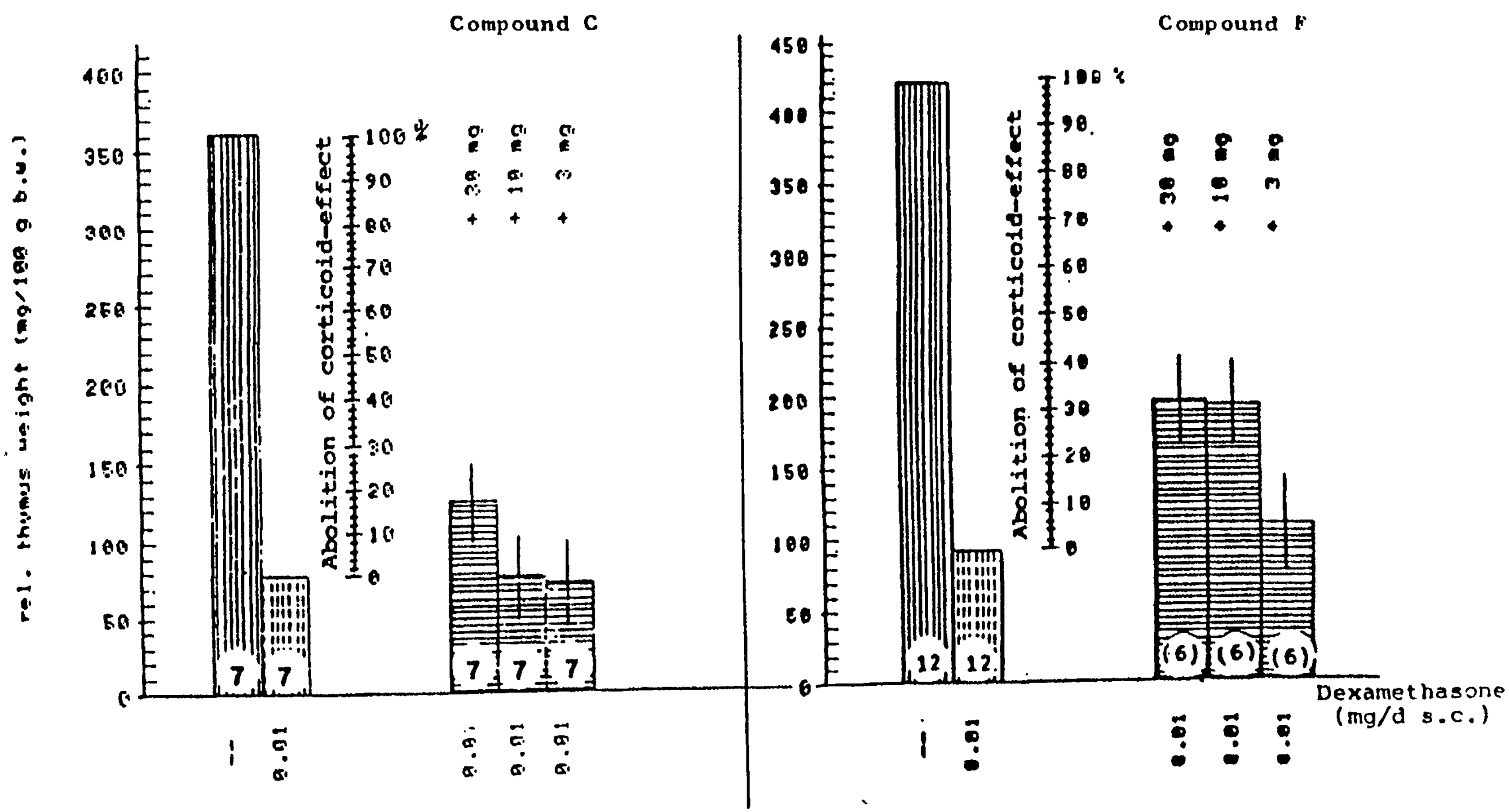

Fig. 2

**Influence of selected compounds given s.c. upon dexamethasone-induced thymus suppression in adrenalectomized ♂ - rats (b.w. 100 - 130 g). -**

Treatment for 4 days (dose mg/d).
Thymus weight determined on day 5.

( )= n rats/group
| = 95% confidence-interval for %-abolition